# EUROPEAN PATENT APPLICATION

(11) **EP 3 260 542 A1**
(43) Date of publication of application: **27.12.2017**
(21) Application number: 16175187.0
(22) Date of filing: 20.06.2016
(51) Int. Cl.: C12N 15/82

(54) **PROTEIN PRODUCTION IN PLANT CELLS**

(71) Applicant: Algentech, 91058 Evry Cedex (FR)
(72) Inventor: SOROKIN, Alexander, 75116 Paris (FR); MALCUIT, Isabelle, 75116 Paris (FR); JAKUBIEC, Anne, 92370 Chaville (FR); CAYLA, Thibaud, 78000 Versailles (FR)
(74) Representative: Cabinet Armengaud Aîné

(57) **Abstract**

Agrobacterium used for plastid transformation such as chloroplasts or mictochondira. An Agrobacterium withoutt VirD2 is used with a vector comprising VirD2 placed on the Ti-plasmid but outside the T-DNA. VirD2 is fused to a plastid signal sequence or has a Spytag allowing bindning to a SpyCatcher fused to a plastid transit sequence.

## Description

The present invention relates to a method for producing heterologous or exogenous DNA and RNA species in plant cell material such as genetically transformed plant cells in culture, plant tissue and plants derived from genetically transformed plant cells. In particular, the method relates to a more efficient method for producing DNA and RNA species and/or heterologous or exogenous proteins in plant organelles comprised in plant cell material, the genetic material required therefor, such as DNA and RNA, vectors, host cells, methods of introduction of genetic material into plant cells, plant cells comprising genetically modified plant organelles, and uses thereof.

Organelle transformation in plants has a great potential for the production of pharmaceuticals in plants, in improving the quality of food, as well as improving environmental stress resistance in plants. However, until the present invention truly efficient technologies, such as bombardment technologies, available for plastid transformation in a broad range of crop plants have been few. However, such plastid transformation events require several rounds of selection to achieve an homoplasmic state of transformation. The bombardment method is not efficient for the transformation of plant mitochondria because the size of mitochondria is considerably smaller than that of chloroplasts. Thus two problems for organelle transformation need addressing:
(i) delivery of transgenic nucleic acid (TNA) into plant organelles; and
(ii) amplification of the TNA to facilitate rapid achievement of an homoplasmic state in transformant plants.

The present invention describes efficient ways for both TNA delivery and amplification to facilitate rapid generation of organelle transformation in a wide range of crops.

For the purposes of the present invention the terms "plastid" and "plastids" and "plastid population" are used interchangeably, as are the terms "plant cell" and "plant cells", unless context demands otherwise. By employing or adapting endogenous cellular processes for the transfer of RNA derived from polynucleotide sequences introduced to the nucleus to the plastid genome, as described herein, the method of the invention is considered to be unique over prior art methods for the generation of plant cells or plants possessing genetically modified organelles, such as plastids and mitochondria.

According to the present invention there is provided an *Agrobacterium* strain comprising
a) dysfunctional native virD2 and/or virE2 DNA sequences, substantially knock out mutations of native virD2 and/or virE2 DNA sequences, or no native virD2 and/or virE2 DNA sequences; and/or
b) an Agrobacterium binary vector comprising a modified VirD2 DNA sequence lying outside of the T-DNA region comprising at least one of:
   i) a DNA sequence encoding an organellar transit peptide fused to the 5' end of a VirD2 DNA sequence;
   ii) a DNA sequence encoding a spytag peptide fused to the 5' end of a VirD2 DNA sequence; and
   iii) a DNA sequence encoding a spytag peptide fused to the 3' end of a VirD2 DNA sequence.

In such Agrobacterium strains, the native functionality of the VirE2 sequence of *Agrobacterium* is at least substantially negated, and the modified *Agrobacterium* VirD2 sequence is under the transcriptional control of a bacterial promoter, typically a chemically inducible bacterial promoter.

The organellar transit peptide can be selected from plastid transit peptides or mitochondria transit peptides. The plastid transit peptide may be selected from transit peptides of chloroplasts, proplastids, etioplasts, chromoplasts, amyloplasts, leucoplasts and elaioplasts. The plant organellar transit peptides may be independently selected from the mitochondrial signal peptide from tobacco F1-ATPase-1 β subunit, and the Arabidopsis CPN60 protein; and the plastidial transit peptide independently selected from the tobacco rbcS-cTP, and the Arabidopsis HSP70-cTP protein. In a preferment the organellar transit peptide may be selected from the transit peptides of Seq ID 10 (plastidial) and Seq ID 11 (mitochondrial).

A DNA coding sequence for a spytag peptide may be any short peptide that has a spytag peptide functionality, such as Seq ID 37.

The Agrobacterium vector may also comprise at least one of
iv) an organellar transgene cassette comprising two origins of replication, one being located adjacent to and at the 5' end of a left flanking sequence and the second being located adjacent to and at the 3' end of a right flanking sequence, at least one DNA sequence of interest under operative control of an organellar promoter, and an organellar terminator; and
v) an organellar transgene cassette comprising two origins of replication located at the 5' and 3' ends of the cassette, respectively, at least one DNA sequence of interest under operative control of an organellar promoter, wherein the organellar promoter is positioned downstream of the origin of replication at the 5' end of the transgene cassette, and an organellar terminator and the organellar cassette does not contain left and right flanking sequences;
wherein the said origins of replication are all derived from a geminivirus and the DNA sequences making up iv) and v), respectively, are all located within left and right T-DNA borders on the vector.

The DNA coding sequence of interest may be selected from that for a recombinant mammalian nucleic acid sequence, an isolated genomic mammalian nucleic acid sequence, a recombinant plant nucleic acid sequence and an isolated genomic plant nucleic acid sequence and two or more thereof.

The DNA coding sequence may be of any protein, polypeptide or peptide of interest, and may or may not include marker genes, such as that of sequence SEQ ID 17 (the aaDa gene sequence), in addition to transgenes of interest for protein production. Suitable DNA coding sequences may include one or more sequences of interest for proteis such as insulin, preproinsulin, proinsulin, glucagon, interferons such as α-interferon, β-interferon, γ-interferon, blood-clotting factors selected from Factor VII, VIII, IX, X, XI, and XII, fertility hormones including luteinising hormone, follicle stimulating hormone growth factors including epidermal growth factor, platelet-derived growth factor, granulocyte colony stimulating factor and the like, prolactin, oxytocin, thyroid stimulating hormone, adrenocorticotropic hormone, calcitonin, parathyroid hormone, somatostatin, erythropoietin (EPO), enzymes such as β-glucocerebrosidase, haemoglobin, serum albumin, collagen, biotic and abiotic stress proteins, such as insecticidal and insect toxic proteins, for example from, or derived from Bacillus thuringiensis, nematicidal proteins, herbicide resistance proteins, (e.g. to glyphosate), salt-tolerance proteins, drought tolerant proteins, proteins capable of conferring cytoplasmic male sterility to plant breeding lines; nutritional enhancement proteins involved in the biosynthesis of phenolics, starches, sugars, alkaloids, vitamins, and edible vaccines, monoclonal antibodies and active fragments thereof, industrial enzymes and active fragments thereof, and the like.

The origins of replication may be selected from those of gemini viruses such as those selected from Maize Streak Virus (MSV, subgroup I), for example SEQ ID 44, Beet Curly Top Virus (BCTV, subgroup II), for example SEQ ID 43, and Tomato Golden Mosaic Virus (TGMV, subgroup III), for example SEQ ID 45.

The left flanking and right flanking sequences (LFS(s) and RFS(s), respectively) may be selected from any plastid as defined herein or mitochondrial source, such as chloroplasts and mitochondria. Suitable chloroplast LFSs that may be used in the construction of vectors of the invention include the tobacco chloroplast LFS of Seq Id 15 and the rice chloroplast LFS of Seq Id 17 and their corresponding RFSs as shown in Seq Id 16 and Seq Id 18, respectively. Mitochondrial LFS and RFS sequences of use in the invention include those LFSs exemplified in Seq Id 23 (tobacco) and Seq Id 25 (rice) and RFSs exemplified in Seq Id 24 (tobacco) and Seq Id 26 (rice).

The organellar promoter may be selected from chloroplasts, proplastids, etioplasts, chromoplasts, amyloplasts, leucoplasts and elaioplasts, and mitochondria, preferably from chloroplassts and mitochondria. Suitable organellar promoters of use in the invention include the tobacco prrn chloroplast promoter (Seq Id 19), the wheat prrn chloroplast promoter (Seq ID 20) the tobacco atp9 mitochondrion promoter (Seq Id 21) and the rice atp6 mitochondrion promoter (Seq Id 22). Other organellar promoters of use in the invention include mitochondrion specific promoters selected from mitochondrial promoter nucleotide sequences, such as ATP6, ATP9, Cob, rrn18, Rps13, Rps19, Cox3, Nad6, Nad9 5' untranslated sequences (promoter region) of tobacco mitochondria, and Arabidopsis mitochondria; and the plastid specific promoter sequences selected from the group consisting of the RNA polymerase promoter, rpo B promoter element, atpB promoter element, the clpP promoter element, the 16S rDNA promoter element, PrbcL, Prps16, the Prrn16, Prrn-62, Pycf2-1577, PatpB-289, Prps2-152, Prps16-107, Pycf1-41, PatpI-207, PclpP-511, PclpP-173, PaccD-129, PaccD-129 promoter of the tobacco accD gene, the PclpP-53 promoter of the clpP gene, the Prrn-62 promoter of the rrn gene, the Prps16-107 promoter of the rps16 gene, the PatpB/E-290 promoter of the tobacco atpB/E gene, and the PrpoB-345 promoter of the rpoB gene.

The expression in the plastid, such as in the chloroplast, is effected by employing a plant plastid promoter such as plastid specific promoters and/or transcription regulation elements as alluded to above. Examples include the RNA polymerase promoter (WO 97/06250) and other promoters described in the art, eg in WO 00/07431, U.S. Pat. No. 5,877,402, WO 97/06250, WO 98/55595, WO 99/46394, WO 01/42441 and WO 01/07590; the rpo B promoter element, the atpB promoter element, the clpP promoter element (see also WO 99/46394) and the 16S rDNA promoter element. The plastid specific promoter may also have a polycistronic "operon" assigned to it (EP-A 1 076 095; WO 00/20611). Further promoters that may be used in the method of the invention also include the PrbcL promoter, the Prps16 promoter, and the Prrn16 promoter described in US Patent application 2006/0253916, the plastid specific promoters Prrn-62, Pycf2-1577, PatpB-289, Prps2-152, Prps16-107, Pycf1-41, PatpI-207, PclpP-511, PclpP-173 and PaccD-129 (WO 97/06250; Hajdukiewicz P T J et al. (1997) EMBO J 16:4041-4048), the PaccD-129 promoter of the tobacco accD gene (WO 97/06250), the PclpP-53 promoter of the clpP gene as highly active NEP promoter in chloroplasts (WO 97/06250), the Prrn-62 promoter of the rrn gene, the Prps16-107 promoter of the rps16 gene, the PatpB/E-290 promoter of the tobacco atpB/E gene (Kapoor S et al. (1997) Plant J 11:327-337), and the PrpoB-345 promoter of the rpoB gene (Liere K & Maliga P (1999) EMBO J 18: 249-257). Furthermore, all those promoters which belong to class III (Hajdukiewicz P T J et al. (1997) EMBO J 16:4041-4048) and all fragments of the class II promoters which control the initiation of transcription by NEP may be utilized in the method of the invention. Such promoters or promoter moieties are not generally known to be highly conserved. ATAGAATAAA is given as consensus near the transcription initiation site of NEP promoters. (Hajdukiewicz P T J et al (1997) EMBO J 16:4041-4048). The organellar terminator may be selected from chloroplasts, proplastids, etioplasts, chromoplasts, amyloplasts, leucoplasts and elaioplasts, and mitochondria, preferably from chloroplassts and mitochondria. Suitable organellar promoters of use in the invention include the tobacco prrn chloroplast promoter (Seq Id 19), the wheat prrn chloroplast promoter (Seq ID 20) the tobacco atp9 mitochondrion promoter (Seq Id 21) and the rice atp6 mitochondrion promoter (Seq Id 22).

In alternative vi), the organellar transgene cassette comprising two origins of replication located at the 5' and 3' ends of the cassette, respectively, at least one DNA sequence of interest under operative control of an organellar promoter, wherein the organellar promoter is positioned downstream of the origin of replication at the 5' end of the transgene cassette, and an organellar terminator and the organellar cassette does not contain left flanking and right flanking sequences.

The DNA sequences making up iv) and v) of the Agrobacterium vector, respectively, are all located within a left border and a right border on the vector (i.e. the left and right borders are 25-base pair repeats on each end of the transfer DNA (also referred to as T-DNA).

A DNA sequence coding for a replication initiation protein (Rep) selected from those of a suitable geminivirus, such as a functional Rep gene coding for a Rep protein selected from Beet Curly Top Virus, B-Rep (Seq Id 46), Maize Streak Virus, M-Rep (Seq Id 47), and Tomato Golden Mosaic Virus (T-Rep) (Seq Id 48) may be utilised to boost replication in the organelle. A vector of the invention, wherein expression of a viral Rep gene as defined herein is either from a transgene DNA coding sequence or from a vector comprising a cassette comprising a *Rep* gene fused to an organellar transit peptide, wherein the fused peptide is under operational control of a nuclear promoter and a nuclear terminator is also provided.

The vector described in v) may be present in the form of a single stranded or double-stranded circular DNA or mini-chromosome.

The nuclear promoter is a constitutive promoter or a chemically inducible promoter. Constitutive promoters may be selected from a plant nuclear promoter (for example, an exogenous nucleus specific promoter) is one that is able to drive expression of a nucleic acid sequence such as a cDNA sequence or a full length gene sequence in the nucleus of a plant cell, forming a transcribed RNA sequence. The plant nuclear promoter is one that is introduced in front of a nucleic acid sequence of interest and is operably associated therewith. Thus a plant nuclear promoter is one that has been placed in front of a selected polynucleotide component. Typically, a plant nuclear promoter, such as an exogenous nucleus specific promoter, is one that is transferred to a host cell or host plant from a source other than the host cell or host plant.

The cDNAs encoding a polynucleotide of the invention contain at least one type of nucleus specific promoter that is operable in a plant cell, for example, an inducible or a constitutive promoter operatively linked to a first and/or second nucleic acid sequence or nucleic acid sequence component as herein defined and as provided by the present invention. As discussed, this enables control of expression of polynucleotides of the invention. The invention also provides plants transformed with polynucleotide sequences or constructs and methods including introduction of such polynucleotide nucleic acid sequences or constructs into a plant cell and/or induction of expression of said first or second nucleic acid sequence or construct within a plant cell, e.g. by application of a suitable stimulus, such as an effective exogenous inducer.

The term "inducible" as applied to a promoter is well understood by those skilled in the art. In essence, expression under the control of an inducible promoter is "switched on" or increased in response to an applied stimulus (which may be generated within a cell or provided exogenously). The nature of the stimulus varies between promoters. Some inducible promoters cause little or undetectable levels of expression (or no expression) in the absence of the appropriate stimulus. Other inducible promoters cause detectable constitutive expression in the absence of the stimulus. Whatever the level of expression is in the absence of the stimulus, expression from any inducible promoter is increased in the presence of the correct stimulus. The preferable situation is where the level of expression increases upon application of the relevant stimulus by an amount effective to alter a phenotypic characteristic. Thus an inducible (or "switchable") promoter may be used which causes a basic level of expression in the absence of the stimulus which level is too low to bring about a desired phenotype (and may in fact be zero). Upon application of the stimulus, expression is increased (or switched on) to a level, which brings about the desired phenotype. One example of an inducible promoter is the ethanol inducible gene switch disclosed in Caddick et al (1998) Nature Biotechnology 16: 177-180. A number of inducible promoters are known in the art.

Chemically regulated promoters can be used to modulate the expression of a gene or a polynucleotide sequence of the invention in a plant through the application of an exogenous chemical regulator. Depending upon the objective, the promoter may be a chemically inducible promoter, where application of the chemical induces gene expression, or a chemical-repressible promoter, where application of the chemical represses gene expression. Chemically inducible promoters are known in the art and include, but are not limited to, the maize In2-2 promoter, which is activated by benzenesulfonamide herbicide safeners, the maize GST promoter, which is activated by hydrophobic electrophilic compounds that are used as pre-emergent herbicides, and the tobacco PR-1a promoter, which is activated by salicylic acid. Other chemically regulated promoters of interest include steroid-responsive promoters (see, for example, the glucocorticoid-inducible promoter in Schena et al. (1991) Proc. Natl. Acad. Sci. USA 88:10421-10425 and McNellis et al. (1998) Plant J. 14(2):247-257) and tetracycline-inducible and tetracycline-repressible promoters (see, for example, Gatz et al. (1991) Mol. Gen. Genet. 227:229-237, and U.S. Patent Nos. 5,814,618 and 5,789,156), herein incorporated by reference.

Where enhanced expression in particular tissues is desired, tissue-specific promoters can be utilized. Tissue-specific promoters include those described by Yamamoto et al. (1997) Plant J. 12(2)255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 112(3):1331-1341; Van Camp et al. (1996) Plant Physiol. 112(2):525-535; Canevascini et al. (1996) Plant Physiol. 112(2):513-524; Yamamoto et al. (1994) Plant Cell Physiol. 35(5):773-778; Lam (1994) Results Probl. Cell Differ. 20:181-196; Orozco et al. (1993) Plant Mol Biol. 23(6):1129-1138; Matsuoka et al. (1993) Proc Natl. Acad. Sci. USA 90(20):9586-9590; and Guevara-Garcia et al. (1993) Plant J. 4(3):495-505.

So-called constitutive promoters may be used in the vectors, and cassettes, and methods of the present invention. Constitutive promoters include, for example, CaMV 35S promoter (Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (U.S. Application Serial No. 08/409,297), and the like. Other constitutive promoters include those in U.S. Patent Nos. 5,608,149; 5,608,144; 5,604,121; 5,569,597; 5,466,785; 5,399,680; 5,268,463; and 5,608,142. In a preferment, the plant nuclear promoter used in the method of the invention is a constitutive promoter selected from the Ubiq3At Arabidopsis Promoter (SEQ ID 30), the cauliflower Mosaic virus 35S promoter (Seq Id 28) and the UbiqM maize Promoter (Seq Id 29).

Naturally, the man skilled in the art will appreciate that other terminator DNA sequences may be present in vectors or constructs comprising Rep DNA as used in the invention. A terminator is contemplated as a DNA sequence at the end of a transcriptional unit which signals termination of transcription. These elements are 3'-non-translated sequences containing polyadenylation signals, which act to cause the addition of polyadenylate sequences to the 3' end of primary transcripts. For expression in plant cells the nopaline synthase transcriptional terminator (A. Depicker et al., 1982, J. of Mol. & Applied Gen. 1:561-573) sequence serves as a transcriptional termination signal (Seq Id 30) as does the Ags terminator (Seq Id 31).

Those skilled in the art are well able to construct vectors and design protocols for recombinant nucleic acid sequences or gene expression. Suitable vectors can be chosen or constructed, containing appropriate regulatory sequences, including promoter sequences, terminator fragments, polyadenylation sequences, enhancer sequences, marker genes and other sequences as appropriate. For further details see, for example, Molecular Cloning: a Laboratory Manual: 2nd edition, Sambrook et al, 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Second Edition, Ausubel et al. eds., John Wiley & Sons, 1992. The disclosures of Sambrook et al. and Ausubel et al. are incorporated herein by reference. Specific procedures and vectors previously used with wide success upon plants are described by Bevan (Nucl. Acids Res. 12, 8711-8721 (1984)) and Guerineau and Mullineaux (1993) (Plant transformation and expression vectors. In: Plant Molecular Biology Labfax (Croy RRD ed.) Oxford, BIOS Scientific Publishers, pp 121-148).

Naturally, the skilled addressee will appreciate that each introduced transgene in a transgene cassette will be under regulatory control of its own exogenous plastidal or mitochondrial promoter, for example a chloroplast promoter and terminator or a mitochondrial promoter and terminator. When two or more target proteins are destined to be produced from a single carrier RNA it is preferable if they are able to be readily separated, for example by binding to different protein-specific antibodies (monoclonal or polyclonal) in the harvesting phase of the plant cell culture system.

Selectable genetic markers may facilitate the selection of transgenic plants and these may consist of chimaeric genes that confer selectable phenotypes such as resistance to antibiotics such as spectinomycin, streptomycin, kanamycin, neomycin, hygromycin, puramycin, phosphinotricin, chlorsulfuron, methotrexate, gentamycin, spectinomycin, imidazolinones, aadA and glyphosate.

When introducing selected nucleic acid sequences according to the present invention into a cell, certain considerations must be taken into account, well known to those skilled in the art. The nucleic acid to be inserted should be assembled within a construct, which contains effective regulatory elements, which will drive transcription. There must be available a method of transporting the construct into the cell. Once the construct is within the cell, integration into the endogenous chromosomal material either will or will not occur. Finally, as far as plants are concerned the target cell type must be such that cells can be regenerated into whole plants.

Plants transformed with DNA segments containing sequences of interest as provided herein may be produced by standard techniques, which are already known for the genetic manipulation of plants. DNA can be transformed into plant cells using any suitable technology, such as a disarmed Ti-plasmid vector carried by Agrobacterium exploiting its natural gene transfer ability (EP-A-270355, EP-A-0116718, NAR 12(22) 8711 -87215 1984), particle or micro projectile bombardment (US 5100792, EP-A-444882, EP-A-434616) microinjection (WO 92/09696, WO 94/00583, EP 331083, EP 175966, Green et al. (1987) Plant Tissue and Cell Culture, Academic Press), electroporation (EP 290395, WO 8706614) other forms of direct DNA uptake (DE 4005152, WO 9012096, US 4684611), liposome mediated DNA uptake (e.g. Freeman et al. Plant Cell Physiol. 29: 1353 (1984)), or the vortexing method (e.g. Kindle, PNAS U.S.A. 87: 1228 (1990d) Physical methods for the transformation of plant cells are reviewed in Oard, 1991, Biotech. Adv. 9: 1-11.

Thus once a nucleic acid sequence or gene has been identified, it may be reintroduced into plant cells using techniques well known to those skilled in the art to produce transgenic plants of the appropriate phenotype.

*Agrobacterium* transformation is widely used by those skilled in the art to transform dicotyledonous species. Production of stable, fertile transgenic plants in almost all economically relevant monocot plants is also now routine:(Toriyama, et al. (1988) Bio/Technology 6, 1072-1074; Zhang, et al. (1988) Plant Cell Rep. 7, 379-384; Zhang, et al. (1988) Theor. Appl. Genet 76, 835-840; Shimamoto, et al. (1989) Nature 338, 274-276; Datta, et al. (1990) Bio/Technology 8, 736-740; Christou, et al. (1991) Bio/Technology 9, 957-962; Peng, et al. (1991) International Rice Research Institute, Manila, Philippines 563-574; Cao, et al. (1992) Plant Cell Rep. 11, 585-591; Li, et al. (1993) Plant Cell Rep. 12, 250-255; Rathore, et al. (1993) Plant Molecular Biology 21, 871-884; Fromm, et al. (1990) Bio/Technology 8, 833-839; Gordon-Kamm, et al. (1990) Plant Cell 2, 603-618; D'Halluin, et al. (1992) Plant Cell 4, 1495-1505; Walters, et al. (1992) Plant Molecular Biology 18, 189-200; Koziel, et al. (1993) Biotechnology 11, 194-200; Vasil, I. K. (1994) Plant Molecular Biology 25, 925-937; Weeks, et al. (1993) Plant Physiology 102, 1077-1084; Somers, et al. (1992) Bio/Technology 10, 1589-1594; WO92/14828). In particular, *Agrobacterium* mediated transformation is now a highly efficient alternative transformation method in monocots (Hiei et al. (1994) The Plant Journal 6, 271-282).

The generation of fertile transgenic plants has been achieved in the cereals rice, maize, wheat, oat, and barley (reviewed in Shimamoto, K. (1994) Current Opinion in Biotechnology 5, 158-162.; Vasil, et al. (1992) Bio/Technology 10, 667-674; Vain et al., 1995, Biotechnology Advances 13 (4) : 653-671; Vasil, 1996, Nature Biotechnology 14 page 702). Wan and Lemaux (1994) Plant Physiol. 104: 37-48 describe techniques for generation of large numbers of independently transformed fertile barley plants.

Micro projectile bombardment, electroporation and direct DNA uptake are preferred where Agrobacterium is inefficient or ineffective. Alternatively, a combination of different techniques may be employed to enhance the efficiency of the transformation process, e.g. bombardment with Agrobacterium coated micro particles (EP-A-486234) or micro projectile bombardment to induce wounding followed by cocultivation with Agrobacterium (EP-A-486233).

Following transformation, a plant may be regenerated, e.g. from single cells, callus tissue or leaf discs, as is standard in the art. Almost any plant can be entirely regenerated from cells, tissues and organs of the plant. Available techniques are reviewed in Vasil et al., Cell Culture and Somatic Cell Genetics of Plants, Vol. I, II and III, Laboratory Procedures and Their Applications, Academic Press, 1984, and Weiss Bach and Weiss Bach, Methods for Plant Molecular Biology, Academic Press, 1989.

The particular choice of a transformation technology will be determined by its efficiency to transform certain plant species as well as the experience and preference of the person practising the invention with a particular methodology of choice. It will be apparent to the skilled person that the particular choice of a transformation system to introduce nucleic acid into plant cells is not essential to or a limitation of the invention, nor is the choice of technique for plant regeneration.

Also according to the invention there is provided a plant cell having incorporated into its genome at least a nucleotide sequence, particularly heterologous nucleotide sequences, as provided by the present invention under operative control of regulatory sequences for control of expression as herein described. The coding sequence may be operably linked to one or more regulatory sequences which may be heterologous or foreign to the nucleic acid sequences employed in the invention, such as those not naturally associated with the nucleic acid sequence(s) for its(their) expression. The nucleotide sequence according to the invention may be placed under the control of an externally inducible promoter to place expression under the control of the user. A further aspect of the present invention provides a method of making such a plant cell involving introduction of nucleic acid sequence(s) contemplated for use in the invention or a suitable vector including the sequence(s) contemplated for use in the invention into a plant cell and causing or allowing recombination between the vector and the plant cell genome to introduce the said sequences into the genome. The invention extends to plant cells containing a nucleotide sequence according to the invention as a result of introduction of the nucleotide sequence into an ancestor cell.

The term "heterologous" may be used to indicate that the gene/sequence of nucleotides in question have been introduced into said cells of the plant or an ancestor thereof, using genetic engineering, ie by human intervention. A transgenic plant cell, i.e. transgenic for the nucleotide sequence in question, may be provided. The transgene may be on an extra-genomic vector or incorporated, preferably stably, into the genome. A heterologous gene may replace an endogenous equivalent gene, ie one that normally performs the same or a similar function, or the inserted sequence may be additional to the endogenous gene or other sequence. An advantage of introduction of a heterologous gene is the ability to place expression of a sequence under the control of a promoter of choice, in order to be able to influence expression according to preference. Furthermore, mutants, variants and derivatives of the wild-type gene, e.g. with higher activity than wild type, may be used in place of the endogenous gene. Nucleotide sequences heterologous, or exogenous or foreign, to a plant cell may be non-naturally occurring in cells of that type, variety or species. Thus, a nucleotide sequence may include a coding sequence of or derived from a particular type of plant cell or species or variety of plant, placed within the context of a plant cell of a different type or species or variety of plant. A further possibility is for a nucleotide sequence to be placed within a cell in which it or a homologue is found naturally, but wherein the nucleotide sequence is linked and/or adjacent to nucleic acid which does not occur naturally within the cell, or cells of that type or species or variety of plant, such as operably linked to one or more regulatory sequences, such as a promoter sequence, for control of expression. A sequence within a plant or other host cell may be identifiably heterologous, exogenous or foreign.

Plants which include a plant cell according to the invention are also provided, along with any part or propagule thereof, seed, selfed or hybrid progeny and descendants. Particularly provided are transgenic crop plants, which have been engineered to carry genes identified as stated above. Examples of suitable plants include tobacco *(Nicotiana tabacum)* and other *Nicotiana* species, carrot, vegetable and oilseed *Brassicas,* melons, Capsicums, grape vines, lettuce, strawberry, sugar beet, wheat, barley, corn(maize), rice, soybean, peas, sorghum, sunflower, tomato, cotton, and potato. Especially preferred transgenic plants of the invention include cotton, rice, oilseed Brassica species such as canola, corn(maize) and soybean.

In addition to a plant, the present invention provides any clone of such a plant, seed, selfed or hybrid progeny and descendants, and any part of any of these, such as cuttings, seed. The invention provides any plant propagule that is any part which may be used in reproduction or propagation, sexual or asexual, including cuttings, seed and so on. Also encompassed by the invention is a plant which is a sexually or asexually propagated offspring, clone or descendant of such a plant, or any part or propagule of said plant, offspring, clone or descendant.

The present invention also encompasses the polypeptide expression product of a nucleic acid molecule according to the invention as disclosed herein or obtainable in accordance with the information and suggestions herein. Also provided are methods of making such an expression product by expression from a nucleotide sequence encoding therefore under suitable conditions in suitable host cells e.g. *E.coli.* Those skilled in the art are well able to construct vectors and design protocols and systems for expression and recovery of products of recombinant gene expression.

The heterologous or exogenous target protein is contemplated to be any protein of interest that may be produced by the method of the invention.

A polypeptide according to the present invention may be an allele, variant, fragment, derivative, mutant or homologue of the(a) polypeptides as mentioned herein. The allele, variant, fragment, derivative, mutant or homologue may have substantially the same function of the polypeptides alluded to above and as shown herein or may be a functional mutant thereof.

"Homology" in relation to an amino acid sequence or polypeptide sequence produced by the method of the invention may be used to refer to identity or similarity, preferably identity. As noted already above, high level of amino acid identity may be limited to functionally significant domains or regions.

In certain embodiments, an allele, variant, derivative, mutant derivative, mutant or homologue of the specific sequence may show little overall homology, say about 20%, or about 25%, or about 30%, or about 35%, or about 40% or about 45%, with the specific sequence. However, in functionally significant domains or regions, the amino acid homology may be much higher. Putative functionally significant domains or regions can be identified using processes of bioinformatics, including comparison of the sequences of homologues.

Functionally significant domains or regions of different polypeptides may be combined for expression from encoding nucleic acid as a fusion protein. For example, particularly advantageous or desirable properties of different homologues may be combined in a hybrid protein, such that the resultant expression product, may include fragments of various parent proteins, if appropriate.

Similarity of amino acid sequences may be as defined and determined by the TBLASTN program, of Altschul et al. (1990) J. Mol. Biol. 215: 403-10, which is in standard use in the art. In particular, TBLASTN 2.0 may be used with Matrix BLOSUM62 and GAP penalties: existence: 11, extension: 1. Another standard program that may be used is BestFit, which is part of the Wisconsin Package, Version 8, September 1994, (Genetics Computer Group, 575 Science Drive, Madison, Wisconsin, USA, Wisconsin 53711). BestFit makes an optimal alignment of the best segment of similarity between two sequences. Optimal alignments are found by inserting gaps to maximize the number of matches using the local homology algorithm of Smith and Waterman (Adv. Appl. Math. (1981) 2: 482-489). Other algorithms include GAP, which uses the Needleman and Wunsch algorithm to align two complete sequences that maximizes the number of matches and minimizes the number of gaps. As with any algorithm, generally the default parameters are used, which for GAP are a gap creation penalty = 12 and gap extension penalty = 4. Alternatively, a gap creation penalty of 3 and gap extension penalty of 0.1 may be used. The algorithm FASTA (which uses the method of Pearson and Lipman (1988) PNAS USA 85: 2444-2448) is a further alternative.

Use of either of the terms "homology" and "homologous" herein does not imply any necessary evolutionary relationship between compared sequences, in keeping for example with standard use of terms such as "homologous recombination" which merely requires that two nucleotide sequences are sufficiently similar to recombine under the appropriate conditions.

In a further aspect of the invention, there is provided an isolated polynucleotide sequence that comprises
a) a dysfunctional VirE2 DNA sequence, substantially no VirE2 DNA or no VirE2 DNA sequence;
b) a modified VirD2 DNA sequence comprising at least one of:
   i) a DNA sequence encoding an organellar transit peptide fused to the 5' end of a VirD2 DNA sequence;
   ii) a DNA sequence encoding a spytag peptide fused to the 5' end of a VirD2 DNA sequence; and
   iii) a DNA sequence encoding a spytag peptide fused to the 3' end of a VirD2 DNA sequence.
      The isolated polynucleotide sequence of this aspect of the invention further may further comprise at least one of
   iv) an organellar transgene cassette comprising two origins of replication, one being located adjacent to and at the 5' end of a left flanking sequence and the second being located adjacent to and at the 3' end of a right flanking sequence, at least one DNA sequence of interest under operative control of an organellar promoter, and an organellar terminator; and
   v) an organellar transgene cassette comprising two origins of replication located at the 5' and 3' ends of the cassette, respectively, at least one DNA sequence of interest under operative control of an organellar promoter, wherein the organellar promoter is positioned downstream of the origin of replication at the 5' end of the transgene cassette, and an organellar terminator and the organellar cassette does not contain left and right flanking sequences; and
   wherein the said origins of replication are all derived from a geminivirus.

Naturally, the skilled addressee will appreciate that the isolated polynucleotide sequence as defined herein may comprise genomic DNA and/or cDNA. The skilled addressee will also appreciate that the description of each of its component parts is as defined herein for other aspects and variants of the invention.

In a further aspect of the invention there is provided use of a polynucleotide sequence as defined herein, in the production of a transgenic plant. Also provided herein is use of a polynucleotide sequence as defined herein, in the production of a polypeptide or protein in a plant.

In a still further aspect of the invention, there is provided a method of transforming a plant cell with a DNA of interest via an *Agrobacterium* vector comprising the steps of:
a) introducing into the plant cell at least a first nucleic acid sequence that comprises at least one of:
   i) an organellar transgene cassette comprising two origins of replication, one being located adjacent to the 5' end of a left flanking sequence and the second being located adjacent to the 3' end of a right flanking sequence, at least one DNA sequence of interest encoding a transgene of interest under operative control of an organellar promoter, and an organellar terminator; and
   ii) an organellar transgene cassette comprising two origins of replication located at the 5' and 3' ends of the cassette, respectively, at least one DNA sequence of interest encoding a transgene of interest under operative control of an organellar promoter, the organellar promoter being positioned downstream of the origin of replication at the 5' end of the transgene cassette, an organellar terminator and wherein the organellar cassette does not contain left and right flanking sequences;
wherein the said origins of replication are all derived from a geminivirus and the DNA sequences making up i) and ii), respectively, are all located within a left border and a right border on the vector.

In this method aspect of the invention, the organellar promoter and organellar terminator are selected from a plant mitochondrion promoter, a plant mitochondrion terminator, a plant plastid promoter, and a plant plastid terminator, respectively. Suitably, the plant organellar promoter and plant organellar terminator are selected from plastid promoters and plant plastid terminators selected from chloroplasts, proplastids, etioplasts, chromoplasts, amyloplasts, leucoplasts and elaioplasts, the promoter and terminator being preferably selected from chloroplasts.

In this method aspect of the invention, the DNA coding sequence of interest is selected from a recombinant mammalian nucleic acid sequence, an isolated genomic mammalian nucleic acid sequence, a recombinant plant nucleic acid sequence and an isolated genomic plant nucleic acid sequence and two or more thereof. The DNA coding sequence of interest or isolated nucleic acid sequence of interest encodes a transgene of interest and may be selected from insulin, preproinsulin, proinsulin, glucagon, interferons such as α-interferon, β-interferon, γ-interferon, blood-clotting factors selected from Factor VII, VIII, IX, X, XI, and XII, fertility hormones including luteinising hormone, follicle stimulating hormone growth factors including epidermal growth factor, platelet-derived growth factor, granulocyte colony stimulating factor and the like, prolactin, oxytocin, thyroid stimulating hormone, adrenocorticotropic hormone, calcitonin, parathyroid hormone, somatostatin, erythropoietin (EPO), enzymes such as β-glucocerebrosidase, haemoglobin, serum albumin, collagen, biotic and abiotic stress proteins, such as insecticidal and insect toxic proteins, for example from, or derived from Bacillus thuringiensis, nematicidal proteins, herbicide resistance proteins, (e.g. to glyphosate), salt-tolerance proteins, drought tolerant proteins, proteins capable of conferring cytoplasmic male sterility to plant breeding lines; nutritional enhancement proteins involved in the biosynthesis of phenolics, starches, sugars, alkaloids, vitamins, and edible vaccines, monoclonal antibodies and active fragments thereof, industrial enzymes and active fragments thereof. Suitably, the DNA coding sequence of interest may be selected from a transgene or isolated nucleic acid sequence that is capable of conferring cytoplasmic male sterility to a plant, for example a DNA sequence selected from the petunia mitochondrion *pcf* sequence, *orf107* sequence of sorghum and orf 79 of rice.

The mitochondrion specific promoter is selected from mitochondrial promoter nucleotide sequences, such as ATP6, ATP9, Cob, rrn18, Rps13, Rps19, Cox3, Nad6, Nad9 5' untranslated sequences (promoter region) of tobacco mitochondria, and Arabidopsis mitochondria; and the plastid specific promoter sequence is selected from the group consisting of the RNA polymerase promoter, rpo B promoter element, atpB promoter element, the clpP promoter element, the 16S rDNA promoter element, PrbcL, Prps16, the Prrn16, Prrn-62, Pycf2-1577, PatpB-289, Prps2-152, Prps16-107, Pycf1-41, PatpI-207, PclpP-511, PclpP-173, PaccD-129, PaccD-129 promoter of the tobacco accD gene, the PclpP-53 promoter of the clpP gene, the Prrn-62 promoter of the rrn gene, the Prps16-107 promoter of the rps16 gene, the PatpB/E-290 promoter of the tobacco atpB/E gene, and the PrpoB-345 promoter of the rpoB gene.

In a further aspect of the invention there is provided a method of transforming a plant cell with a DNA of interest via an *Agrobacterium* vector comprising the steps of:
a) introducing into the plant cell at least a first nucleic acid sequence that comprises:
   i) an organellar transgene cassette comprising two origins of replication located at the 5' and 3' ends of the cassette, respectively, at least one DNA sequence of interest encoding a transgene of interest under operative control of an organellar promoter, the organellar promoter being positioned downstream of the origin of replication at the 5' end of the transgene cassette, an organellar terminator and wherein the organellar cassette does not contain left and right flanking sequences; and
   ii) and introducing into the plant cell at least a second nucleic acid sequence comprising a viral Rep gene co-presented on a nuclear cassette comprising a *Rep* gene fused to an organellar transit peptide, wherein the fused peptide is under operational control of a nuclear promoter and a nuclear terminator ; and
   wherein the origin of replication is derived from a geminivirus and the DNA sequences making up the organellar transgene cassette of i) are all located within a left border and a right border on the vector.

Naturally, the skilled addressee will appreciate that the organellar transit peptide of ii) and the Rep gene are as defined herein.

In a still further aspect of the invention there is provided a plant cell obtained according to the plant cell transformation method, above. Further more there is provided a plant cell transformed with a vector, transgene cassette, transgene or isolated DNA sequence as defined herein.

In a still further aspect of the invention there is provided a plant including transformed organelles selected from plant plastids and mitochondria transformed as defined herein. There is also provided a transformed plant organelle as defined herein and a population of transformed plant organelles as defined herein comprised in a plant cell. The population of transformed plant organelles of the invention may be located in plant cells selected from tobacco *(Nicotiana tabacum)* and other *Nicotiana* species, arabidopsis, potato, corn(maize), canola (rape), rice, wheat, barley, brassica sp. such as cauliflower, broccoli (e.g. green and purple sprouting), cabbage (e.g. red, green and white cabbages), curly kale, Brussels sprouts, cotton, algae (e.g. blue green species), lemnospora, or moss (e.g. *physcomitrella patens),* tomato, capsicum, squashes, sunflower, soyabean, carrot, melons, grape vines, lettuce, strawberry, sugar beet, peas, and sorghum. In a preferment, the population of transformed plant organelles are located in plant cells selected from cotton, rice, oilseed Brassica species such as canola, corn(maize) and soyabean.

In yet a further apsect of the invention there is provided a method of producing at least a heterologous or exogenous protein in a plant that comprises:
1) introducing into a regenerable plant cell a vector, transgene cassette, transgene or isolated DNA sequence as defined herein;
2) growing said regenerable plant cell of step 1);
3) selecting a plant cell of 2), wherein the transgene or isolated DNA sequence is integrated into the organellar genome;
4) regenerating a plant from the plant cell of 3); and
5) growing the plant of (7).

In this aspect of the invention, the plant organellar genome is independently selected from that of plant mitochondria and plant plastids.

In a further apsect of the invention there is provided a host cell containing a heterologous polynucleotide or nucleic acid vector as defined herein. The host cell may be a plant cell or a bacterial cell. Typically, the host cell is comprised in a plant as defined herein, a plant part or a plant propagule, or an extract or derivative of a plant or in a plant cell culture.

The teaching of all references cited herein is incorporated in its entirety into the present description. There now follow non-limiting examples and figures illustrating the invention.

### FIGURES

**Figure 1****.** Schematic presentation of wild type Ec86 retron (A), and reshuffled version of the retron for reverse transcription of TNA (B). Constructs with fusion between Ec86 reverse transcriptase and groupII intron-encoded protein (IEP) such as LtrA, RmInt IEP and al2 IEP were used to reverse transcribe TNA-RNA translocated into the organelles (C). A rigid linker and transit peptide (TP) were added to optimise expression and targeting of the fused peptide to corresponding organelles.
**Figure 2****.** GroupII intron-based vectors for TNA-RNA delivery into the plant organelles. TNA was inserted either in domainIV of the intron (A) or flanked by the intron on 5' or 3'- end of the TNA (B). Each construct contains reshuffled retron at 3'-end for reverse transcription of the TNA-RNA into ssDNA. The Ec86 RT-IEP fusion can both translocate TNA into the organelles and perform reverse transcription of the TNA.

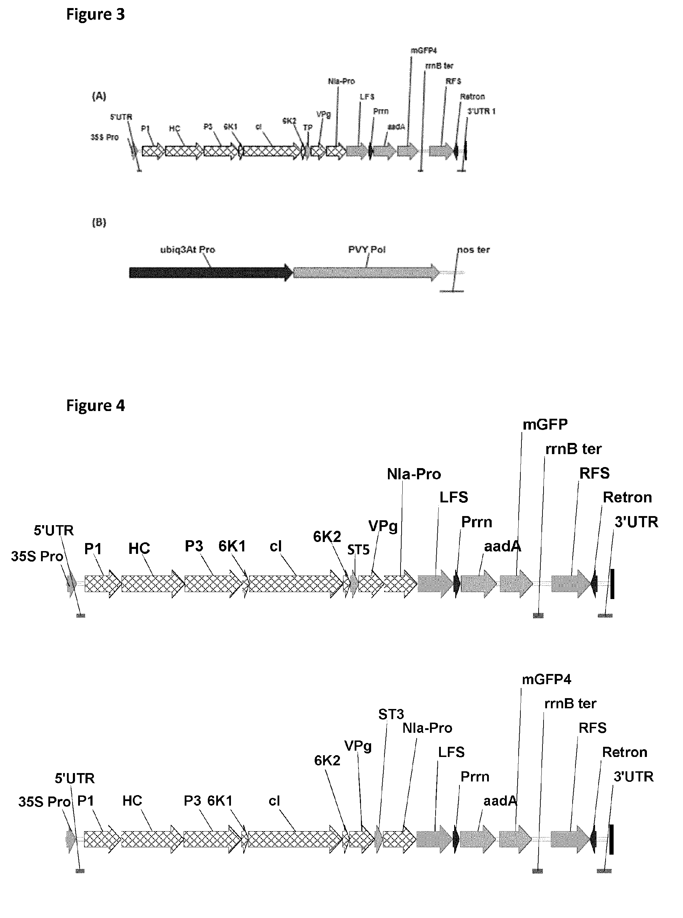

**Figure 3****.** Potato Virus Y (PVY)-base vector for TNA-RNA delivery into the plant organelles. PVY polymerase and coat protein were replaced by TNA with the reshuffled retron at 3'-end (A). Thus the vector contains all viral genes at its 5'-end, and the TNA at the 3'-end. Viral VPg protein was functionally fused with chloroplast or mitochondrial transit peptide (TP) for translocation of viral-TNA RNA covalently linked with VPg to specific organelles. A fusion of 35S promoter and viral sequence provides precise transcription start position. Viral polymerase was delivered in trans under constitutive nuclear promoter (B).
**Figure 4****.** Schematic presentation of modified PVY-based vector where SpyTag sequence was functionally fused either at 5'- (ST5) or 3'-ends (ST3) of the gene encoding VPg protein.
**Figure 5****.** Vectors for overexpression of the SpyCatcher peptide. The SpyCatcher could be expressed either from constitutive nuclear promoter or from inducible promoter, such as DEX -inducible promoter. The SpyCatcher peptide is also fused with chloroplast or mitochondrial transit peptide for translocation of TNA into organelles.
**Figure 6****.** Binary vectors containing modified virD2 gene. A cassette containing *Agrobacterium* virD1 promoter, virD1 gene, cTP- or mTP-virD2 fusion and rrnB terminator was inserted into the pBIN19 binary vector outside of the T-DNA boarders. When the vector delivered into *Agrobacterium,* modified virD2 protein will be produced in bacteria upon induction with acetosyringon.
**Figure 7****.** Binary vectors containing virD2 gene modified by fusion of SpyTag sequence to 5'-(ST5) or 3'-ends (ST3).
**Figure 8****.** Vectors for TNA amplification in the organelles using Geminivirus replication system. Two viral origins of replication are provided on flanks of the TNA from Maize Streak Virus (MOR), Beet Curly Top Virus (BOR), and Tomato Golden Mosaic Virus (TOR). As TNA contains LFS and RFS, amplification of the TNA facilitates quick achievement of homoplasmic state of transformants.
**Figure 9****.** Vectors for generation of autonomous mini-chromosome in the organelles, based on Geminivirus replication system. As the cassettes do not contain LFS and RFS, they will not be inserted in the genome of organelles, but the cassette will be amplified as long as a source of replicase is provided either from the mini-chromosome, or from plant nucleus. MOR- viral origin of replication from maize streak virus, BOR- viral origin of replication from beet top curly virus, TOR- viral origin of replication from tomato golden mosaic virus.
**Figure 10****.** Vectors containing cassette for overexpression of replication initiation protein (Rep) from geminivirus. The Rep gene can be fused to either chloroplast or mitochondrial transit peptides to generate amplification of TNA in organelles.
**Figure 11****.** PCR analysis of spectinomycin resistant plants for insertion of transgene into the chloroplast genome of tobacco (A) and rice (B).
   (A): lane 1-3- OTV1; lanes 4-5-OTV2; lanes 6-7-OTV3: lanes 8-9-OTV4, lanes 10-12-OTV5; lanes 13-14-OTV6.
   (B) : lane 1-WT DNA of rice; lanes 2-5- OTV7; lanes 6-9- OTV8; lanes 10-13- OTV9; lanes 14-15-OTV10, lane 16-negative control.
**Figure 12****.** PCR analysis of spectinomycin resistant plants generated using Potato Virus Y translocation sequence. Lanes 1-4- OTV21; lanes 5-8 - OTV22 + OTV27; lanes 9-12- OTV23 + OTV27.
**Figure 13****.** PCR analysis of transgene flanking sequence using virD2 approach for chloroplast transformation in tobacco. Lanes 1-5-OTV21; lanes 6-7- OTV22 + OTV27; lanes 8-9- OTV23 + OTV27.
**Figure 14****.** Southern analysis for amplification of the TNA in tobacco chloroplasts. (A) lanes 1-4- BCTV-based replicon (OTV33+OTV39);
lanes 5-6- TGMV-based replicon (OTV35+OTV41). (B) lanes 1-8- MSV-based replicon (OTV34+OTV40).
**Figure 15****.** Southern analysis for replication of mini-chromosome in tobacco without insertion into the chloroplast genome. Lanes 1-5-BCTV-based replicon (OTV45+OTV39); lanes 6-10-TGMV-based replicon (OTV46+OTV41).
**Figure 16****.** PCR analysis of flanking sequences for mitochondrial transgene insertion in tobacco (A) and rice (B) using groupII intron and PVY-based translocation sequences.
   (A) Lane 1-DNA of WT tobacco; lanes 2-3-OTV11; lanes 4-6 OTV12; lane7-OTV13, lane 8-OTV14, lanes 9-10- OTV15; lane 11- OTV16; lanes 12-13- OTV24; lanes 14-15- OTV25+OTV28; lane 16- OTV26+OTV28, lane 17- negative control.
   (B) lanes 1-3- OTV17; lanes 4-6- OTV18; lane 7-8- OTV19; lane 9-OTV20, lane 10- negative control.

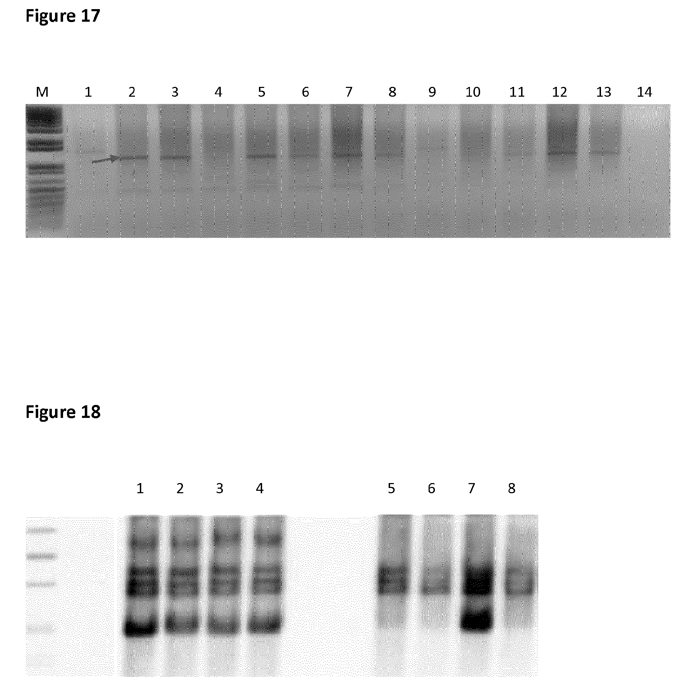

**Figure 17****.** PCR analysis of transgene flanking sequence using virD2 approach for mitochondria transformation in tobacco. Lane 1- DNA of WT tobacco; lanes 2-5- OTV30; lanes 6-9- OTV31+OTV28; lanes 10-13-OTV32+OTV28, lane 14-negative control. The expected size of band indicated by arrow.
**Figure 18****.** Southern analysis of the TNA mini-chromosome amplification in the mitochondria using Geminivirus replication system. Lanes 1-4- BCTV-based replicon (OTV47+OTV42); lanes 5-8-TGMV-based replicon (OTV48+OTV44).

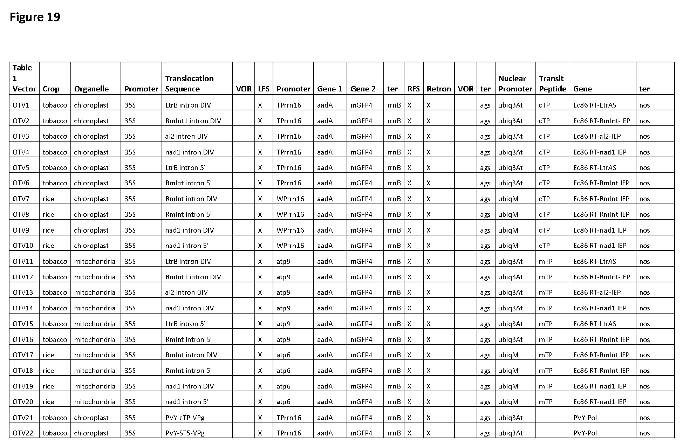

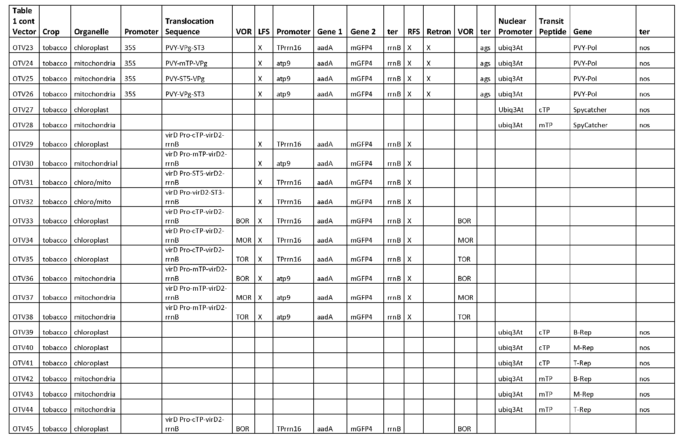

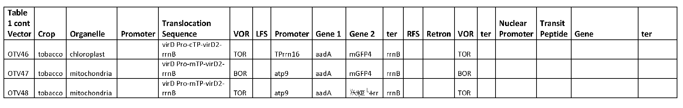

**Figure 19****.** Table of Constructs used in performing the invention and variants 1 and 2

### EXPERIMENTAL SECTION

### Nucleic acid amplification for plant organelle transformation and gene expression in plant organelles.

### Summary.

Sequences employed in the invention are included hereinbelow. Table 1 shows a list of constructs employed in the three variants of the invention.

We have employed a combination of transgene nucleic acid (TNA) delivery and its amplification in the organelle to improve the efficiency of organelle transformation and transgene expression in plant organelles.

The RNA approach for transgene nucleic acid delivery utilised complex and conserved structure of group II introns and reverse transcription of the RNA in the organelles using modified retron-specific reverse transcriptase. Utilisation of the covalent link between VPg protein from Potato Virus A (PVA) or Potato Virus Y and viral RNA with transgene nucleic acid or transgene nucleic acid in combination with the SpyTag-SpyCatcher system also gave rise to efficient delivery of transgene nucleic acid into the plant organelles.

The DNA approach utilised a covalent link between specific protein and transgene nucleic acid to target it to the organelles. Utilisation of virD2 protein directly from Agrobacterium for T-DNA delivery into the organelles is described herein. Improvement of DNA delivery into organelles using a SpyTag-SpyCatcher system is also described herein.

Amplification of transgene nucleic acid in the plant organelle is achieved by utilising the replication system of plant-specific gemini viruses. Placing of the transgene nucleic acid between two viral origins of replication with simultaneous delivery of viral replication initiation protein into the plant organelles was sufficient to amplify transgene nucleic acid located between two viral origins in linear and circular forms of dsDNA, as well as in the circular form of ssDNA. Amplification of transgene nucleic acid allows efficient saturation of the organelle genome with transgene insertion, or efficient transgene expression in the plant organelle from mini-chromosomes generated from the amplification vector.

### Introduction.

Organelle transformation in plants has a great potential for the production of pharmaceuticals in plants, in improving the quality of food, as well as improving environmental stress resistance in plants. However, until the present invention there have been no truly efficient technologies available for organelle transformation in a broad range of crops. To date, only the bombardment method has routinely yielded transformation events in chloroplasts of tobacco, in which, however a few rounds of selection are required to achieve an homoplasmic state of transformation. The bombardment method cannot be used for the transformation of plant mitochondria, because the size of mitochondria is considerably smaller than that of chloroplasts. Thus two problems for organelle transformation needed to be addressed:
(i) delivery of transgenic nucleic acid (TNA) into organelles; and
(ii) amplification of the TNA to facilitate rapid achievement of homoplasmic state of transformants.

We have developed efficient ways for both TNA delivery and amplification to facilitate rapid generation of organelle transformation in a wide range of crops.

### RNA approach for delivery of transgene nucleic acids (TNA) into the organelles.

The RNA approach of the present invention for delivery and insertion of transgene nucleic acid (TNA) into the plant organelle is based on (i) expression of a TNA cassette from the nucleus, (ii) recruiting TNA RNA from the cytoplasm into the organelles, (iii) reverse transcription of the recruited TNA RNA into single stranded DNA (ssDNA) in the organelles, and (iv) insertion of the TNA into the organelle genome using homologous recombination. A traditional vector is used which contains a constitutive nuclear promoter driving a TNA cassette fused with sequences for RNA translocation into the organelle and reverse transcription. Transformation could be achieved by both transient overexpression and stable transformation of the nuclear cassette.

### Reverse transcription of RNA-TNA in the organelles.

In order to generate insertion of the TNA into the organelle genome, RNA containing the TNA is first reverse transcribed into ssDNA. For this purpose we have utilised a retron-based reverse transcription system.

A retron is a distinct DNA sequence found in the genome of many bacteria species that codes for reverse transcriptase and a unique single-stranded DNA/RNA hybrid called multicopy single-stranded DNA (msDNA). Retron msr RNA is the non-coding RNA produced by retron elements and is the immediate precursor to the synthesis of msDNA. The retron *msr* RNA folds into a characteristic secondary structure that contains a conserved guanosine residue at the end of a stem loop. Synthesis of DNA by the retron-encoded reverse transcriptase (RT) results in the DNA/RNA chimera which is composed of a short single-stranded DNA linked to a short single-stranded RNA. The RNA strand is joined to the 5' end of the DNA chain via a 2'-5' phosphodiester linkage that occurs from the 2' position of the conserved internal guanosine residue (Lampson et al., 2005).

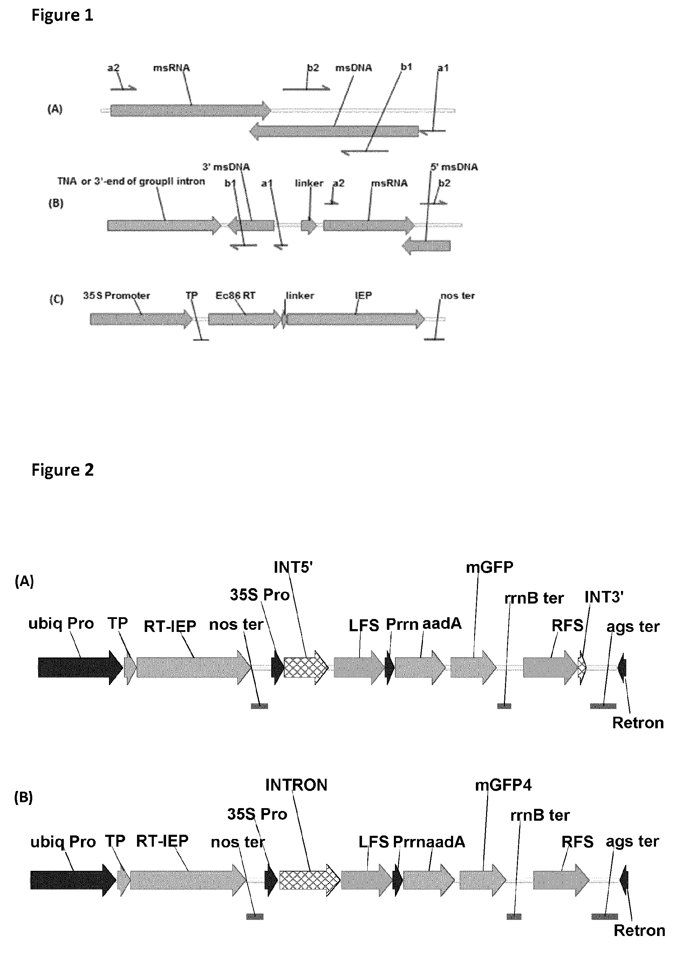

Retron-encoded reverse transcriptase has high efficiency for reverse transcription of fragments of up to 1000bp, but amplification of longer fragments appears to be difficult due to the processivity - that is to say, fragment size limited processing power - of retron-encoded reverse transcriptase. Attempts at improving reverse transcription using reshuffled retrons have been made (Shimamoto et al., 1998, Rozwadowski and Lydiate, 2003), but no successful amplification of fragments longer than 1000bp has been reported. Since chloroplast cassette for delivery of TNA exceeds significantly the length of 1000bp, a more processive or powerful reverse transcriptase had to be engineered. We have optimized a retron-based reverse transcription system by the introduction of a reshuffled retron sequence (Figure 1B) and fusion of this retron reverse transcriptase to a more processive reverse transcriptase encoded by a group II intron, such as LtrA from *Lactococus lactis,* RmInt ORF from *Sinorhizobium meliloti,* and the al2 intron encoded protein from *Saccharomyces cerevisiae* (Figure 1C). The combination of the reshuffled retron with an engineered reverse transcriptase significantly improved reverse transcription of longer fragments. Thus, the combination of RNA delivery to plant organelles with an improved reverse transcription system considerably increased the efficiency of organelle transformation.
**SEQ ID 1**
   **Reshuffled *Ec86* retron** 
Ctgatgctctccgagccaaccaggaaacccgtttttctgacgtaagggtgcgcaactttcgagctcg
cctgctgtgccagccggcgagcgtcgacatgcgcacccttagcgagaggtttatcattaaggtcaacc
tctggatgttgtttcggcatcctgcattgaatctgagttactgtctgttttccttgttggaacggaga
gcatcgctctagagtctc
**SEQ ID 2**
   ***Ec86* RT-LtrA fusion (the linker is in bold italics)** 
atgaaatccgctgaatatttgaacacttttagattgagaaatctcggcctacctgtcatgaacaattt
gcatgacatgtctaaggcgactcgcatatctgttgaaacacttcggttgttaatctatacagctgatt
ttcgctataggatctacactgtagaaaagaaaggcccagagaagagaatgagaaccatttaccaacct
tctcgagaacttaaagccttacaaggatgggttctacgtaacatttagataaactgtcgtcatctcc
tttttctattggatttgaaaagcaccaatctatttaaataatgctaccccgcatattgggcaaact
ttatactgaatattgatttggaggatttttcccaagtttaactgctaacaagtttttggagtgttc
cattctcttggttataatcgactaatatcttcagttttgacaaaaatatgttgttataaaaatctgct
accacaaggtgctccatcatcacctaaattagctaatctaatatgttctaaacttgattatcgtattc
agggttatgcaggtagtcggggcttgatatatacgagatatgccgatgatctcaccttatctgcacag
tctatgaaaaaggttgttaaagcacgtgatttttatttctataatcccaagtgaaggattggttat
 
taactcaaaaaaacttgtattagtgggcctcgtagtcagaggaaagttacaggtttagttatttcac
aagagaaagttgggataggtagagaaaaatataaagaaattagagcaaagatacatcatatatttgc
ggtaagtcttctgagatagaacacgttaggggatggttgtcatttattttaagtgtggattcaaaag
ccataggagattaataacttatattagcaaattagaaaaaaatatggaaagaaccctttaaataaag
cgaagaccggatccaccgtggatgcggcgctggcggcggcagactgcggcggcggcggtcgag
aacatgaagccaacaatggcaatcctcgaacgaatctctaagaactcacaggagaacatcgacgaggt
cttcacaagactttaccgttaccttctccgtcctgacatctactacgtggcatatcagaacctctact
ctaacaagggagcttctacaaagggaatcctcgatgatacagctgatggattctgaggagaagatc
aagaagatcatccaatctttgaaggacggaacttactaccctcagcctgtccgaagaatgtacatcgc
aaagaagaactctaagaagatgagacctcttggaatcccaactttcacagacaagttgatccaggagg
ctgtgagaatcatccttgaatctatctatgagcctgtcttcgaggatgtgtctcacggtttccgacct
cagcgaagctgtcacacagctttgaagacaatcaagagagagttcggaggtgcaagatggttcgtgga
gggagatatcaaggatgcttcgataacatcgaccacgtcacactcatcggactcatcaaccttaaga
tcaaggatatgaagatgagccagttgatctacaagttcctcaaggcaggttacctcgaaaactggcag
taccacaagacttacagcggaacacctcagggcggaatcctctctcctctcctcgctaacatctatct
tcatgaattggacaagttcgttctccaactcaagatgaagttcgaccgagagagtccagagagaatca
cacctgaataccgggagcttcacaacgagatcaaaagaatctctcaccgtctcaagaagttggagggc
gaggagaaggctaaggttctcttggaataccaggagaagaggaagaggttgcctacactcccttgtac
atcacaaacaaacaaggtcttgaagtacgtccgatacgctgacgacttcatcatctctgttaaggaa
gcaaggaggactgtcaatggatcaaggagcaattgaagctcttcatccataacaagctcaagatggaa
ttgagtgaggagaagacactcatcacacatagcagtcagcctgctcgtttcctcggatacgacatccg
agtcaggagaagtggaactatcaagcgatctggaaaggtcaagaagagaacactcaacggagtgtgg
agcttctcatccctctccaagacaagatccgtcaattcatcttcgacaagaagatcgctatccagaag
aaggatagctcatggttcccagttcacaggaagtaccttatccgttcaacagacttggagatcatcac
aatctacaactctgaattgagaggtatctgcaactactacggtctcgcaagtaacttcaaccagctca
actacttcgcttaccttatggaatactcttgcttgaagactatcgcatctaagcataaggaacactc
tcaaagaccatctctatgttcaaggatggaagtggttcttggggaatcccttacgagatcaagcaggg
gaagcagaggagatacttcgccaacttcagtgaatgcaaatctccttaccaattcactgatgagatca
gtcaagctcctgtgctttacggatacgctcggaacactcttgagaacagacttaaggctaagtgttgt
gagctttgtggaacatctgatgagaacacatcttacgagatccaccacgtcaacaaggtcaagaacct
taagggaaaggagaagtgggagatggcaatgatcgctaagcagcggaagactcttgttgtttgcttcc
attgtcatcgtcacgtgatccataagcacaagtga
**SEQ ID 3**
   **Ec86 RT-RmInt IEP fusion** 
atgaaatccgctgaatatttgaacacttttagattgagaaatctcggcctacctgtcatgaacaattt
gcatgacatgtctaaggcgactcgcatatctgttgaaacacttcggttgttaatctatacagctgatt
ttcgctataggatctacactgtagaaaagaaaggcccagagaagagaatgagaaccatttaccaacct
tctcgagaacttaaagccttacaaggatgggttctacgtaacatttagataaactgtcgtcatctcc
tttttctattggatttgaaaagcaccaatctatttaaataatgctaccccgcatattgggcaaact
ttatactgaatattgatttggaggatttttcccaagtttaactgctaacaaagtttttggagtgttc
cattctcttggttataatcgactaatatcttcagttttgacaaaaatatgttgttataaaatctgct
accacaaggtgctccatcatcacctaaattagctaatctaatatgttctaacttgattatcgtattc
agggttatgcaggtagtcggggcttgatatatacgagatatgccgatgatctcaccttatctgcacag
tctatgaaaaaggttgttaaagcacgtgatttttatttctataatcccaagtgaaggattggttat
taactcaaaaaaacttgtattagtgggcctcgtagtcagaggaaagttacaggtttagttatttcac
aagagaaagttgggataggtagagaaaaatataaagaaattagagcaaagatacatcatatatttgc
 
ggtaagtcttctgagatagaacacgttaggggatggttgtcatttattttaagtgtggattcaaaaag
ccataggagattaataacttatattagcaaattagaaaaaaatatggaaagaaccctttaaataaag
cgaagaccggatccaccgtggatgcggcgctggcggcgcagactgcggcggcggcggtcgag
aacatgacttcggaaagtacgacagacaagccgtttcgaattgagaaacgtcgagtgtacgaagctta
caaagcggtcaaagccaaccgtggcgcggccggggtggacggcagacgctggagatatttgagaaag
accttgcagcaaacctctacaagatctggaatcggatgtcctcgggaacctactttccgccgccggtg
cgcgccgtctccattccgaagaaggctggaggcgaaagggttttgggtgtgcccacggtcagcgatcg
gatcgcgcagatggtggtcaagcagatgatcgagccggatttggactccctctttcttccggactcct
acggttacaggccgggaaaatcggccctggatgctgtcggagtgacgcgtcagcggtgctggaagtat
gattgggttttggaattcgacatcaaaggctgtttgacaatcttccgcatgatctcttgctgaaggc
ggtcagaaaagacgtcaaatgcaactgggctctgctctacatcgaaagatggctgactgcgcctatgg
aaaagaacggagaagtcatcgagcggtcacgcggtacccacagggaggcgtggttagcccgatcttg
gcgaatctctttctgcactatgcatttgatctctggatgacgcggacgcatcccgaccttccatggtg
tcgatatgccgacgatggtcttgttcactgccagagcgagcaacaagccgaagccctcagggtggagc
tgagttctcggctggcagcgtgcggacttcagatgcatccgacaaagaccaagattgtctactgcaag
gatcaacggcgcagggaggcgtatccgaatgtcacgttcgactttctcgggtatcagttccggccgcg
acgggtggcgaacacacagcgggacgagttcttctgtggctacacgcctgcggtcagtccgacggcgc
tcaagtcgatgcgggcaacgatcaaaagtttgaacatcccgcggcagacgccggggacgctggccgaa
atagccaaacagctcaatccactccttcggggatggattgcctactatggacggtacagtcgttcggc
cctgtccactctggctgattacgttaatcagaaactcagggcttggatcaggcgaaagttcaaacgct
ttcagtcccataagacacgcgccagcctcttcttgcgaaagctggcgcggaaaatccggggctgttc
gtgcattggaaggcgttcggaacgaacacgtttacctga
**SEQ ID 4**
   **Ec86 RT-al2 IEP fusion** 
atgaaatccgctgaatatttgaacacttttagattgagaaatctcggcctacctgtcatgaacaattt
gcatgacatgtctaaggcgactcgcatatctgttgaaacacttcggttgttaatctatacagctgatt
ttcgctataggatctacactgtagaaaagaaaggcccagagaagagaatgagaaccatttaccaacct
tctcgagaacttaaagccttacaaggatgggttctacgtaacattttagataaactgtcgtcatctcc
tttttctattggatttgaaaagcaccaatctattttaaataatgctaccccgcatattggggcaaact
ttatactgaatattgatttggaggatttttcccaagtttaactgctaacaaagtttttggagtgttc
cattctcttggttataatcgactaatatcttcagttttgacaaaaatatgttgttataaaaatctgct
accacaaggtgctccatcatcacctaaattagctaatctaatatgttctaaacttgattatcgtattc
agggttatgcaggtagtcggggcttgatatatacgagatatgccgatgatctcaccttatctgcacag
tctatgaaaaaggttgttaaagcacgtgatttttatttctataatcccaagtgaaggattggttat
taactcaaaaaaacttgtattagtgggcctcgtagtcagaggaaagttacaggtttagttatttcac
aagagaaagttgggataggtagagaaaaatataaagaattagagcaaagatacatcatatatttgc
ggtaagtcttctgagatagaacacgttaggggatggttgtcatttattttaagtgtggattcaaaaag
ccataggagattaataacttatattagcaaattagaaaaaaatatggaaagaaccctttaaataaag
cgaagaccggatccaccgtggatgcggcgctggcggcggcgcagactgcggcggcggcggtcgag
aacatgccgtttcgcttaatttatcactgtattgaagtgttaattgataacatatctctgtttattc
aattaatgaaaactttaccgtatcatttggttctggttattagtagtaacatacatagtatttagat
acgtaaaccatatggcttacccagttggggccaactcaacggggacaatagcatgccataaaagcgct
ggagtaaaacagccagcgcaaggtaagaactgtccgatggctaggttaacgaattcctgtaaagaatg
tttagggttctcattaactccttcccacttggggattgtgattcatgcttatgtattggaagaagagg
tacacgagttaaccaaaaatgaatcattagctttaagtaaaagttggcatttggagggctgtacgagt
tcaaatggaaaattaagaaatacggggattgtccgaaaggggaaaccctgggggataacggagtcttcat
 
agtacccaaatttaatttaaataaagcgagatactttagtactttatctaaattaaatgcaaggaagg
aagacagtttagcgtatttaacaaagattaatactacggattttccgagttaaataaattaatagaa
aataatcataataaacttgaaaccattaatactagaattttaaaattaatgtcagatattagaatgtt
attaattgcttataataaaattaaaagtaagaaaggtaatatatctaaaggttctaataatattacct
tagatgggattaatatttcatatttaaataaattatctaaagatattaacactaatatgtttaaattt
tctccggttagaagagttgaaattcctaaaacatctggaggatttagacctttaagtgttggaaatcc
tagagaaaaaattgtacaagaaagtatgagaataatattagaattatctataataatagtttctctt
attattctcatggatttagacctaacttatcttgtttaacagctattattcaatgtaaaattatatg
caatactgtaattggtttattaaagtagatttaaataaatgctttgatacaattccacataatatgtt
aattaatgtattaaatgagagaatcaaagataaaggtttcatagacttattatataaattattaagag
ctggatatgttgataaaaataataattatcataatacaactttaggaattcctcaaggtagtgttgtc
agtcctattttatgtaatatttttttagataaattagataaatatttagaaaataaatttgagaatga
attcaatactggaaatatgtctaatagaggtagaaatccaatttataatagtttatcatctaaaattt
atagatgtaaattattatctgaaaaattaaaattgattagattaagagaccattaccaaagaaatatg
ggatccgataaaagttttaaaagagcttattttgttagatatgctgatgatattatcattggtgtaat
gggttctcataatgattgtaaaaatatttaaacgatattaataacttcttaaaagaaaatttaggta
tgtcaattaatatagataaatccgttattaaacattctaaagaaggagttagtttttagggtatgat
gtaaaagttacaccttggaaaaaagaccttatagaatgattaaaaaaggtgataattttattagggt
tagacatcatactagtttagttgttaatgcccctattagaagtattgtaataaaattaaataaacatg
gctattgttctcatggtattttaggaaaacccagaggggttggaagattaattcatgaagaatgaaa
accattttaatgcattacttagctgttggtagaggtattataaactattatagattagctaccaattt
taccacattaagaggtagaattacatacattttattttattcatgttgtttaacattagcaagtaaat
ttaaattaaatactgttaagaaagttattttaaaattcggtaaagtattagttgatcctcattcaaaa
gttagttttagtattgatgattttaaaattagacataaaataaatataactgattctaattatacacc
tgatgaaattttagatagatataaatatatgttacctagatctttatcattatttagtggtatttgtc
aaatttgtggttctaacatgatttagaagtacatcacgtaagaacattaaataatgctgccaataaa
attaaagatgattatttattaggtagaatgattaagataaatagaaaacaaattactatctgtaaaac
atgtcattttaaagttcatcaaggtaaatataatggtccaggtttatag
 **Delivery of transgene nucleic acid to organelle using groupII intron.**
   We utilise groupII introns to deliver RNA of transgene into the organelles. The cassette containing transgene nucleic acid was inserted into domainIV of LtrB intron from *Lactococus lactis,* RmInt1 intron from *Sinorhizobium meliloti,* al2 intron from *Saccharomyces cerevisiae,* tobacco groupII intron from *nad1* gene containing *matK* intron-encoded gene (Figure 1A). The transgenic nucleic acid can be fused at the 5' or 3'-prime ends of the groupII intron (Figure 1B), and is translocated to organelle with the same efficiency as in case when TNA was inserted in domain IV of the groupII intron. We did not observed splicing of the groupII intron in the cytoplasm of the plants and only in environment of the plant organelle intron could be spliced. Thus TNA located at any end of intron can still be translocated to organelles.
**SEQ ID 5**
   ***Lactococcus lactis* LtrB intron (the cloning site for TNA in domain IV is in bold)** 
Gtgcgcccagatagggtgttaagtcaagtagtttaaggtactactctgtaagataacacagaaaacag
ccaacctaaccgaaaagcgaaagctgatacgggaacagagcacggttggaaagcgatgagttacctaa
agacaatcgggtacgactgagtcgcaatgttaatcagatataaggtataagttgtgtttactgaacgc
aagtttctaatttcggttatgtgtcgatagaggaaagtgtctgaaacctctagtacaaagaaaggtaa
gttatggttgtggacttatctgttatcaccacatttgtacaatctgtaggagaacctatgggaacgaa
acgaaagcgatgccgagaatctgaatttaccaagacttaacactaactgggataccctaaacaagaa
tgcctaatagaaaggaggaaaaaggctatagcactagagcttgaaaatcttgcaagggtacggagtac
tcgtagtagtctgagaagggtaacgccctttacatggcaaaggggtacagttattgtgtactaaaatt
aaaaattgattagggaggaaaacctcaaaatgaaaccaacaatggcaatttagaaagaatcagtaaa
aattcacaagaaaatatagacgaagtttttacaagactttatcgttatcttttacgtccagatattta
ttacgtggcgggcgcgccacgcgtgcggccgctgggaaatggcaatgatagcgaaacaacgtaaaact
cttgttgtatgctttcattgtcatcgtcacgtgattcataaacacaagtgaattttacgaacgaaca
ataacagagccgtatactccgagaggggtacgtacggttcccgaagagggtggtgcaaaccagtcaca
gtaatgtgaacaaggcggtacctccctacttcac
**SEQ ID 6**
   ***Sinorhizobium meliloti* RmInt1 intron** 
gtgtgctgcagaggcacggaaggagttcaacatgaactaagaccgtggcgtaaagctgcgtgaatgat
ggggacggccctccgggatcggctttcaggagcgggtctcaaaccagtccgagctgctgcggtaaag
agccgtggtggtgagcgtcggatgaaacgttcggacgagatccgagcaggtgcatgtccaaaagacga
acgaaagtgaaccctccgaggacgcgtcgttatgaacgtaagtgtcgtcgaaaccaggaccgtttcgt
catcctgggacaagtccgccagatgcctgatgaccggcggcggcgaccggcgtagagggggcgtga
gttggacataggctttcacgcggaactgcaggaaccaggctcctgatgtcaagggagaagctcaagcg
gcgcaaaccgcaaggcgagagtaccgatgcaggagactggggcggatcgccccgtatgagcgtcgagg
accctgtaatggggtcggagcaaagggggcggatcaggccgtcgtattgtttgaaacaactggaaaca
ggatgacttcggaaagtacgacagacaagccgtttcgaattgagaaacgtcgagtgtacgaagcttac
aaagcggtcaaagccaaccgtggcgcggccgggtggacggcagacgctggagatatttgagaaagg
gcgcgccacgcgtgcggccgcgccagcctcttcttgcgaaagctggcgcggaaaatccggggctgtt
cgtgcattggaaggcgttcggaacgaacacgtttacctgatgggagcggtgtgaatcgagaggttcac
gcaccgttctgcgagaggccggctggtgaaactcctccggcctactcacc
**SEQ ID 7**
   ***Saccharomyces cerevisiae* al2 intron** 
Gcgccgtttcgcttaatttatcactgtattgaagtgttaattgataaacatatctctgtttattcaat
taatgaaaactttaccgtatcattttggttctgattattagtagtaacatacatagtatttagatacg
taaaccatatggcttacccagttggggccaactcaacggggacaatagcatgccataaaagcgctgga
gtaaaacagccagcgcaaggtaagaactgtccgatggctaggttaacgaattcctgtaaagaatgttt
agggttctcattaactccttcccacttgggattgtgattcatgcttatgtattggaagaagaggtac
acgagttaaccaaaaatgaatcattagctttaagtaaaagttgacatttggagggctgtacgagttca
aatggaaaattaagaaatacgggattgtccgaaaggggaaaccctggggataacggagtcttcatagt
acccaaatttaatttaaataaagcgagatactttagtactttatctaaattaaatgcaaggaaggaag
acagtttagcgtatttaacaaagattaatactacggatttttccgagttaaataaattaatagaaggc
gcgccacgcgtgcggccgcatgattaagataaatagaaaacaaattactatctgtaaaacatgtcatt
ttaaagttcatcaaggtaaatataatggtccaggtttataataattatactccttcggggtcgcc
gcgggggcgggccggactattaaatatgcgttaaatggagagccgtatgatatgaaagtatcacgtac
ggttcggagagggctcttttatatgaatgttattacattcagataggtttgctactctaaa
**SEQ ID 8**
   **Tobacco nad1 intron** 
gtgcggggctttgcatctgacattcgttgggcttctctcttcggagcctgcgccccggcgtttttgt
gcaataaacccctccggccgaagactagtggtaggtggtcctgcggagctttcggaaaagggtagcct
tgtgtgtaagcacagcaatgaaccgcggcgaaccctcagacgacctatctaagattagggggggatcc
tcagtagtggtgaccctttcactcttccacggactgatacatgtaccgaatgctcatacggaaagtt
tactcctgggtctggaacctgggggttgctccgagaaatcctttctttctcgtccactcagggggt
gcggacacacctgcgcggattacaggtgacagttacaagaatggcggggaagttaacagtacccgacg
acattcagggatggatgtagacccatcgggcagggataatcattccggtcctgggagaagtggcgacc
attctcaagaaccaaaaagactgagctgagggaagccctatgagtcactgaaacgacggcaggagtgc
ccttttctatcaatagagggagcaaaaaacgggctttgctcccctttacaatatgaagaaagaaata
agggtcgaagtttagaccgctcacagtagttctacctatagaaaggatcatgaaagaggcgatcagaa
tggtactcgaatccatttacgatctcgagtttccagacacatcgcacttccgctcgggtcgaggcttc
cactccgtcctaagacggggcgcgccacgcgtgcggccgctagagcttgggaagctcggatccggtca
agatccgaacaacaatgagcactcaactactagtaaaaggagaaagttgactttgagaagaaggt
gcttcttgccgctttattagtaagtaagcttgttttatatctcctcaataaaggcgaaagatcactcc
taaaagcaagctttctcttatatacgataccataccacataatttcatttgccttcctgcttaaggca
ctagttcggatgga
**SEQ ID 9**
   **Tobacco matR gene from nad1 intron** 
atgaaagaggcgatcagaatggtactcgaatccatttacgatctcgagtttccagacacatcgcactt
ccgctcgggtcgaggcttccactccgtcctaagacggatcaaagaagagtggggaacctctcgctggt
ttttggaattcgacatcaggaagtgttttcacaccatcgaccgacatcgactcatcccaatctttaag
gaagagatcgacgatcccaagttcttttaccccattcagaaagtcttttccgccggacgactcgtagg
aggtgagaagggcccttactccgtcccacacagtgtattactatcggccctaccaggcaacatctacc
tacacaagctcgatcaggagatagggaggatccgacagaagtacgaaattccgattgttcagagaata
agatcggttctattaagaacaggtcgtattgatgaccaagaaaagtcttccgaagaagcaagcttcaa
cgctccccaagacaacagagccatcattgtggggaggttaaagagcatccaacgcaaagcggcctttc
attcccttgtttcgtcgtggcacacccccccacaagcacccccggctcagggggaccagaaaacg
cctttcgttttccacccttcgtcggcccttgccgccttccttaacaagccctcgagcctcctttgcgc
cgccttcttcatagaagccgccgggtttacccggaagtccgaattctatggtagagaacgctgtaata
ataattgggccatgagagactcttttaagtattgcaaaagaaagggcccgctgatagagctgggcggg
gaggcgatacttgttatcaggtcagagagaggcctggcccgtaagctggccccccttaaaaacctatta
cttaataaggatttgttacgcgcgatatgccgacgacttactactggaatcgtgggttccgtcgagc
ttctcatagaaatacaaaaacgtatcgcccacttcctacaatctggcttgaacctttgggtagactct
gcaggatcaacaaccatagctgcacggagtacggtagaattcctcggtacggtcattcgggaagtccc
tccgagggcgactcccatacaattcttgcgagagctggagaagcgtctacgggtaaagcaccgtatcc
atataactgcttgccacctacgctccgccatccattcaaagtttaggaacctaggtaatagtatcccg
atcaaagagctgacgaaggggatgagcggaacagggagtctactggacgcggttcaactagcggagac
tcttggaacagctggagtaagaagtccccaagtgagcgtcttatgggggccgtcaagcacatacggc
aaggatcaaggagatctcgttgttgcatagctcaggtcggagcaaggtgccatcggacgttcaacag
gtagtctcacgatcgggcactcatgccccgacattgtcattgtatactcccgcgggtcggaaggcggc
gggggaaggaggggacactgggcgagatctatcagcagcgaattccccatacaaatagaggcaccta
tcaaaaagatacttcgaaggcttcggatcgaggtctcattagccgaagaagaccctggccaatccac
gtggcctgcttgacgaacgtcagcgacggagacatcgtaaattggtccgcgggcatcgcgataagtcc
 
tctgtcctactacaggtgctgcgacaacctttaccaagtccgaacgattgtcgaccaccagatccgct
ggtctgcaatattcaccccggcccacaagcacaaatcctcggcgcggaatataatcctaaagtactcc
aaagactcaaatatagtcaatcaagaaggtggtaagacccttgcagagttccccaacagcatagagct
tgggaagctcggatccggtcaagatccgaacaacaatgagcactcaactactagtaaaaaggagaaa
gttga
**SEQ ID 10**
   **Chloroplast Transit Peptide** 
Atggcttcttctgctcaaatacacggtctcggaaccgcttctttctcttccctcaaaaaccctcttc
catatccggcaactccaaaacccttttcttcggtcagcgactcaattccaaccactctcccttcaccc
gcgccgcattccctaaattaagtagcaaaacctttaagaagggtttcactttgagagtt
**SEQ ID 11**
   **Mitochondria Transit Peptide** 
Atggcttctcggaggcttctcgcctctctcctccgtcaatcggctcaacgtggcggcggtctaatttc
ccgatcgttaggaaactccatccctaaatccgcttcacgcgcctcttcacgcgcatcccctaaggat
tcctcttaaaccgcgccgtacagtacgctacctccgcagcggcaccggcatctcagccatca
**SEQ ID 12**
   **Tobacco chloroplast LFS** 
Gcgttcgaactccttcttaaacaacatcgaattaaaccaccatctttccatagagttttcttgccccc
tatttgcatgaaaatacaatagatgaatagtcattcgctataaaattatttatttgaatatcttattt
cctatcagactaagcatagaaatccaatcactaggattattaactaataaggattgtgagtattgaaa
aaaagttctgaatctgggggaacacttcactatatattaatatgttggaacccccttttatattattta
aaataatataattttttaataaagggcggcttctcctatgtcgtgtcaaattcgcatcgaaaaaagaga
tttgtcctctcctataaagaaataaaaaaataattgtttcgtaaaatctcgtctaatactaatatcta
atcactaacaaatctaaaatttaataaaaaaataagtaataaattaaggttctatttcaacacggaac
aaaggggacaatatacaggatgggtagaaagaggtgtgatacttggcttgattcagggaaactacaaa
ctacaggatagaaaagaatataccaatcctaaggatccgtaggattaattgtggatccaagacaacaa
tagaaagatttgag
**SEQ ID 13**
   **Tobacco chloroplast RFS** 
Ctagattttgtatttcaaatcttgtatatctaggtaagtatatacttagtcaaaatatatgcaataga
atctttgttgtattcggctcaatccttttagtaaaagattgggccgagtttaattgcaattcaattaa
gagaacgaaggataattacttgagttctttctccttatccttctttatttcctgctaatttatctgct
aatgtctactgtttttacttatccaaaacgtccactgctgcaaaattaaatacgatctctttccatac
ttcacaagcagcagctagttccgggctccatttgcaagcctcgcgaataatttcattaccttcctgag
caagatcacgtccttcattacgagcttttacacatgcttctagagctactcgattagctacggcacct
ggcgcattaccccaaggatgtcctaaagttcctccaccgaactgtagtacggaatcatccccaaagat
ctcggtcagagcaggcatatgccaaacgtgaatacctcctgaagccacgggtagaacacctggtaaag
agacccaatcttgagtgaaataaataccgcgacttcgatcttgttcaacaaaatcatcacgcagtaaa
tcaacaaagcccaaagttatgtct
**SEQ ID 14**
   **Rice chloroplast LFS** 
Ccgtgtcaatcacttccattcctctcatcaacccatctgtagcactcatagctacagctctaactcga
ttatttcctaataattgttgtacctcacaagttacattaatttgcttaccgtcagtgtctcgactctt
gactaccaaagcattataaatataaggtaacttgcccggggaaaagtgacatccagcacgggtccaa
taatttgatcgatacgccctgtactttttcttcaattgtagaaaccccggacgagaagtagtagga
ttggttctcataattatcacataattttcaaaaaaaggaatttatcgaaattttgatttttttcttg
ttgaataatgccaaatcaacaccaaaaaatatccaaaatccaaaagtcaaaggaaatgaattagt
taattcaataagagagaaaggggaccagcacttgatttcgttgcccaaacgaatcccattcaatcgt
ttactcatggaatgagcccgtcggaaagttcaatcaatctttttttcatatacatttgccttttgta
aacgatttgtgcctactctactttcttatctaggacttcgatatacaaaatatatactactgtgaagc
atagattgctgtcaacagagaattttcgtagtatttaggtatttccactcaaaataagaaaaggggt
ctattaagaacttaataaggattagaagttgatttggggttgcgctatatctattaaagagtatacaa
taaagatggatttggtgaatcaaatccatggtttaataacgaagcatgttaacttaccataacaacaa
c
**SEQ ID 15**
   **Rice chloroplast RFS** 
Tcaattcttatcgaattcctatagtagaattcctatagcatagaatgtacacagggtgtacccattat
atatgaatgaaacatattatatgaatgaaacatattcattaacttaagcatgccccccatttcttta
atgagttgatattaattgaatatcttttttttaagattttgcaaaggtttcatttacgcctaatcca
tatcgagtagaccctgtcgttgtgagaattcttaattcatgagttgtagggagggacgtatgtcacca
caaacagaaactaaagcaagtgttggatttaaagctggtgttaaggattataaattgacttactacac
cccggagtacgaaaccaaggacactgatatcttggcagcattccgagtaactcctcagccgggggttc
cgcccgaagaagcaggggctgcagtagctgccgaatcttctactggtacatggacaactgtttggact
gatggacttaccagtcttgatcgttacaaaggccgatgctatcacatcgagcccgttgttgggagga
taatcaatatatcgcttatgtagcttatccattagacctatttgaagagggttctgttactaacatgt
ttacttccattgtgggtaacgtatttggtttcaaagccctacgcgctctacgtctggaggatctgcga
attccccctacttattcaaaaactttccaaggtccgcctcatggtatccaagttgaaaggataagtt
gaacaaatacggtcgtcctttattggatgtactattaaaccaaaattgggattatctgcaaaaaatt
atggtagagcatgttatgagtgtctacgcggtgg
**SEQ ID 16**
   **rrnB terminator** 
aggcatcaaataaaacgaaaggctcagtcgaaagactgggcctttcgttttatctgttgtttgtcggt
gaacgctctcctgagtaggacaaatccgccc
**SEQ ID 17**
   **aadA gene** 
atgagggaagcggtgatcgccgaagtatcgactcaactatcagaggtagttggcgtcatcgagcgcca
tctcgaaccgacgttgctggccgtacatttgtacggctccgcagtggatggcggcctgaagccacaca
gtgatattgatttgctggttacggtgaccgtaaggcttgatgaaacaacgcggcgagctttgatcaac
gaccttttggaaacttcggcttcccctggagagagcgagattctccgcgctgtagaagtcaccattgt
tgtgcacgacgacatcattccgtggcgttatccagctaagcgcgaactgcaatttggagaatggcagc
 
gcaatgacattcttgcaggtatcttcgagccagccacgatcgacattgatctggctatcttgctgaca
aaagcaagagaacatagcgttgccttggtaggtccagcggcggaggaactctttgatccggttcctga
acaggatctatttgaggcgctaaatgaaaccttaacgctatggaactcgccgcccgactgggctggcg
atgagcgaaatgtagtgcttacgttgtcccgcatttggtacagcgcagtaaccggcaaaatcgcgccg
aaggatgtcgctgccgactgggcaatggagcgcctgccggcccagtatcagcccgtcatacttgaagc
tagacaggcttatcttggacaagaagaagatcgcttggcctcgcgcgcagatcagttggaagaatttg
tccactacgtgaaaggcgagatcaccaaggtagtcggcaaataa
**SEQ ID 18**
   **mGFP4 gene** 
atgagtaaaggagaagaacttttcactggagttgtcccaattcttgttgaattagatggtgatgttaa
tgggcacaaattttctgtcagtggagagggtgaaggtgatgcaacatacggaaaacttacccttaaat
ttatttgcactactggaaaactacctgttccatggccaacacttgtcactactttctcttatggtgtt
caatgcttttcaagatacccagatcatatgaagcggcacgacttcttcaagagcgccatgcctgaggg
atacgtgcaggagaggaccatcttcttcaaggacgacgggaactacaagacacgtgctgaagtcaagt
ttgagggagacaccctcgtcaacaggatcgagcttaagggaatcgatttcaaggaggacggaaacatc
ctcggccacaagttggaatacaactacaactcccacaacgtatacatcatggcagacaaacaaaagaa
tggaatcaaagttaacttcaaaattagacacaacattgaagatggaagcgttcaactagcagaccatt
atcaacaaaatactccaattggcgatggccctgtccttttaccagacaaccattacctgtccacacaa
tctgccctttcgaaagatcccaacgaaaagagagaccacatggtccttcttgagtttgtaacagctgc
tgggattacacatggcatggatgaactatacaaataa
**SEQ ID 19**
   **Tobacco Prrn chloroplast promoter** 
Caatgtgagtttttgtagttggatttgctcccccgccgtcgttcaatgagaatggataagaggctcgt
gggattgacgtgagggggcagggatggctatatttctgggagcgaactccgggcgaatatgaagcgca
tcgatacaagt
**SEQ ID 20**
   **Wheat Prrn chloroplast promoter** 
Caatgtgagtttttctattttgacttactcccccgccacgagcgaacggaatggataagaggcttg
tgggattgacgtgatagggtagggttggctatactgctggtggcgaactccaggctaataatctgaag
cgcatggatacaagttatccttggaaggaaagacaattccgaatctgctttgtctacgaataaggaag
ctataagtaatgcaactatgaatctcatg
**SEQ ID 21**
   **Tobacco atp9 mitochondrial promoter** 
Ggataagtgaaatcgtatgtatccatccatggtgtatctggtgctctcgtatataagagaagggcag
catttatgagtaatcgatctcacaaactatcaatttcataagagaagacgaagacggatcaaattgaa
taatcgaagagagatgggaccctagctacgagtcattccctctgacgtcgaatgatctacttgcttgt
acttctctttgtcgagattcagttggtcttcagtctaccactccgtgggtataagatcgcaaagaatg
cattccaagtgagatgtccaagatcaaaggaacgagggtaagaatcgacgaggaatcaataagatata
agataagtga
**SEQ ID 22**
   **Rice atp6 mitochondrial promoter** 
Acataagccatccgaaaccagtattggaaagtgttcagtttcgttttccattctgaaatgttcatagt
agtatagtatgttttccgttgggtcgacgccatgtgatcgctactaaagatagagtttccttggaaaa
accgaggccagttgagatcagtctccctttctaggagcagagcttaaaaagatgggaaattcc
**SEQ ID 23**
   **Tobacco mitochondrial LFS** 
Tatgtgtggaacctggtctttttcggttccagcctctccctcgaatacatagggtaggtagggctggg
tgagaaatggttccctcttgccaataaactttccccggccttcgattaaccttactcataaagggtct
tacggtcgggagaactacctaactaaagaaaaatagtgttctttctaagagtaggcgtggagagcttt
ttgcggggaaacttgcaagtacagtttgggggaggcgggcgtcgaccctaccttatgagtattcgga
ctataacagttccgatgaacagtcactcacttttgacagttatacgattccagaagatgatccagaat
tgggtcaatcacgtttattagaagtcgacaatagagtggttgtaccagcaaaaagttatatacgtttt
attgtaacatctgctgatgtacctcatagttgggctgtaccttccttaggtgtcaaatgtgatgctgt
acctggtcgtttaaatcagacctctatttcggtacaacgagaaggagtttactatggtcagtgcagtg
agatttgtggaactaatcatgcctttatgcctatcgtcgtagaagctgttcctaggaaagattatggg
tctcgggtatccaatcaattaatcccacaaaccggggaagcttaagcggaaatgaaagaggaggtga
gggaagccactaaattgagggcttcgctcgctcgctctaacgctcgtttagtagacagcgagtggagt
gcataagcccctttagagataggggtgagtactacacgagctcgtaagtaaagtacggaacgagcctt
gtctacgaagcagagcgacctcatcttgcttgcttctggcgaagcttctagctctaaataattggaat
tctggtatggcaggaatactgtcgaccattacgagcgatagcgaagccaagccgtataaaggcgagca
gcccttatagcaatagcaaacggcctacttatagcctat
**SEQ ID 24**
   **Tobacco mitochondrial RFS** 
Caacaggtcagtcaatatcagtaggggtcctcttgcctaacggagtcagcccaacatggacaatgata
ggcagaccaaagatttacgcagtcgttgcgtgcttgctttgcgcaccggcatagcagaattcgaatcc
gctggctcagatgagtggctcttggcttcgtaaacatatctatgttgttgctttttcactaccaatga
gtaggcagctttggatgcttatggagatatggctttggtaaagatctgcttagcgtgtgctttctcgg
gtgctacttagaatagagatagtcagactctaacttgagaatgttatagcgctgtgaaataaggacat
tctgatcgacccgattggctctcgttctggtttggcggaaaggtgaaaagcactaaatctttcttcct
ggttggtgtactagggcgaggcgaatcccaaccccttcgttagctagcttagctttccctcttttcaa
tctatatcagatcctccattacttcttcgccaatacctttagctttcctttagctgctacttttttcc
cagtccacgcccaatcagagtagtcagtgtgcctgctccgtccttctttgacgaaatggatgctgtag
gagaggttgggaaggagggacttcgctaaagatggtctgtctgtgcgcgaggaaggtctttttccttt
ctccttccattgcttgactaggttcgctttgcaaggaagggaaggcatccgtgcaggtagaaaaaggc
ggaggtcaagctatgggcacaaggaggtaaggtatagtaagttacttcttcgtcttttgcttgtcatt
ggattggaagccgcaggcgatgccttcttgcttgtgtagttggccttgcctgcttagtgcggaagtgc
gtaaagtaggctcattctttggtttataaagatcttgtagtagccgaaggtagtccgcttgttagatt
gaattgaatcttatataacaaccggggccttattaattaagagactttatcaatagtataagtggacc
tctcaaaggtataagtagacattagtcttgctggttcgggcggtaaggccctgggtaag
**SEQ ID 25**
   **Rice mitochondrial LFS** 
Ggtcgatacgatatgactaataataccaaatccaggcagaatgagaatatacacctctggatgaccga
agaaccaaaagagatgctggtataatattgggtctccccctcctgcaggatcaaaaaggttgtatta
aagtttcgatcggttaataacattgtaattgcccccgccagtaccggaagtgataataaaagtaggaa
tgctgtcactagaacggaccacacaaaaagtggtaatctatgcatagtcattccaggtccacgcatgt
tgaagatagttgttataaaattgatagaacctaaaattgatgaaatacctgatagatgaagactaaaa
attgctaaatcaactgctcctccagaatggctggtaataccacttaagggcggatagactgtccaccc
agtgccgctgcccacttctactaaggctgagcttaataggagcaagagacttggtggcaacaaccaga
atgatatattatttaatcgtggaaatgccatgtcaggtgcacctatcagaatcggaacaaaccaatta
ccaaatccacctatcatcgccggcataaccataaaaagatcattaaaaagcatgagccgttattaa
aacattataaagttgatgattcccaccaagaatttgatcgccgggtcggctaattccatacgaatca
gtacggagaagcatgtgcccatcactcctgcaatggcaccgaagatgaaatagagagtcccaatatcc
ttgtggttagtagagaagagccatcgaaccatatttgtcatttttatttgagaaatgcaaactttcc
ttatcaaagagggccgggggctggaagagaagaacttgaatactaaacgctggaagagaagaacct
taatactaaaccaagtttcgggaacttcttggtgacttgattggttcccttcccccaatttgcaaagg
atgattcccgtgaaggtgatctcgatcaccattctatgatatttctggatgcttttgag
**SEQ ID 26**
   **Rice mitochondrial RFS** 
Ttccttttacctaatgccggctaccgacaacttacttcatgctattactaacacttatgactgagccg
cacttgctttccaaaagaaatggaaactatcatgcctgagactagccaatagaagaaagagccacaag
caagccatagcagcatcctttttcttcgctttcttcaacaatgcgaatctacctcactcctcatcata
actcaaatacaaattcgagttccaaattgatatttcctcacgtaagcaataaaatgtgaaaccaatat
tcatcatgaaacttcagacactgatgattgtgaggttctggaagagagacgacgtaggctgaaaaaaa
gtaaacagaaaccaccccttaaactcatttgctcaacattctttccacagcaactagaaaagtggag
aaaatccaataagggaggtcccggtgaatacaatcaattggaaaccgaaccccgcattcatgtctc
taacaaggctgtctaagctaagcggccatggacccatggacccggggaatctgaaccattaggtagag
tttcagctgaaagaaaaccaggtcaatcttccgatcgcgagtctttacaagcttgaaacaacttaagc
acaggcgggagtcgccccttttaagtcagtatttatgcggcgctgaactaacgagcggatacctaacc
ttcgaaggagaagaaaagacggatgtatctttcattcatatcgatcagatgtgctttgctcaggactc
ccattttaccattgcttaagccatattacataaagcatagtgagtgatacgcaatgctggtacaccat
gtttttttcctcactctgtgtagccacactcgtttgtccatttctacttattatttatgttaaatagt
atccgttggttgtagaagcactggcgttcagggattgcaaaatccataatatcaagaagcggtaggaa
cctggctaacttcgatgcggataacgcgctgtagaagaaagtggatcaaccaaagtagac
**SEQ ID 27**
   **Ubiq3At Arabidopsis Promoter** 
taccggatttggagccaagtctcataaacgccattgtggaagaaagtcttgagttggtggtaatgtaa
cagagtagtaagaacagagaagagagagagtgtgagatacatgaattgtcggcaacaaaaatcctga
acatcttattttagcaaagagaaagagttccgagtctgtagcagaagagtgaggagaaatttaagctc
ttggacttgtgaattgttccgcctcttgaatacttcttcaatcctcatatattcttcttctatgttac
ctgaaaaccggcatttaatctcgcgggtttattccggttcaacatttttttgttttgagttattatc
tgggcttaataacgcaggcctgaaataaattcaaggcccaactgtttttttttttaagaagttgctgt
taaaaaaaaaaaaaggaattaacaacaacaacaaaaaaagataaagaaaataataacaattacttta
attgtagactaaaaaaacatagattttatcatgaaaaaaagagaaaagaaataaaaacttggatcaaa
aaaaaaacatacagatcttctaattattaactttcttaaaaattaggtcctttttcccaacaattag
gtttagagttttggaattaaaccaaaaagattgttctaaaaaatactcaatttggtagataagtttc
 
cttattttaattagtcaatggtagatactttttttttcttttcttattagagtagattagaatctttt
atgccaagtattgataaattaaatcaagaagataaactatcataatcaacatgaaattaaaagaaaaa
tctcatatatagtattagtattctctatatatattatgattgcttattcttaatgggttgggttaacc
aagacatagtcttaatggaaagaatctttttttgaactttttccttattgattaaattcttctatagaa
aagaaagaaattatttgaggaaaagtatatacaaaaagaaaaatagaaaaatgtcagtgaagcagatg
taatggatgacctaatccaaccaccaccataggatgtttctacttgagtcggtcttttaaaaacgcac
ggtggaaaatatgacacgtatcatatgattccttcctttagtttcgtgataataatcctcaactgata
tcttcctttttttgttttggctaaagatattttattctcattaatagaaaagacggttttgggctttt
ggtttgcgatataaagaagaccttcgtgtggaagataataattcatcctttcgtctttttctgactct
tcaatctctcccaaagcctaaagcgatctctgcaaatctctcgcgactctctctttcaaggtatattt
tctgattctttttgtttttgattcgtatctgatctccaatttttgttatgtggattattgaatctttt
gtataaattgcttttgacaatattgttcgtttcgtcaatccagcttctaaatttgtcctgattacta
agatatcgattcgtagtgtttacatctgtgtaatttcttgcttgattgtgaaattaggattttcaagg
acgatctattcaatttttgtgttttctttgttcgattctctctgttttaggtttcttatgtttagatc
cgtttctctttggtgttgttttgatttctcttacggcttttgatttggtatatgttcgctgattggtt
tctacttgttctattgttttatttcaggt
**SEQ ID 28**
   **35S Promoter** 
Gatctctctgccgacagtggtcccaaagatggacccccacccacgaggagcatcgtggaaaaagaaga
cgttccaaccacgtcttcaaagcaagtggattgatgtgacatctccactgacgtaagggatgacgcac
aatcccactatccttcgcaagacccttcctctatataaggaagttcatttcatttggagagga
**SEQ ID 29**
   **UbiqM maize Promoter** 
tgcagcgtgacccggtcgtgcccctctctagagataatgagcattgcatgtctaagttataaaaatt
accacatatttttttgtcacacttgtttgaagtgcagtttatctatctttatacatatatttaaact
ttactctacgaataatataatctatagtactacaataatatcagtgttttagagaatcatataaatga
acagttagacatggtctaaaggacaattgagtattttgacaacaggactctacagttttatcttttta
gtgtgcatgtgttctcctttttttttgcaaatagcttcacctatataatacttcatccattttattag
tacatccatttagggtttagggttaatggttttatagactaatttttttagtacatctattttattc
tattttagcctctaaattaagaaaactaaaactctattttagtttttttatttaataatttagatata
aaatagaataaaataaagtgactaaaaattaaacaaatacccttaagaaattaaaaaaactaaggaa
acatttttcttgtttcgagtagataatgccagcctgttaaacgccgacgacgagtctaacggacacca
accagcgaaccagcagcgtcgcgtcggccaagcgaagcagacggcacggcatctctgtcgctgcctc
tggacccctgtcgagagttccgctccaccgttggacttgctccgctgtcggcatccagaaattgcgtg
gcggagcggcagacgtgagccggcacggcaggcggcctcctcctctcacggcaccggcagctacg
ggggattcctttcccaccgctccttcgctttcccttcctcgcccgccgtaataaatagacacccctc
cacaccctctttccccaacctcgtgttgttcggagcgcacacacacacaaccagatctcccccaaatc
cacccgtcggcacctccgcttcaaggtacgccgctcgtcctccccccccccccctctctaccttctct
agatcggcgttccggtccatggttagggcccggtagttctacttctgttcatgtttgtgttagatccg
tgtttgtgttagatccgtgctgctagcgttcgtacacggatgcgacctgtacgtcagacacgttctga
ttgctaacttgccagtgtttctctttggggaatcctgggatggctctagccgttccgcagacgggatc
gatttcatgattttttttgtttcgttgcatagggtttggtttgcccttttcctttatttcaatatatg
ccgtgcacttgtttgtcgggtcatcttttcatgcttttttttgtcttggttgtgatgatgtggtctgg
ttgggcggtcgttctagatcggagtagaattaattctgtttcaaactacctggtggatttattaattt
 
tggatctgtatgtgtgtgccatacatattcatagttacgaattgaagatgatggatggaaatatcgat
ctaggataggtatacatgttgatgcgggttttactgatgcatatacagagatgctttttgttcgcttg
gttgtgatgatgtggtgtggttgggcggtcgttcattcgttctagatcggagtagaatactgtttcaa
actacctggtgtatttattaattttggaactgtatgtgtgtgtcatacatcttcatagttacgagttt
aagatggatggaaatatcgatctaggataggtatacatgttgatgtgggttttactgatgcatataca
tgatggcatatgcagcatctattcatatgctctaaccttgagtacctatctattataataaacaagta
tgttttataattattttgatcttgatatacttggatgatggcatatgcagcagctatatgtggatttt
tttagccctgccttcatacgctatttatttgcttggtactgtttcttttgtcgatgctcaccctgttg
tttggtgttacttctgcag
**SEQ ID 30**
   **Nos terminator** 
Gtcaagcagatcgttcaaacatttggcaataaagtttcttaagattgaatcctgttgccggtcttgcg
atgattatcatataatttctgttgaattacgtgaagcatgtaataattaacatgtaatgcatgacgtt
atttatgagatgggtttttatgattagagtcccgcaattatacatttaatacgcgatagaaaacaaaa
tatagcgcgcaaactaggataaattatcgcgcgcggtgtcatctatgttactagatcgac
**SEQ ID 31**
   **Ags terminator**

gaattaacagaggtggatggacagacccgttcttacaccggactgggcgcgggataggatattcagat
tgggatgggattgagcttaaagccggcgctgagaccatgctcaaggtaggcaatgtcctcagcgtcga
gcccggcatctatgtcgagggcattggtggagcgcgcttcggggataccgtgcttgtaactgagaccg
gatatgaggccctcactccgcttgatcttggcaaagatatttgacgcatttattagtatgtgttaatt
ttcatttgcagtgcagtattttctattcgatctttatgtaattcgttacaattaataaatattcaaat
cagattattgactgtcatttgtatcaaatcgtgtttaatggatatttttattataatattgatgat

### Delivery on transgene nucleic acid to organelle using covalent link between viral VPg protein and viral RNA containing transgene nucleic acid.

In order to translocate TNA to the plant organelles, a covalent link between a specific protein and the nucleic acid cassette containing TNA was utilised. It has been shown that some RNA viruses from the genus *Potyvirus* such as Potato Virus A, Potato virus Y and *Sobemovirus* such as Rice Yellow Mottle Virus (RYMV) utilise protein primed replication of their genome. A specific VPg protein is covalently linked to 5'-end of viral RNA and serves as a priming mechanism for replication of the viral genome (Ivanov et al., 2014; Rantalainen et al., 2008; Grzela et al., 2008; Olspert et al., 2011). Formation of this covalent bond also facilitates stabilisation and protection of viral RNA from host endonucleases.

In order to deliver RNA of the TNA into organelles using VPg protein, we used two approaches:

### i) Fusion of VPg protein with organelle transit peptide

In this approach we fused VPg protein with an organelle transit peptide. In this case viral polymerase and coat protein of the complete viral genome were replaced with TNA, while polymerase was delivered in trans (Figure 2A and B). VPg protein within the viral genome was modified by fusion to a chloroplast or mitochondrial transit peptide. In this approach, although TNA was efficiently delivered to the plant organelle, the replication of viral genome was dramatically reduced, as the majority of the VPg protein was translocated to the organelle.

### ii) Use of a SpyTag-SpyCatcher system

To avoid the potential problem of reduced viral replication caused by fusion of transit peptide to VPg protein, we have developed a second approach, where we have utilised the SpyTag-SpyCatcher system (see review by Veggiani et al., 2014). The SpyTag-SpyCatcher system was described by Li et al., 2014, and is based on spontaneous isopeptide bond formation. An isopeptide bond is an amide bond in a protein connecting a side chain to a side chain or a side chain to the protein's main chain. Spontaneous intermolecular isopeptide bond formation between adjacent subunits then locks the rings together, forming 'protein chainmail' (Wikoff et al., 2000). In summary a small peptide of SpyTag (13 aa) is functionally fused to the viral VPg protein at the N- or C-terminus of the protein. Such a short peptide either does not interfere with, or substantially does not appear to interfere with the function of the VPg protein and does not appear to materially affect the efficiency of viral replication. A SpyCatcher peptide is fused to an organelle transit peptide and expressed under a nuclear inducible or nuclear constitutive promoter. The Spycatcher peptide recognises the shorter SpyTag peptide and forms a strong covalent bond between these two proteins. As SpyCatcher is fused to an organellar transit peptide of choice, all complexes between SpyTag-VPg-TNA and SpyCatcher are subsequently translocated to the organelles.

Vectors with both N- and C-terminus fusion of the SpyTag to VPg were prepared (Figure 3). The SpyCatcher sequence was fused to chloroplast or mitochondrial transit peptide under constitutive 35S or inducible DEX promoter (Figure 4).
**SEQ ID 32**
   **Potato Virus Y base vector with chloroplast transit peptide fused to VPg gene** (chloroplast transit peptide is underlined, VPg is presented in bold, cloning site for the TNA is underlined and in bold) 
aaattaaaacaactcaatacaacataagaaaatcaacgcaaaaacactcacaaaagctttcaactcta
attcaaacaatttgttaagtttcaatttcgatcttcatcaaacaaactctttcaatttcagtgtaagc
tatcgtaattcagtaagttatttcaactctcgtaaattgcagaagatcatccatggcaatttacaca
 
tcaacaatccagtttggttccattgaatgcaaacttccatactcacccgctcctttgggctagttgc
ggggaaacgagaagtttcaaccaccactgaccccttcgcaagtttggagatgcagctcagtgcgcgat
tacgaaggcaggagtttgcaactattcgaacatccaagaatggtacttgcatgtatcgatacaagact
gatgtccagattgcgcgcattcaaaagaagcgcgaggaaagagaaagagaggaatataatttccaaat
ggctgcgtcaagtgttgtgtcgaagatcactattgctggtggagagccaccttcaaaacttgaatcac
aagtgcggaggggtgtcatccacacaactccaaggatgcgcacagcaaaaacatatcacacgccaaag
ttgacagagggacaaatgaaccaccttatcaagcaggtgaagcaaattatgtcaaccaaaggagggtc
tgtccaactgattagcaagaaaagtacccatgttcactataaagaagttttgggatcacatcgcgcag
ttgtttgcactgcacatatgagaggtttacgaaagagagtggactttcggtgtgataaatggaccgtt
gtgcgtctacagcatctcgccaggacggacaagtggactaaccaagttcgtgctactgatctacgcaa
gggcgatagtggagttatattgagtaatactaatctcaaaggaaactttgggagaagctcggagggcc
tattcatagtgcgtgggtcgcacgaaggaaaaatctatgatgcacgttccaaggttactcaaggggtt
atggattcaatggttcagttctcaagcgctgaaagcttttggaaggattggacggcaattgggcaca
aatgagatatcctacagatcatacatgtgtggcaggcttaccagttgaagactgtggcagagttgcag
cgataatgacacacagtattttaccgtgctataagattacctgccctacctgtgcccaacaatatgcc
aacttgccagccagtgacttacttaagatattacacaagcacgcaagtgatggtctaaatcgattggg
ggcagacaaagatcgctttgtgcatgtcaaaaagttcttgacaatcttagagcacttaactgaaccgg
ttgatctgagtctagaaattttcaatgaagtattcaagtctataggggagaagcaacaatcacctttc
aaaaacctgaatattctgaataatttctttttgaaaggaaaggaaaatacagctcgtgaatggcaggt
ggctcaattaagcttacttgaattggcaagattccaaaagaacagaacggataatatcaagaaaggag
acatctcgttctttaggaataaactatctgccaaagcaaattggaacttgtatctgtcatgtgataac
cagctggataagaatgcaagcttcctgtggggacagaggaatatcatgctaagcgatttttctcgaa
ctatttcgaggaaattgatccagcgaagggctattcagcatacgaaaatcgtttgcatccgaatggga
caagaaaacttgcaattggaacctaattgtaccacttgatctggctgagtttaggcggaagatgaaa
ggtgattataaaagacagccaggggtgagtaagaagtgcacgagctcgaaggatggaaactacgtgta
tccctgttgttgcactacacttgatgatggctcagctgttgaatcaacatttacccgccaactaaga
agcacctcgtaataggtaatagtggcgaccaaaagtatgttgacttaccaaaagggaattctgagatg
ttatatattgccaggcaaggcttctgttacattaacatttcctcgcgatgttgattaacattagtga
ggaagatgcaaaggatttcactaagaaggttcgtgacatgtgtgtgccaaagcttggaacctggccaa
ccatgatggatctggctacaacttgtgctcaaatgaaaatattctaccctgatgttcatgatgcagaa
ctgcctagaatactagtcgatcacgaaacgcagacatgccatgtagttgactcgtttggctcacaaac
aactgggtatcatattttgaaagcatctagcgtgtcccaacttattttgtttgctaatgatgagttgg
agtctgacattaagcactatagagttggtggtattcctggagcatgccctgagcttgggtccacaata
tcaccttttagagaaggaggaatcataatgtctgagtcagcagcgctaaaactgctcctaaaggaat
ttttaggcccaaagtgatgaagcaattgctactggatgaaccatatttgctcattttatcgatattat
ctcctggtatacttatggctatgtacaacaatgggatatttgagttagcggtgaagttgtggatcaat
gagaaacaatctatagccatgatagcatcgttattgtccgccttggctttacgagtgtcagcagcaga
aacactcgttgcacagaggattataattgacacggcagcaacagatcttctcgatgctacgtgtgatg
gattcaatttaaatctgacatatcccactgcactcatggtgttgcaagttgttaagaacagaaatgaa
tgtgatgatacgttgtttaaagcaggtttttcacattacaacatgagtgtcgtgcagattatggaaaa
aaattatctaagcctcttgggcgatgcctggaaagatttaacctggcgagaaaaattatccgcaacat
ggcactcatacaaagcaaagcgctctatcactcagttcataaaacccataggcaaagcagatttaaaa
gggttgtacaacatatcaccgcaagcattcttgggtcagggcgtacagagagtcaaaggcaccgcctc
agggttgaatgagcgactcaataattatatcaatactaagtgtgtaaatatttcatcctttttcattc
gtagaattttccggcgcttgccaacttttgtaactttcattaattcattattagttattagtatgcta
actagtgtagtagcagtgtgtcaagcaataattctagatcaaaggaagtatagaaaagaaattgagtt
gatgcagattgagaagaatgaaattgtttgtatggagttgtatgcgagtctgcaggtaagtttctgct
tctacctttgatatatatataataattatcattaattagtagtaatataatatttcaaatattttttt
 
caaaataaaagaatgtagtatatagcaattgcttttctgtagtttataagtgtgtatattttaattta
taacttttctaatatatgaccaaaatttgttgatatgcagcgcaaacttgagcgtgaattcacatggg
atgaatatatggaatatttgaaatctgtgaatccccagatagttcaattcgcgcaagctcaaatggaa
gaatataatgtgcgacatcagcgctccacaccaggtgttaagaatttagagcaggtggtagcatttat
aactctaattatcatgatgtttgatgctgaaaggagcgactgtgtattcaagactctcaacaaattca
aaggcatcgtttcttcaatggatcatgaagttaaacaccagtccttggatgatgtaatcaagaatttc
gatgaaaggaacgaagttattgattttgagctaaatgaggatacaattaaaacatcatcagtgttgga
cacgaagtttagcgactggtgggatcggcaaatccaatgggacacacacttccccattatagaactg
agggacacttcatggaattcacaaggcaactgctgtacaagtggccaacgacatcgcgcatagtgag
cacctagactttctagtgagggggagctgttgggtctggaaaatctactggactgcctgtccatctcag
tgcagctggatccgtgcttttgatagaaccaactcgaccacttgcagaaaacgtgttcaagcaattat
ccagtgaaccgtttttcaagaagccaacactgcgcatgcgaggaaatagtgtgtttggttcctctcca
atctccatcatgactagcggctttgcgttgcactactatgctaataatcgctctcagctaactcagtt
taatttcataatttttgatgaatgtcatgttttagatccttctgcaatggcatttcgtagcttgttaa
gtgtgtatcaccaaacatgcaaagtgttaaaggtgtcagccactccagtgggaaggaggtcgagttc
acaacacaacaaccagttaaattggtggttgaggatacactttcattccaatcttttgttgatgcgca
aggctcaaaaaccaatgccgacgttgttcagcatggttcgaacatactcgtgtatgtgtcgagttaca
atgaagtggatacattagccaagcttctaacagataggaatatggtagtctcaaaagttgatggcaga
acaatgaagcacggatgcttagaaattgtaacgaaagggactagtgcaaagccacattttgtcgtagc
aaccaacattattgaaaatggagtaactttagatatagatgtagttgtagatttggacttaaagtct
caccgttttttagatattgacaataggagcattgcatacaataagattagtgttagctatggagaaaga
attcagaggttgggccgtgttgggcgctttaagaagggagtggcattgcgtattggacacaccgaaaa
gggaattattgagattccaagtatgattgctagtgaagctgcgcttgcgtgctttgcatacaatttgc
cagtaatgacaggggggtgtttcaactagcctcattggcaattgtactgttcgtcaagttaaaactatg
caacaatttgagctgagtccattctttatacaaaatttgttgcccatgatggatcaatgcatcctgt
catacatgacattcttaagaagtataaactgcgagattgtatgacgcccttgtgtgatcaatccatac
cttacagagcctcaagcacttggttgtctgttagtgagtacgaacgactcggagtggttttggacatt
ccaaaacagatcaagattgcattccacatcaaggacatccctcctaagttgcatgaaatgctttggga
aacagttatcaaatataaggatgtttgtttgtttccaagtattcgggcttcatccattagcaaaattg
catacacactgcgcactgatcttttttgcaattcccagaaccctaattctagttgaaagattgatcgag
gaggaacgagtgaaacagagtcaattcagaagtctcattgatgaaggatgctcaagcatgttttcaat
tgttaatttaacaaacactcttagagctagatatgcaaaggattacactgcaggtaagtttctgcttc
tacctttgatatatatataataattatcattaattagtagtaatataatatttcaaatatttttttca
aaataaaagaatgtagtatatagcaattgcttttctgtagtttataagtgtgtatattttaatttata
acttttctaatatatgaccaaaatttgttgatatgcagaaaacatacagaagctcgagaaagtgagaa
gtcagttaaaggagttctcaaatttaaatggctctgcatgtgaggagaacttaatgaagaggtatgaa
tctctacagtttgtgcatcatcaagcaacaacttcactcgcaaaggatttgaagttgaaaggagtttg
gaagaagtcattagttgtgcaggacttactcatagcgggtgccgttgctattggtggaatagggctca
tctatagttggtttactcaatcagttgaaactgtgtctcaccagatggcttcttctgctcaaatacac
ggtctcggaaccgcttctttctcttccctcaaaaaccctcttccatatccggcaactccaaaaccct
tttcttcggtcagcgactcaattccaaccactctcccttcacccgcgccgcattccctaaattaagta
gcaaaacctttaagaagggtttcactttgagagttggcaagaacaaatccaaaagaattcaagcattg
aagtttcgacacgcccgcgataagagggctggctttgaaattgataacaatgatgatacaatagagga
attctttggatctgcatacaggaagaagggaaaggtaaaggcaccactgttggtatgggcaagtcaa
gcaggaggtttgttaatatgtatggatttgacccaacagaatattcattcatccagttcgttgatccg
ctcactggagctcaaattgaagagaacgtctatgctgatattagagacatccaagagcgctttagtga
tgtccgcaagaaaatggtagaggatgatgaaatcgaattgcaagcattgggcagcaacacaaccattc
atgcttacttcaggaaagattggtctgacaaggctctaaaaattgatttgatgccacacaacccactc
 
aaaatctgtgataaatcgaatggcattgctaagtttcctgaaagagaacttgagttgaggcaaactgg
gccagcaatagaggttgatgtgaaagacattccaaaacaggaagtggagcatgaagccaaatcactca
tgagaggtttaagggatttcaatccaattgctcaaacagtttgcagagtaaaagtgtctgttgaatat
ggaacgtctgaaatgtatgggttcggttttggtgcgtatattatagtaaaccaccatctattcaagag
cttcaatggatccatggaagtgcgatcaatgcatggaacattcagagtgaagaatttgcatagcttga
gcgttttaccgatcaaaggcagagacattatcatcataaagatgccaaaggatttccctgttttccca
caaaaactgcacttccgagctccagtgcagaatgagaggatttgtttggttggaactaattttcaaga
aaaacatgcatcatcaatcatcacagaaacgagtactacatacaatgtaccgggcagcactttttgga
agcattggattgaaacaaatgatgggcattgtggattaccagtagtgagtacagctgatggatgtcta
gttggaatacacagcttggcgaataatgtgcaaaccacgaattattattcagcctttgatgaggattt
tgaaagtaagtatctccgaactaatgagcataatgagtggaccaaatcgtgggtatataacccagata
ctgtgttgtggggtccattgaagctcaaggagagtacccctaaaggcctgtttaagacaacaaaactt
gtacaggatttaattgatcatgatgttgttgtagagcaatagggcgcgccacgcgtgcggccgcttgt
agtgtctttccggacgatatatagatatttatgtttgcagtaagtattttggcttttcctgtactact
tttatcgcaattaataatcgtttgaatattactggcagataggggtggtatagcgattccgtcgttgt
agtgaccttagctgtcgtttctgtattattatgtttgtataaaagtgccgggttgttgttgttgtggc
tgatctatcgattaggtgatgttgcgatttgtcgtagcagtgactatgtctggatttagttacttggg
tgatgctgtgattctgtcatagcagtgactgtaaacttcaatcaggagaccccgggg
**SEQ ID 33**
   **Potato Virus Y base vector with mitochondrial transit peptide fused to VPg gene** (mitochondrial transit peptide is underlined, VPg is presented in bold, cloning site for the TNA is underlined and in bold) 
aaattaaaacaactcaatacaacataagaaaatcaacgcaaaaacactcacaaaagctttcaactcta
attcaaacaatttgttaagtttcaatttcgatcttcatcaaacaaactctttcaatttcagtgtaagc
tatcgtaattcagtaagttatttcaaactctcgtaaattgcagaagatcatccatggcaatttacaca
tcaacaatccagtttggttccattgaatgcaaacttccatactcacccgctccttttgggctagttgc
ggggaaacgagaagtttcaaccaccactgaccccttcgcaagtttggagatgcagctcagtgcgcgat
tacgaaggcaggagtttgcaactattcgaacatccaagaatggtacttgcatgtatcgatacaagact
gatgtccagattgcgcgcattcaaaagaagcgcgaggaaagagaaagagaggaatataatttccaaat
ggctgcgtcaagtgttgtgtcgaagatcactattgctggtggagagccaccttcaaaacttgaatcac
aagtgcggagggtgtcatccacacaactccaaggatgcgcacagcaaaaacatatcacacgccaaag
ttgacagagggacaaatgaaccaccttatcaagcaggtgaagcaaattatgtcaaccaaaggagggtc
tgtccaactgattagcaagaaaagtacccatgttcactataaagaagttttggatcacatcgcgcag
ttgtttgcactgcacatatgagaggtttacgaaagagagtggactttcggtgtgataaatggaccgtt
gtgcgtctacagcatctcgccaggacggacaagtggactaaccaagttcgtgctactgatctacgcaa
gggcgatagtggagttatattgagtaatactaatctcaaaggaaactttgggagaagctcggagggcc
tattcatagtgcgtgggtcgcacgaaggaaaaatctatgatgcacgttccaaggttactcaaggggtt
atggattcaatggttcagttctcaagcgctgaaagcttttggaagggattggacggcaattgggcaca
aatgagatatcctacagatcatacatgtgtggcaggcttaccagttgaagactgtggcagagttgcag
cgataatgacacacagtattttaccgtgctataagattacctgccctacctgtgcccaacaatatgcc
aacttgccagccagtgacttacttaagatattacacaagcacgcaagtgatggtctaaatcgattggg
ggcagacaaagatcgctttgtgcatgtcaaaaagttcttgacaatcttagagcacttaactgaaccgg
ttgatctgagtctagaaattttcaatgaagtattcaagtctatagggagaagcaacaatcacctttc
aaaaacctgaatattctgaataatttctttttgaaaggaaaggaaaatacagctcgtgaatggcaggt
ggctcaattaagcttacttgaattggcaagattccaaaagaacagaacggataatatcaagaaaggag
 
acatctcgttctttaggaataaactatctgccaaagcaaattggaacttgtatctgtcatgtgataac
cagctggataagaatgcaagcttcctgtggggacagaggaatatcatgctaagcgattttctcgaa
ctatttcgaggaaattgatccagcgaagggctattcagcatacgaaaatcgtttgcatccgaatggga
caagaaaacttgcaattggaaacctaattgtaccacttgatctggctgagtttaggcggaagatgaaa
ggtgattataaaagacagccaggggtgagtaagaagtgcacgagctcgaaggatggaaactacgtgta
tccctgttgttgcactacacttgatgatggctcagctgttgaatcaacatttacccgccaactaaga
agcacctcgtaataggtaatagtggcgaccaaaagtatgttgacttaccaaagggaattctgagatg
ttatatattgccaggcaaggcttctgttacattaacatttcctcgcgatgttgattaacattagtga
ggaagatgcaaaggatttcactaagaaggttcgtgacatgtgtgccaaagcttggaacctggccaa
ccatgatggatctggctacaacttgtgctcaaatgaaaatattctaccctgatgttcatgatgcagaa
ctgcctagaatactagtcgatcacgaaacgcagacatgccatgtagttgactcgtttggctcacaaac
aactgggtatcatatttgaaagcatctagcgtgtcccaacttattttgtttgctaatgatgagttgg
agtctgacattaagcactatagagttggtggtattcctggagcatgccctgagcttgggtccacaata
tcaccttttagagaaggaggaatcataatgtctgagtcagcagcgctaaaactgctcctaaagggaat
tttaggcccaaagtgatgaagcaattgctactggatgaaccatatttgctcattttatcgatattat
ctcctggtatacttatggctatgtacaacaatgggatatttgagttagcggtgaagttgtggatcaat
gagaaacaatctatagccatgatagcatcgttattgtccgccttggctttacgagtgtcagcagcaga
aacactcgttgcacagaggattataattgacacggcagcaacagatcttctcgatgctacgtgtgatg
gattcaatttaaatctgacatatcccactgcactcatggtgttgcaagttgttaagaacagaaatgaa
tgtgatgatacgttgtttaaagcaggttttcacattacaacatgagtgtcgtgcagattatggaaaa
aaattatctaagcctcttgggcgatgcctggaaagatttaacctggcgagaaaaattatccgcaacat
ggcactcatacaaagcaaagcgctctatcactcagttcataaaacccataggcaaagcagatttaaaa
gggttgtacaacatatcaccgcaagcattcttgggtcagggcgtacagagagtcaaaggcaccgcctc
agggttgaatgagcgactcaataattatatcaatactaagtgtgtaaatatttcatccttttcattc
gtagaattttccggcgcttgccaacttttgtaactttcattaattcattattagttattagtatgcta
actagtgtagtagcagtgtgtcaagcaataattctagatcaaaggaagtatagaaaagaaattgagtt
gatgcagattgagaagaatgaaattgtttgtatggagttgtatgcgagtctgcaggtaagtttctgct
tctacctttgatatatatataataattatcattaattagtagtaatataatatttcaaatattttttt
caaaataaaagaatgtagtatatagcaattgcttttctgtagtttataagtgtgtatattttaattta
taacttttctaatatatgaccaaaatttgttgatatgcagcgcaaacttgagcgtgaattcacatggg
atgaatatatggaatatttgaaatctgtgaatccccagatagttcaattcgcgcaagctcaaatggaa
gaatataatgtgcgacatcagcgctccacaccaggtgttaagaatttagagcaggtggtagcatttat
aactctaattatcatgatgtttgatgctgaaggagcgactgtgtattcaagactctcaacaaattca
aaggcatcgtttcttcaatggatcatgaagttaaacaccagtccttggatgatgtaatcaagaatttc
gatgaaaggaacgaagttattgattttgagctaaatgaggatacaattaaaacatcatcagtgttgga
cacgaagtttagcgactggtgggatcggcaaatccaaatggacacacacttccccattatagaactg
agggacacttcatggaattcacaaggcaactgctgtacaagtggccaacgacatcgcgcatagtgag
cacctagactttctagtgagggagctgttgggtctggaaaatctactggactgcctgtccatctcag
tgcagctggatccgtgcttttgatagaaccaactcgaccacttgcagaaaacgtgttcaagcaattat
ccagtgaaccgtttttcaagaagccaacactgcgcatgcgaggaaatagtgtgtttggttcctctcca
atctccatcatgactagcggctttgcgttgcactactatgctaataatcgctctcagctaactcagtt
taatttcataatttttgatgaatgtcatgttttagatccttctgcaatggcatttcgtagcttgttaa
gtgtgtatcaccaaacatgcaaagtgttaaaggtgtcagccactccagtgggaaggaggtcgagttc
acaacacaacaaccagttaaattggtggttgaggatacactttcattccaatcttttgttgatgcgca
aggctcaaaaaccaatgccgacgttgttcagcatggttcgaacatactcgtgtatgtgtcgagttaca
atgaagtggatacattagccaagcttctaacagataggaatatggtagtctcaaaagttgatggcaga
acaatgaagcacggatgcttagaaattgtaacgaaaggactagtgcaaagccacatttgtcgtagc
aaccaacattattgaaaatggagtaactttagatatagatgtagttgtagattttggacttaaagtct
 
caccgtttttagatattgacaataggagcattgcatacaataagattagtgttagctatggagaaaga
attcagaggttgggccgtgttgggcgctttaagaaggagtggcattgcgtattggacacaccgaaaa
gggaattattgagattccaagtatgattgctagtgaagctgcgcttgcgtgctttgcatacaatttgc
cagtaatgacagggggtgtttcaactagcctcattggcaattgtactgttcgtcaagttaaaactatg
caacaatttgagctgagtccattcttatacaaaatttgttgcccatgatggatcaatgcatcctgt
catacatgacattcttaagaagtataaactgcgagattgtatgacgcccttgtgtgatcaatccatac
cttacagagcctcaagcacttggttgtctgttagtgagtacgaacgactcggagtggttttggacatt
ccaaaacagatcaagattgcattccacatcaaggacatccctcctaagttgcatgaaatgctttggga
aacagttatcaaatataaggatgtttgtttgtttccaagtattcgggcttcatccattagcaaaattg
catacacactgcgcactgatctttttgcaattcccagaaccctaattctagttgaaagattgatcgag
gaggaacgagtgaaacagagtcaattcagaagtctcattgatgaaggatgctcaagcatgttttcaat
tgttaatttaacaaacactcttagagctagatatgcaaaggattacactgcaggtaagtttctgcttc
tacctttgatatatatataataattatcattaattagtagtaatataatatttcaaatatttttttca
aaataaaagaatgtagtatatagcaattgcttttctgtagtttataagtgtgtatattttaatttata
acttttctaatatatgaccaaaatttgttgatatgcagaaaacatacagaagctcgagaaagtgagaa
gtcagttaaaggagttctcaaatttaaatggctctgcatgtgaggagaacttaatgaagaggtatgaa
tctctacagtttgtgcatcatcaagcaacaacttcactcgcaaaggatttgaagttgaaaggagtttg
gaagaagtcattagttgtgcaggacttactcatagcgggtgccgttgctattggtggaatagggctca
tctatagttggtttactcaatcagttgaaactgtgtctcaccagatgtatcgtttcgcttctaacctc
gcctccaaggcaaggattgctcaaaacgctcgccaggtttccagcagaatgagctggagcaggaacta
tggcaagaacaaatccaaaagaattcaagcattgaagtttcgacacgcccgcgataagagggctggct
ttgaaattgataacaatgatgatacaatagaggaattctttggatctgcatacaggaagaagggaaaa
ggtaaaggcaccactgttggtatgggcaagtcaagcaggaggtttgttaatatgtatggatttgaccc
aacagaatattcattcatccagttcgttgatccgctcactggagctcaaattgaagagaacgtctatg
ctgatattagagacatccaagagcgctttagtgatgtccgcaagaaaatggtagaggatgatgaaatc
gaattgcaagcattgggcagcaacacaaccattcatgcttacttcaggaaagattggtctgacaaggc
tctaaaaattgatttgatgccacacaacccactcaaaatctgtgataaatcgaatggcattgctaagt
ttcctgaaagagaacttgagttgaggcaaactgggccagcaatagaggttgatgtgaaagacattcca
aaacaggaagtggagcatgaagccaaatcactcatgagaggtttaagggatttcaatccaattgctca
aacagtttgcagagtaaaagtgtctgttgaatatggaacgtctgaaatgtatgggttcggttttggtg
cgtatattatagtaaaccaccatctattcaagagcttcaatggatccatggaagtgcgatcaatgcat
ggaacattcagagtgaagaatttgcatagcttgagcgttttaccgatcaaaggcagagacattatcat
cataaagatgccaaaggatttccctgttttcccacaaaaactgcacttccgagctccagtgcagaatg
agaggatttgtttggttggaactaattttcaagaaaaacatgcatcatcaatcatcacagaaacgagt
actacatacaatgtaccgggcagcacttttggaagcattggattgaaacaaatgatggcattgtgg
attaccagtagtgagtacagctgatggatgtctagttggaatacacagcttggcgaataatgtgcaaa
ccacgaattattattcagcctttgatgaggattttgaaagtaagtatctccgaactaatgagcataat
gagtggaccaaatcgtgggtatataacccagatactgtgttgtggggtccattgaagctcaaggagag
taccccctaaaggcctgtttaagacaacaaaacttgtacaggatttaattgatcatgatgttgttgtag
agcaatagggcgcgccacgcgtgcggccgcttgtagtgtctttccggacgatatatagatatttatgt
ttgcagtaagtattttggcttttcctgtactacttttatcgcaattaataatcgtttgaatattactg
gcagatagggggtggtatagcgattccgtcgttgtagtgaccttagctgtcgtttctgtattattatgt
ttgtataaaagtgccgggttgttgttgtggctgatctatcgattaggtgatgttgcgatttgtcg
tagcagtgactatgtctggatttagttacttgggtgatgctgtgattctgtcatagcagtgactgtaa
acttcaatcaggagaccccgggg
**SEQ ID 34**
   **Potato Virus Y base vector with SpyTag fused to 5'-end of VPg gene** (SpyTag is underlined, VPg is presented in bold, cloning site for the TNA is underlined and in bold 
aaattaaaacaactcaatacaacataagaaatcaacgcaaaacactcacaaaagctttcaactcta
attcaaacaatttgttaagtttcaatttcgatcttcatcaaacaaactctttcaatttcagtgtaagc
tatcgtaattcagtaagttatttcaaactctcgtaaattgcagaagatcatccatggcaatttacaca
tcaacaatccagtttggttccattgaatgcaaacttccatactcacccgctcctttttgggctagttgc
ggggaaacgagaagtttcaaccaccactgacccttcgcaagtttggagatgcagctcagtgcgcgat
tacgaaggcaggagtttgcaactattcgaacatccaagaatggtacttgcatgtatcgatacaagact
gatgtccagattgcgcgcattcaaaagaagcgcgaggaaagagaaagagaggaatataatttccaaat
ggctgcgtcaagtgttgtgtcgaagatcactattgctggtggagagccaccttcaaacttgaatcac
aagtgcggaggggtgtcatccacacaactccaaggatgcgcacagcaaaaacatatcacacgccaaag
ttgacagaggacaaatgaaccaccttatcaagcaggtgaagcaaattatgtcaaccaaaggaggtc
tgtccaactgattagcaagaaaagtacccatgttcactataaagaagttttgggatcacatcgcgcag
ttgtttgcactgcacatatgagaggtttacgaaagagagtggactttcggtgtgataaatggaccgtt
gtgcgtctacagcatctcgccaggacggacaagtggactaaccaagttcgtgctactgatctacgcaa
gggcgatagtggagttatattgagtaatactaatctcaaaggaaactttgggagaagctcggagggcc
tattcatagtgcgtgggtcgcacgaaggaaaaatctatgatgcacgttccaaggttactcaaggggtt
atggattcaatggttcagttctcaagcgctgaaagcttttggaaggattggacggcaattgggcaca
aatgagatatcctacagatcatacatgtgtggcaggcttaccagttgaagactgtggcagagttgcag
cgataatgacacacagtatttaccgtgctataagattacctgccctacctgtgcccaacaatatgcc
aacttgccagccagtgacttacttaagatattacacaagcacgcaagtgatggtctaaatcgattggg
ggcagacaaagatcgctttgtgcatgtcaaaaagttcttgacaatcttagagcacttaactgaaccgg
ttgatctgagtctagaaattttcaatgaagtattcaagtctatagggagaagcaacaatcacctttc
aaaaacctgaatattctgaataatttctttttgaaaggaaaggaaaatacagctcgtgaatggcaggt
ggctcaattaagcttacttgaattggcaagattccaaaagaacagaacggataatatcaagaaaggag
acatctcgttctttaggaataaactatctgccaaagcaaattggaacttgtatctgtcatgtgataac
cagctggataagaatgcaagcttcctgtggggacagaggaatatcatgctaagcgatttttctcgaa
ctatttcgaggaaattgatccagcgaagggctattcagcatacgaaaatcgtttgcatccgaatggga
caagaaaacttgcaattggaaacctaattgtaccacttgatctggctgagtttaggcggaagatgaaa
ggtgattataaaagacagccaggggtgagtaagaagtgcacgagctcgaaggatggaaactacgtgta
tccctgttgttgcactacacttgatgatggctcagctgttgaatcaacatttacccgccaactaaga
agcacctcgtaataggtaatagtggcgaccaaaagtatgttgacttaccaaaagggaattctgagatg
ttatatattgccaggcaaggcttctgttacattaacatttcctcgcgatgttgattaacattagtga
ggaagatgcaaaggatttcactaagaaggttcgtgacatgtgtgtgccaaagcttggaacctggccaa
ccatgatggatctggctacaacttgtgctcaaatgaaaatattctaccctgatgttcatgatgcagaa
ctgcctagaatactagtcgatcacgaaacgcagacatgccatgtagttgactcgtttggctcacaaac
aactgggtatcatatttgaaagcatctagcgtgtcccaacttattttgtttgctaatgatgagttgg
agtctgacattaagcactatagagttggtggtattcctggagcatgccctgagcttgggtccacaata
tcacctttagagaaggaggaatcataatgtctgagtcagcagcgctaaaactgctcctaaaggaat
ttttaggcccaaagtgatgaagcaattgctactggatgaaccatatttgctcatttatcgatattat
ctcctggtatacttatggctatgtacaacaatgggatatttgagttagcggtgaagttgtggatcaat
gagaaacaatctatagccatgatagcatcgttattgtccgccttggctttacgagtgtcagcagcaga
aacactcgttgcacagaggattataattgacacggcagcaacagatcttctcgatgctacgtgtgatg
gattcaatttaaatctgacatatcccactgcactcatggtgttgcaagttgttaagaacagaaatgaa
tgtgatgatacgttgtttaaagcaggtttttcacattacaacatgagtgtcgtgcagattatggaaaa
 
aaattatctaagcctcttgggcgatgcctggaaagatttaacctggcgagaaaattatccgcaacat
ggcactcatacaaagcaaagcgctctatcactcagttcataaaacccataggcaaagcagatttaaaa
gggttgtacaacatatcaccgcaagcattcttgggtcagggcgtacagagagtcaaaggcaccgcctc
agggttgaatgagcgactcaataattatatcaatactaagtgtgtaaatatttcatccttttcattc
gtagaattttccggcgcttgccaacttttgtaactttcattaattcattattagttattagtatgcta
actagtgtagtagcagtgtgtcaagcaataattctagatcaaaggaagtatagaaaagaaattgagtt
gatgcagattgagaagaatgaaattgtttgtatggagttgtatgcgagtctgcaggtaagtttctgct
tctacctttgatatatatataataattatcattaattagtagtaatataatatttcaaatattttttt
caaaataaaagaatgtagtatatagcaattgcttttctgtagtttataagtgtgtatattttaattta
taactttctaatatatgaccaaaatttgttgatatgcagcgcaaacttgagcgtgaattcacatggg
atgaatatatggaatatttgaaatctgtgaatccccagatagttcaattcgcgcaagctcaaatggaa
gaatataatgtgcgacatcagcgctccacaccaggtgttaagaatttagagcaggtggtagcatttat
aactctaattatcatgatgtttgatgctgaaaggagcgactgtgtattcaagactctcaacaaattca
aaggcatcgtttcttcaatggatcatgaagttaaacaccagtccttggatgatgtaatcaagaatttc
gatgaaaggaacgaagttattgattttgagctaaatgaggatacaattaaaacatcatcagtgttgga
cacgaagtttagcgactggtgggatcggcaaatccaaatgggacacacttccccattatagaactg
agggacacttcatggaattcacaaggcaactgctgtacaagtggccaacgacatcgcgcatagtgag
cacctagactttctagtgaggggagctgttgggtctggaaaatctactggactgcctgtccatctcag
tgcagctggatccgtgcttttgatagaaccaactcgaccacttgcagaaaacgtgttcaagcaattat
ccagtgaaccgtttttcaagaagccaacactgcgcatgcgaggaaatagtgtgtttggttcctctcca
atctccatcatgactagcggctttgcgttgcactactatgctaataatcgctctcagctaactcagtt
taatttcataatttttgatgaatgtcatgttttagatccttctgcaatggcatttcgtagcttgttaa
gtgtgtatcaccaaacatgcaaagtgttaaaggtgtcagccactccagtgggaaggaggtcgagttc
acaacacaacaaccagttaaattggtggttgaggatacactttcattccaatcttttgttgatgcgca
aggctcaaaaaccaatgccgacgttgttcagcatggttcgaacatactcgtgtatgtgtcgagttaca
atgaagtggatacattagccaagcttctaacagataggaatatggtagtctcaaaagttgatggcaga
acaatgaagcacggatgcttagaaattgtaacgaaaggactagtgcaaagccacattttgtcgtagc
aaccaacattattgaaaatggagtaactttagatatagatgtagttgtagattttggacttaaagtct
caccgttttttagatattgacaataggagcattgcatacaataagattagtgttagctatggagaaaga
attcagaggttgggccgtgttgggcgctttaagaagggagtggcattgcgtattggacacaccgaaaa
gggaattattgagattccaagtatgattgctagtgaagctgcgcttgcgtgctttgcatacaatttgc
cagtaatgacaggggggtgtttcaactagcctcattggcaattgtactgttcgtcaagttaaaactatg
caacaatttgagctgagtccattctttatacaaaattttgttgcccatgatggatcaatgcatcctgt
catacatgacattcttaagaagtataaactgcgagattgtatgacgcccttgtgtgatcaatccatac
cttacagagcctcaagcacttggttgtctgttagtgagtacgaacgactcggagtggttttggacatt
ccaaaacagatcaagattgcattccacatcaaggacatccctcctaagttgcatgaaatgctttggga
aacagttatcaaatataaggatgtttgtttgtttccaagtattcgggcttcatccattagcaaaattg
catacacactgcgcactgatcttttttgcaattcccagaaccctaattctagttgaaagattgatcgag
gaggaacgagtgaaacagagtcaattcagaagtctcattgatgaaggatgctcaagcatgttttcaat
tgttaatttaacaaacactcttagagctagatatgcaaaggattacactgcaggtaagtttctgcttc
tacctttgatatatatataataattatcattaattagtagtaatataatatttcaaatatttttttca
aaataaaagaatgtagtatatagcaattgcttttctgtagtttataagtgtgtatattttaatttata
acttttctaatatatgaccaaaatttgttgatatgcagaaaacatacagaagctcgagaaagtgagaa
gtcagttaaaggagttctcaaatttaaatggctctgcatgtgaggagaacttaatgaagaggtatgaa
tctctacagtttgtgcatcatcaagcaacaacttcactcgcaaaggatttgaagttgaaaggagtttg
gaagaagtcattagttgtgcaggacttactcatagcgggtgccgttgctattggtggaatagggctca
tctatagttggtttactcaatcagttgaaactgtgtctcaccagggcaagaacaaagcgcatattgtg
atggtggatgcgtataaaccgaccaaaggcaagaacaatccaaaagaattcaagcattgaagtttcg
 
acacgcccgcgataagaggctggctttgaaattgataacaatgatgatacaatagaggaattctttg
gatctgcatacaggaagaagggaaaaggtaaaggcaccactgttggtatgggcaagtcaagcaggagg
tttgttaatatgtatggatttgacccaacagaatattcattcatccagttcgttgatccgctcactgg
agctcaaattgaagagaacgtctatgctgatattagagacatccaagagcgctttagtgatgtccgca
agaaaatggtagaggatgatgaaatcgaattgcaagcattgggcagcaacacaaccattcatgcttac
ttcaggaaagattggtctgacaaggctctaaaattgatttgatgccacacaacccactcaaatctg
tgataaatcgaatggcattgctaagtttcctgaaagagaacttgagttgaggcaaactgggccagcaa
tagaggttgatgtgaaagacattccaaaacaggaagtggagcatgaagccaaatcactcatgagaggt
ttaagggatttcaatccaattgctcaaacagtttgcagagtaaaagtgtctgttgaatatggaacgtc
tgaaatgtatgggttcggttttggtgcgtatattatagtaaaccaccatctattcaagagcttcaatg
gatccatggaagtgcgatcaatgcatggaacattcagagtgaagaatttgcatagcttgagcgtttta
ccgatcaaaggcagagacattatcatcataaagatgccaaaggatttccctgttttcccacaaaact
gcacttccgagctccagtgcagaatgagaggatttgtttggttggaactaattttcaagaaaaacatg
catcatcaatcatcacagaaacgagtactacatacaatgtaccggcagcactttttggaagcattgg
attgaaacaaatgatgggcattgtggattaccagtagtgagtacagctgatggatgtctagttggaat
acacagcttggcgaataatgtgcaaaccacgaattattattcagcctttgatgaggattttgaaagta
agtatctccgaactaatgagcataatgagtggaccaaatcgtgggtatataacccagatactgtgttg
tggggtccattgaagctcaaggagagtacccctaaaggcctgtttaagacaacaaaacttgtacagga
tttaattgatcatgatgttgttgtagagcaatagggcgcgccacgcgtgcggccgcttgtagtgtctt
tccggacgatatatagatatttatgtttgcagtaagtattttggcttttcctgtactacttttatcgc
aattaataatcgtttgaatattactggcagatagggggtggtatagcgattccgtcgttgtagtgacct
tagctgtcgtttctgtattattatgtttgtataaaagtgccgggttgttgttgttgtggctgatctat
cgattaggtgatgttgcgatttgtcgtagcagtgactatgtctggatttagttacttgggtgatgctg
tgattctgtcatagcagtgactgtaaacttcaatcaggagac
**SEQ ID 35**
   **Potato Virus Y base vector with SpyTag fused to 3'-end of fused to VPg gene** (SpyTag is underlined, VPg is presented in bold, cloning site for the TNA is underlined and in bold) 
aaattaaaacaactcaatacaacataagaaaatcaacgcaaaaacactcacaaaagctttcaactcta
attcaaacaatttgttaagtttcaatttcgatcttcatcaaacaaactctttcaatttcagtgtaagc
tatcgtaattcagtaagttatttcaaactctcgtaaattgcagaagatcatccatggcaatttacaca
tcaacaatccagtttggttccattgaatgcaaacttccatactcacccgctccttttgggctagttgc
ggggaaacgagaagtttcaaccaccactgaccccttcgcaagtttggagatgcagctcagtgcgcgat
tacgaaggcaggagtttgcaactattcgaacatccaagaatggtacttgcatgtatcgatacaagact
gatgtccagattgcgcgcattcaaaagaagcgcgaggaaagagaaagagaggaatataatttccaaat
ggctgcgtcaagtgttgtgtcgaagatcactattgctggtggagagccaccttcaaaacttgaatcac
aagtgcggagggtgtcatccacacaactccaaggatgcgcacagcaaaaacatatcacacgccaaag
ttgacagagggacaaatgaaccaccttatcaagcaggtgaagcaaattatgtcaaccaaaggagggtc
tgtccaactgattagcaagaaaagtacccatgttcactataaagaagttttgggatcacatcgcgcag
ttgtttgcactgcacatatgagaggtttacgaaagagagtggactttcggtgtgataaatggaccgtt
gtgcgtctacagcatctcgccaggacggacaagtggactaaccaagttcgtgctactgatctacgcaa
gggcgatagtggagttatattgagtaatactaatctcaaaggaaactttggagaagctcggagggcc
tattcatagtgcgtgggtcgcacgaaggaaaaatctatgatgcacgttccaaggttactcaaggggtt
atggattcaatggttcagttctcaagcgctgaaagcttttggaagggattggacggcaattgggcaca
aatgagatatcctacagatcatacatgtgtggcaggcttaccagttgaagactgtggcagagttgcag
cgataatgacacacagtattttaccgtgctataagattacctgccctacctgtgcccaacaatatgcc
 
aacttgccagccagtgacttacttaagatattacacaagcacgcaagtgatggtctaaatcgattggg
ggcagacaaagatcgctttgtgcatgtcaaaaagttcttgacaatcttagagcacttaactgaaccgg
ttgatctgagtctagaaattttcaatgaagtattcaagtctatagggagaagcaacaatcacctttc
aaaaacctgaatattctgaataatttcttttgaaaggaaaggaaaatacagctcgtgaatggcaggt
ggctcaattaagcttacttgaattggcaagattccaaaagaacagaacggataatatcaagaaaggag
acatctcgttctttaggaataaactatctgccaaagcaaattggaacttgtatctgtcatgtgataac
cagctggataagaatgcaagcttcctgtggggacagagggaatatcatgctaagcgatttttctcgaa
ctatttcgaggaaattgatccagcgaagggctattcagcatacgaaaatcgtttgcatccgaatggga
caagaaaacttgcaattggaaacctaattgtaccacttgatctggctgagtttaggcggaagatgaaa
ggtgattataaaagacagccagggtgagtaagaagtgcacgagctcgaaggatggaaactacgtgta
tccctgttgttgcactacacttgatgatggctcagctgttgaatcaacatttacccgccaactaaga
agcacctcgtaataggtaatagtggcgaccaaaagtatgttgacttaccaaaagggaattctgagatg
ttatatattgccaggcaaggcttctgttacattaacatttcctcgcgatgttgattaacattagtga
ggaagatgcaaaggatttcactaagaaggttcgtgacatgtgtgtgccaaagcttggaacctggccaa
ccatgatggatctggctacaacttgtgctcaaatgaaaatattctaccctgatgttcatgatgcagaa
ctgcctagaatactagtcgatcacgaaacgcagacatgccatgtagttgactcgtttggctcacaaac
aactgggtatcatattttgaaagcatctagcgtgtcccaacttattttgtttgctaatgatgagttgg
agtctgacattaagcactatagagttggtggtattcctggagcatgccctgagcttgggtccacaata
tcaccttttagagaaggaggaatcataatgtctgagtcagcagcgctaaaactgctcctaaagggaat
tttaggcccaaagtgatgaagcaattgctactggatgaaccatatttgctcattttatcgatattat
ctcctggtatacttatggctatgtacaacaatgggatatttgagttagcggtgaagttgtggatcaat
gagaaacaatctatagccatgatagcatcgttattgtccgccttggctttacgagtgtcagcagcaga
aacactcgttgcacagaggattataattgacacggcagcaacagatcttctcgatgctacgtgtgatg
gattcaatttaaatctgacatatcccactgcactcatggtgttgcaagttgttaagaacagaaatgaa
tgtgatgatacgttgtttaaagcaggttttcacattacaacatgagtgtcgtgcagattatggaaaa
aaattatctaagcctcttgggcgatgcctggaaagatttaacctggcgagaaaaattatccgcaacat
ggcactcatacaaagcaaagcgctctatcactcagttcataaaacccataggcaaagcagatttaaaa
gggttgtacaacatatcaccgcaagcattcttgggtcagggcgtacagagagtcaaaggcaccgcctc
agggttgaatgagcgactcaataattatatcaatactaagtgtgtaaatatttcatccttttcattc
gtagaattttccggcgcttgccaacttttgtaactttcattaattcattattagttattagtatgcta
actagtgtagtagcagtgtgtcaagcaataattctagatcaaaggaagtatagaaaagaaattgagtt
gatgcagattgagaagaatgaaattgtttgtatggagttgtatgcgagtctgcaggtaagtttctgct
tctacctttgatatatatataataattatcattaattagtagtaatataatatttcaaatattttttt
caaaataaaagaatgtagtatatagcaattgcttttctgtagtttataagtgtgtatattttaattta
taacttttctaatatatgaccaaaatttgttgatatgcagcgcaaacttgagcgtgaattcacatggg
atgaatatatggaatatttgaaatctgtgaatccccagatagttcaattcgcgcaagctcaaatggaa
gaatataatgtgcgacatcagcgctccacaccaggtgttaagaatttagagcaggtggtagcatttat
aactctaattatcatgatgtttgatgctgaaaggagcgactgtgtattcaagactctcaacaaattca
aaggcatcgtttcttcaatggatcatgaagttaaacaccagtccttggatgatgtaatcaagaatttc
gatgaaaggaacgaagttattgattttgagctaaatgaggatacaattaaaacatcatcagtgttgga
cacgaagtttagcgactggtgggatcggcaaatccaaatgggacacacacttccccattatagaactg
agggacacttcatggaattcacaagggcaactgctgtacaagtggccaacgacatcgcgcatagtgag
cacctagactttctagtgaggggagctgttgggtctggaaaatctactggactgcctgtccatctcag
tgcagctggatccgtgcttttgatagaaccaactcgaccacttgcagaaaacgtgttcaagcaattat
ccagtgaaccgtttttcaagaagccaacactgcgcatgcgaggaaatagtgtgtttggttcctctcca
atctccatcatgactagcggctttgcgttgcactactatgctaataatcgctctcagctaactcagtt
taatttcataatttttgatgaatgtcatgttttagatccttctgcaatggcatttcgtagcttgttaa
gtgtgtatcaccaaacatgcaaagtgttaaaggtgtcagccactccagtgggaaggaggtcgagttc
 
acaacacaacaaccagttaaattggtggttgaggatacactttcattccaatcttttgttgatgcgca
aggctcaaaaaccaatgccgacgttgttcagcatggttcgaacatactcgtgtatgtgtcgagttaca
atgaagtggatacattagccaagcttctaacagataggaatatggtagtctcaaaagttgatggcaga
acaatgaagcacggatgcttagaaattgtaacgaaaggactagtgcaaagccacatttgtcgtagc
aaccaacattattgaaatggagtaactttagatatagatgtagttgtagattttggacttaaagtct
caccgttttagatattgacaataggagcattgcatacaataagattagtgttagctatggagaaaga
attcagaggttgggccgtgttgggcgctttaagaagggagtggcattgcgtattggacacaccgaaaa
gggaattattgagattccaagtatgattgctagtgaagctgcgcttgcgtgctttgcatacaatttgc
cagtaatgacaggggtgtttcaactagcctcattggcaattgtactgttcgtcaagttaaaactatg
caacaatttgagctgagtccattctttatacaaattttgttgcccatgatggatcaatgcatcctgt
catacatgacattcttaagaagtataaactgcgagattgtatgacgcccttgtgtgatcaatccatac
cttacagagcctcaagcacttggttgtctgttagtgagtacgaacgactcggagtggttttggacatt
ccaaaacagatcaagattgcattccacatcaaggacatccctcctaagttgcatgaaatgctttggga
aacagttatcaaatataaggatgtttgtttgtttccaagtattcgggcttcatccattagcaaaattg
catacacactgcgcactgatcttttgcaattcccagaaccctaattctagttgaaagattgatcgag
gaggaacgagtgaaacagagtcaattcagaagtctcattgatgaaggatgctcaagcatgttttcaat
tgttaatttaacaaacactcttagagctagatatgcaaaggattacactgcaggtaagtttctgcttc
tacctttgatatatatataataattatcattaattagtagtaatataatatttcaaatatttttttca
aaataaaagaatgtagtatatagcaattgcttttctgtagtttataagtgtgtatatttaatttata
acttttctaatatatgaccaaaatttgttgatatgcagaaaacatacagaagctcgagaaagtgagaa
gtcagttaaaggagttctcaaatttaaatggctctgcatgtgaggagaacttaatgaagaggtatgaa
tctctacagtttgtgcatcatcaagcaacaacttcactcgcaaaggatttgaagttgaaaggagtttg
gaagaagtcattagttgtgcaggacttactcatagcgggtgccgttgctattggtggaatagggctca
tctatagttggtttactcaatcagttgaaactgtgtctcaccagggcaagaacaaatccaaaagaatt
caagcattgaagtttcgacacgcccgcgataagagggctggctttgaaattgataacaatgatgatac
aatagaggaattctttggatctgcatacaggaagaagggaaaaggtaaaggcaccactgttggtatgg
gcaagtcaagcaggaggtttgttaatatgtatggatttgacccaacagaatattcattcatccagttc
gttgatccgctcactggagctcaaattgaagagaacgtctatgctgatattagagacatccaagagcg
ctttagtgatgtccgcaagaaaatggtagaggatgatgaaatcgaattgcaagcattgggcagcaaca
caaccattcatgcttacttcaggaaagattggtctgacaaggctctaaaaattgatttgatgccacac
aacccactcaaaatctgtgataaatcgaatggcattgctaagtttcctgaaagagaacttgagttgag
gcaaactgggccagcaatagaggttgatgtgaaagacattccaaaacaggaagcgcatattgtgatgg
tggatgcgtataaaccgaccaaagtggagcatgaagccaaatcactcatgagaggtttaaggatttc
aatccaattgctcaaacagtttgcagagtaaaagtgtctgttgaatatggaacgtctgaaatgtatgg
gttcggttttggtgcgtatattatagtaaaccaccatctattcaagagcttcaatggatccatggaag
tgcgatcaatgcatggaacattcagagtgaagaatttgcatagcttgagcgttttaccgatcaaaggc
agagacattatcatcataaagatgccaaaggatttccctgttttcccacaaaaactgcacttccgagc
tccagtgcagaatgagaggatttgtttggttggaactaattttcaagaaaaacatgcatcatcaatca
tcacagaaacgagtactacatacaatgtaccgggcagcacttttggaagcattggattgaaacaaat
gatgggcattgtggattaccagtagtgagtacagctgatggatgtctagttggaatacacagcttggc
gaataatgtgcaaaccacgaattattattcagcctttgatgaggattttgaaagtaagtatctccgaa
ctaatgagcataatgagtggaccaaatcgtgggtatataacccagatactgtgttgtggggtccattg
aagctcaaggagagtaccccctaaaggcctgtttaagacaacaaaacttgtacaggatttaattgatca
tgatgttgttgtagagcaatagggcgcgccacgcgtgcggccgcttgtagtgtctttccggacgatat
atagatatttatgtttgcagtaagtattttggcttttcctgtactacttttatcgcaattaataatcg
tttgaatattactggcagatagggggtggtatagcgattccgtcgttgtagtgaccttagctgtcgttt
ctgtattattatgtttgtataaaagtgccgggttgttgttgttgtggctgatctatcgattaggtgat
 
gttgcgatttgtcgtagcagtgactatgtctggatttagttacttgggtgatgctgtgattctgtcat
agcagtgactgtaaacttcaatcaggagac
**SEQ ID 36**
   **Potato Virus Y polymerase gene** 
atggctaaacattctgcgtggatgtatgaggctctaacagggaatttgcaagctgtggcgacaatgaa
gagtcagctagtgacaaagcacgtggtcaaggggagtgtcggcacttcaaagagttcttaactgtgg
attcggaagcagaagctttcttcaggcctttgatggatgcttatggaagagcttgttaaatagagaa
gcatatataaaggacataatgaaatactcaaagcctattgatgttggaatagtagactgtgatgcttt
tgaagaggctatcaatagggttatcatttatctgcaagtgcatggcttccagaaatgcaattacatca
ccgatgagcaggaaattttcaaagctctcaatatgaaagctgctgtcggagctatgtatggaggcaag
aagaaagactacttcgagcattttactgaggcggataaagaggaaattgttatgcaagttgctttcg
attgtacaaggctcgcttggcatatggaatggatcattgaaagcagaacttcggtgcaaagagaaga
tacttgcaaataagacaaggacattcactgctgcacctttagatactctactgggtggaaaggtgtgc
gttgatgattttaataatcaattctactcaaagaacattgaatgctgctggactgttggaatgactaa
gttttatggaggttgggacaaattgcttcggcgtctacctgaaaattgggtgtactgcgatgccgatg
gttcacaattcgatagttcactcaccccatacctaattaatgctgttctcatcatcagaagcacatac
atggaagattggacttggggttgcaaatgttgcgcaatttgtacacagaaataatttacacaccaat
ctcaactccagatggaacaattgtcaagaagtttagaggtaataatagcggtcaaccttctaccgttg
tggataattctctcatggttgtccttgctatgcattacgctctcattaaggagtgcgttgagtttgaa
gaaatcgacagcacgtgtgtattctttgttaatggtgatgacttattgattgctgtgaatccggagaa
agagagcattctcgatagaatgtcacaacatttctcagatcttggtttgaactatgatttttcgtcga
gaacaagaaggaaggaggaattgtggttcatgtcccatagaggcctgctaatcgaggatatgtacgtg
ccaaagcttgaagaagagagaattgtatccattctgcaatggatagagctgatctgccagagcacag
attagaagcgatttgtgcagcaatgatagaatcctggggttattttgagttaacgcaccaatcagga
gattctactcatggttgttgcaacagcaacctttttcaacgatagcacaggaaggaaaagctccatac
atagcgagcatggcattgaagaagctgtacatgaataggacagtagatgaggaggaactgaaggcttt
cactgaaatgatggttgccttggatgatgaatttgagtgcgatacttatgaagtgcaccatcaatag
**SEQ ID 37**
   **SpyTag**
   gcgcatattgtgatggtggatgcgtataaaccgaccaaa
**SEQ ID 38**
   **SpyCatcher**

atggttgataccttatcaggtttatcaagtgagcaaggtcagtccggtgatatgacaattgaagaaga
tagtgctacccatattaaattctcaaaacgtgatgaggacggcaaagagttagctggtgcaactatgg
agttgcgtgattcatctggtaaaactattagtacatggatttcagatggacaagtgaaagatttctac
ctgtatccaggaaaatatacatttgtcgaaaccgcagcaccagacggttatgaggtagcaactgctat
tacctttacagttaatgagcaaggtcaggttactgtaaatggcaaagcaactaaaggtgacgctcata
tttaa

### DNA approach for delivery of transgene nucleic acid into the organelles.

We have developed a simple and reliable system for DNA delivery into plant organelles using *Agrobacterium* mediated transformation. It has been shown in the past that the virD2 protein is covalently linked with T-DNA in bacterial cells, forming a complex which is then injected into the cytoplasm of the plant cell. At the same time, Agrobacterium injects virE2 protein into the cytoplasm which binds to the T-DNA protecting it from degradation by plant endonucleases, as well as facilitating delivery of the T-DNA into the cell nucleus.

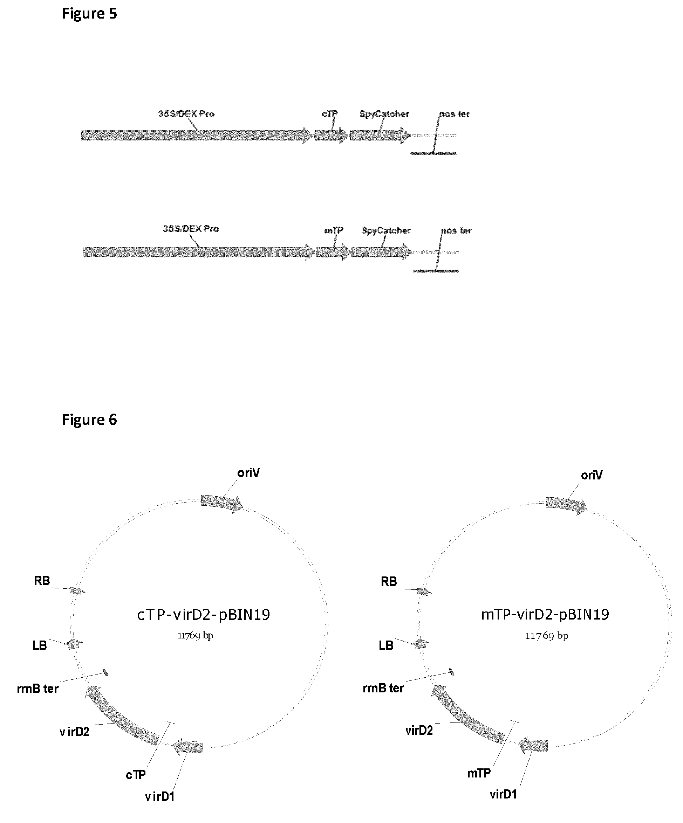

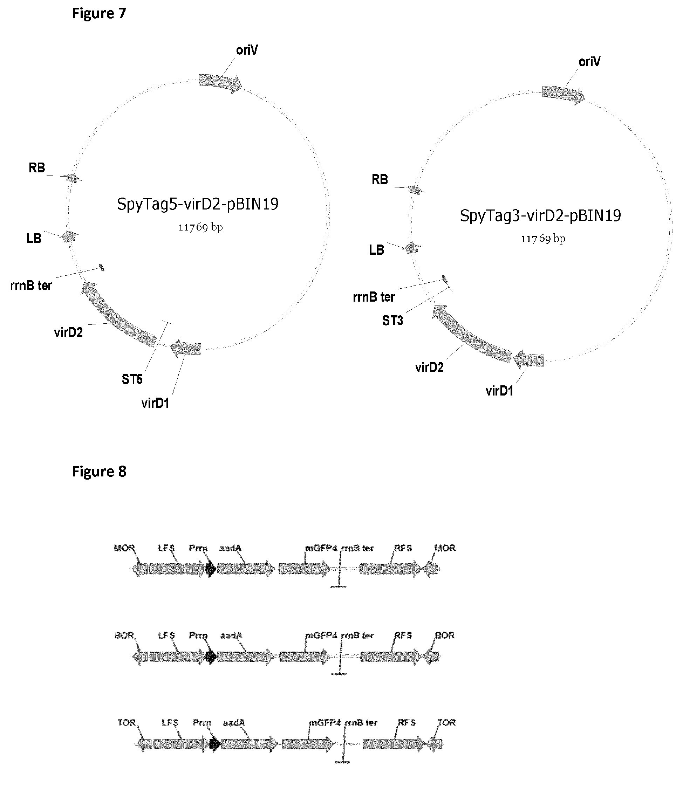

We have utilised an Agrobacterium strain where both the virD2 and virE2 gene native functionality was compromised or substantially reduced and/or substantially knocked out so as to inhibit or diminish nuclear transport of the T-DNA to the plant cell nucleus. To replace the functions of bacterial virD2 protein, we modified the virD2 protein by fusing it with organellar transit peptides, such as chloroplast and mitochondrial transit peptides, or by fusing it with a SpyTag peptide, and have introduced such modified virD2 cassettes on a binary vector under the control of a native bacterial promoter (Figures 6 and 7). As a result, the virD2 modified proteins form a covalent complex with T-DNA in the bacterial cell which is then injected into the cytoplasm of the plant cell. The virD2 protein fused with either chloroplast or mitochondrial transit peptide directs delivery of the T-DNA to the organelles instead of the nucleus. The absence of significant virE2 protein functionality also facilitates more efficient translocation of the T-DNA complex to the plant organelles. The SpyTag-SpyCatcher system can also be utilised for translocating T-DNA into the organelles by overexpression of the Transit Peptide-SpyCatcher peptide in plant cells before challenging of the plant cells with Agrobacterium containing virD2-SpyTag gene on the binary vector.
**SEQ ID 39**
   **cTP virD2 cassette** (chloroplast transit peptide is underlined, virD2 is in bold) 
ctgtcgattttgtgaagcggaagtgtgtctgtacttttatttgtgtgtatgattttgcgataattcat
aagtaatgtagtaattacctgattttatatttcaattttattgtaatataatttcaattgtaataata
taaaaataaatatcccttatgtgttcttgatttcgttttgtatatggctagattcccatctgccacga
cgaggaaatgctacggcggggcaagttcagatctttccgtcttctatggaggaagctatgtcgcaagg
cagtaggcccacctcaagtgacattgccgtcaaccagcgcgaatgcgtgaaggttgaaggcttcaagg
tcgtcagtacccgattaagatcggccgaatatgagagtttttctcatcaggcacgcttgctgggcctc
tccgacagcatggccatacgggttgcggtgcgccgcattggtggctttcttgaaatcgacgcagagac
tcgtcataggatggaggccatactacaatccataggaacactctcaagcaacattgccgcgctgctat
ctgcctatgccgaaaatccgacaatggatttggaggctttgcgagctgaacgtatcgccttcggtaaa
tctttcgctgacctcgacggcttgctccgttccattttgtccgtatcacggcggcggatcgacggttg
ctcgctgctgaaagacgccttgtagcactgacgtagcacttggcggggaacatattcgatggcttctt
ctgctcaaatacacggtctcggaaccgcttctttctcttccctcaaaaaccctcttccatatccggc
aactccaaaaccctttttcttcggtcagcgactcaattccaaccactctcccttcacccgcgccgcatt
ccctaaattaagtagcaaaacctttaagaagggtttcactttgagagttatgcccgatcgtgctcaag
 
ttatcattcgcattgtgccgggaggtggcaccaagacccttcaacaaattatcaatcagttggagtat
ctatcccggaagggcaggctggagctgcagcgttcagcccgacatctcgatattcccctgccaccgga
tcaaatccacgaacttgcccgaagctgggttcaagagactggaacttatgacgaaagtcagccagacg
aggaaaggcaacaggagttgaccacccatattattgttagcttccccgccggtacaagccaggtagcg
gcttatgcggcgagccgggagtgggcagccgagatgtttgggtcaggcgcagggggggccgatacaa
ctatcttacggccttccacatcgatcgcgaccacccacatctgcatgtcgtcgtcaatcggcgcgaac
ttttaggacacggctggctgaagatatctcggcgccatccccaactgaattacgacgccctgcgcata
aagatggccgagatttcacttcgtcatggcattgccctcgatgcgagccgacgagcagaacgtggcat
caccgagcggccgatcacttatgcccaatatcggcgccttgagcgggagcaggctcgccaaatccgtt
tcgaagacgcggatttggaacagtcgtcgccgcaaggagatcatccagagttcagccaacctttcgat
acatccccatttgaagcatccgcaggcggaccggaggacatgcctcggcccaacaatcggcagaatga
gtcgcaagttcatctccaggagccagctggtgtcagcaacgaagccggtgtccttgtgcgggttgcat
tggagacggagcgccttgctcaaccattcgtttccgaaaccattctcgcggacgacatagggagcggc
tcttcgcgtgttgccgagggccgtgtggagagcgcaaaccgcactcccgatattcctcgcgcagcaac
tgaagctgccacgcacacgacacacgaccggcagcggcgtgcaaagcgtcctcatgatgacgacggag
ggccgagtggagcaaaacgtgtgacattggaaggcatcgcggttggcccccaggcgaacgccggcgaa
caggctggcagtagtggccccttagtacggcaagctggaacgtctcggccatctccaccgacggccac
gacgcgggccagcaccgcaaccgcttcattgtctgctacagcccacctccagcaacggagaggtgtcc
tttcaaagcgtccgcgtgaagatgatgatggagaaccgagtgaacgcaaacgcgagagagatgagcgc
agcaaggacgggcgtgggggaaataggagatag gagcttcgacaggcatcaaataaaacgaaaggctc
agtcgaaagactgggcctttcgttttatctgttgtttgtcggtgaacgctctcctgagtaggacaaat
ccgccc
**SEQ ID 40**
   **mTP-virD2 casette** (mitochondrial transit peptide is underlined, virD2 is in bold) 
ctgtcgattttgtgaagcggaagtgtgtctgtacttttatttgtgtgtatgattttgcgataattcat
aagtaatgtagtaattacctgattttatatttcaattttattgtaatataatttcaattgtaataata
taaaaataaatatcccttatgtgttcttgatttcgttttgtatatggctagattcccatctgccacga
cgaggaaatgctacggcggggcaagttcagatctttccgtcttctatggaggaagctatgtcgcaagg
cagtaggcccacctcaagtgacattgccgtcaaccagcgcgaatgcgtgaaggttgaaggcttcaagg
tcgtcagtacccgattaagatcggccgaatatgagagtttttctcatcaggcacgcttgctgggcctc
tccgacagcatggccatacgggttgcggtgcgccgcattggtggctttcttgaaatcgacgcagagac
tcgtcataggatggaggccatactacaatccataggaacactctcaagcaacattgccgcgctgctat
ctgcctatgccgaaaatccgacaatggatttggaggctttgcgagctgaacgtatcgccttcggtaaa
tctttcgctgacctcgacggcttgctccgttccattttgtccgtatcacggcggcggatcgacggttg
ctcgctgctgaaagacgccttgtagcactgacgtagcacttggcggggaacatattcgatgtatcgtt
tcgcttctaacctcgcctccaaggcaaggattgctcaaaacgctcgccaggtttccagcagaatgagc
tggagcaggaactatatgcccgatcgtgctcaagttatcattcgcattgtgccgggaggtggcaccaa
gacccttcaacaaattatcaatcagttggagtatctatcccggaagggcaggctggagctgcagcgtt
cagcccgacatctcgatattcccctgccaccggatcaaatccacgaacttgcccgaagctgggttcaa
gagactggaacttatgacgaaagtcagccagacgaggaaaggcaacaggagttgaccacccatattat
tgttagcttccccgccggtacaagccaggtagcggcttatgcggcgagccgggagtggcagccgaga
tgtttgggtcaggcgcagggggggccgatacaactatcttacggccttccacatcgatcgcgaccac
ccacatctgcatgtcgtcgtcaatcggcgcgaacttttaggacacggctggctgaagatatctcggcg
ccatccccaactgaattacgacgccctgcgcataaagatggccgagatttcacttcgtcatggcattg
ccctcgatgcgagccgacgagcagaacgtggcatcaccgagcggccgatcacttatgcccaatatcgg
 
cgccttgagcgggagcaggctcgccaaatccgtttcgaagacgcggatttggaacagtcgtcgccgca
aggagatcatccagagttcagccaacctttcgatacatccccatttgaagcatccgcaggcggaccgg
aggacatgcctcggcccaacaatcggcagaatgagtcgcaagttcatctccaggagccagctggtgtc
agcaacgaagccggtgtccttgtgcgggttgcattggagacggagcgccttgctcaaccattcgtttc
cgaaaccattctcgcggacgacatagggagcggctcttcgcgtgttgccgagggccgtgtggagagcg
caaaccgcactcccgatattcctcgcgcagcaactgaagctgccacgcacacgacacacgaccggcag
cggcgtgcaaagcgtcctcatgatgacgacggagggccgagtggagcaaaacgtgtgacattggaagg
catcgcggttggcccccaggcgaacgccggcgaacaggctggcagtagtggccccttagtacggcaag
ctggaacgtctcggccatctccaccgacggccacgacgcgggccagcaccgcaaccgcttcattgtct
gctacagcccacctccagcaacggagaggtgtcctttcaaagcgtccgcgtgaagatgatgatggaga
accgagtgaacgcaaacgcgagagagatgagcgcagcaaggacggcgtgggggaaataggagatagg
agcttcgacaggcatcaaataaaacgaaaggctcagtcgaaagactgggcctttcgttttatctgttg
tttgtcggtgaacgctctcctgagtaggacaaatccgccc
**SEQ ID 41**
   **SpyTag-virD2 cassette** (SpyTag is underlined, virD2 is in bold) 
ctgtcgatttgtgaagcggaagtgtgtctgtacttttatttgtgtgtatgattttgcgataattcat
aagtaatgtagtaattacctgattttatatttcaattttattgtaatataatttcaattgtaataata
taaaaataaatatcccttatgtgttcttgatttcgttttgtatatggctagattcccatctgccacga
cgaggaaatgctacggcggggcaagttcagatctttccgtcttctatggaggaagctatgtcgcaagg
cagtaggcccacctcaagtgacattgccgtcaaccagcgcgaatgcgtgaaggttgaaggcttcaagg
tcgtcagtacccgattaagatcggccgaatatgagagtttttctcatcaggcacgcttgctgggcctc
tccgacagcatggccatacgggttgcggtgcgccgcattggtggctttcttgaaatcgacgcagagac
tcgtcataggatggaggccatactacaatccataggaacactctcaagcaacattgccgcgctgctat
ctgcctatgccgaaaatccgacaatggatttggaggctttgcgagctgaacgtatcgccttcggtaaa
tctttcgctgacctcgacggcttgctccgttccattttgtccgtatcacggcggcggatcgacggttg
ctcgctgctgaaagacgccttgtagcactgacgtagcacttggcggggaacatattcgatggcgcata
ttgtgatggtggatgcgtataaaccgaccaaaatgcccgatcgtgctcaagttatcattcgcattgtg
ccgggaggtggcaccaagacccttcaacaaattatcaatcagttggagtatctatcccggaagggcag
gctggagctgcagcgttcagcccgacatctcgatattcccctgccaccggatcaaatccacgaacttg
cccgaagctgggttcaagagactggaacttatgacgaaagtcagccagacgaggaaaggcaacaggag
ttgaccacccatattattgttagcttccccgccggtacaagccaggtagcggcttatgcggcgagccg
ggagtgggcagccgagatgtttgggtcaggcgcaggggggggccgatacaactatcttacggccttcc
acatcgatcgcgaccacccacatctgcatgtcgtcgtcaatcggcgcgaacttttaggacacggctgg
ctgaagatatctcggcgccatccccaactgaattacgacgccctgcgcataaagatggccgagatttc
acttcgtcatggcattgccctcgatgcgagccgacgagcagaacgtggcatcaccgagcggccgatca
cttatgcccaatatcggcgccttgagcgggagcaggctcgccaaatccgtttcgaagacgcggatttg
gaacagtcgtcgccgcaaggagatcatccagagttcagccaacctttcgatacatccccatttgaagc
atccgcaggcggaccggaggacatgcctcggcccaacaatcggcagaatgagtcgcaagttcatctcc
aggagccagctggtgtcagcaacgaagccggtgtccttgtgcgggttgcattggagacggagcgcctt
gctcaaccattcgtttccgaaaccattctcgcggacgacatagggagcggctcttcgcgtgttgccga
gggccgtgtggagagcgcaaaccgcactcccgatattcctcgcgcagcaactgaagctgccacgcaca
cgacacacgaccggcagcggcgtgcaaagcgtcctcatgatgacgacggagggccgagtggagcaaaa
cgtgtgacattggaaggcatcgcggttggcccccaggcgaacgccggcgaacaggctggcagtagtgg
ccccttagtacggcaagctggaacgtctcggccatctccaccgacggccacgacgcgggccagcaccg
caaccgcttcattgtctgctacagcccacctccagcaacggagaggtgtcctttcaaagcgtccgcgt
gaagatgatgatggagaaccgagtgaacgcaaacgcgagagagatgagcgcagcaaggacgggcgtgg
 
gggaaataggagatag gagcttcgacaggcatcaaataaaacgaaaggctcagtcgaaagactgggcc
tttcgttttatctgttgtttgtcggtgaacgctctcctgagtaggacaaatccgccc
**SEQ ID 42**
   **virD2-SpyTag cassette** (SpyTag is underlined, virD2 is in bold)

ctgtcgattttgtgaagcggaagtgtgtctgtacttttatttgtgtatgattttgcgataattcat
aagtaatgtagtaattacctgattttatatttcaattttattgtaatataatttcaattgtaataata
taaaaataaatatcccttatgtgttcttgatttcgttttgtatatggctagattcccatctgccacga
cgaggaaatgctacggcgggcaagttcagatctttccgtcttctatggaggaagctatgtcgcaagg
cagtaggcccacctcaagtgacattgccgtcaaccagcgcgaatgcgtgaaggttgaaggcttcaagg
tcgtcagtacccgattaagatcggccgaatatgagagtttttctcatcaggcacgcttgctgggcctc
tccgacagcatggccatacgggttgcggtgcgccgcattggtggctttcttgaaatcgacgcagagac
tcgtcataggatggaggccatactacaatccataggaacactctcaagcaacattgccgcgctgctat
ctgcctatgccgaaaatccgacaatggatttggaggctttgcgagctgaacgtatcgccttcggtaaa
tctttcgctgacctcgacggcttgctccgttccattttgtccgtatcacggcggcggatcgacggttg
ctcgctgctgaaagacgccttgtagcactgacgtagcacttggcggggaacatattcgatgcccgatc
gtgctcaagttatcattcgcattgtgccgggaggtggcaccaagacccttcaacaaattatcaatcag
ttggagtatctatcccggaagggcaggctggagctgcagcgttcagcccgacatctcgatattcccct
gccaccggatcaaatccacgaacttgcccgaagctgggttcaagagactggaacttatgacgaaagtc
agccagacgaggaaaggcaacaggagttgaccacccatattattgttagcttccccgccggtacaagc
caggtagcggcttatgcggcgagccgggagtgggcagccgagatgtttgggtcaggcgcaggggggg
ccgatacaactatcttacggccttccacatcgatcgcgaccacccacatctgcatgtcgtcgtcaatc
ggcgcgaactttaggacacggctggctgaagatatctcggcgccatccccaactgaattacgacgcc
ctgcgcataaagatggccgagatttcacttcgtcatggcattgccctcgatgcgagccgacgagcaga
acgtggcatcaccgagcggccgatcacttatgcccaatatcggcgccttgagcgggagcaggctcgcc
aaatccgtttcgaagacgcggatttggaacagtcgtcgccgcaaggagatcatccagagttcagccaa
cctttcgatacatccccatttgaagcatccgcaggcggaccggaggacatgcctcggcccaacaatcg
gcagaatgagtcgcaagttcatctccaggagccagctggtgtcagcaacgaagccggtgtccttgtgc
gggttgcattggagacggagcgccttgctcaaccattcgtttccgaaaccattctcgcggacgacata
gggagcggctcttcgcgtgttgccgagggccgtgtggagagcgcaaaccgcactcccgatattcctcg
cgcagcaactgaagctgccacgcacacgacacacgaccggcagcggcgtgcaaagcgtcctcatgatg
acgacggagggccgagtggagcaaaacgtgtgacattggaaggcatcgcggttggcccccaggcgaac
gccggcgaacaggctggcagtagtggccccttagtacggcaagctggaacgtctcggccatctccacc
gacggccacgacgcgggccagcaccgcaaccgcttcattgtctgctacagcccacctccagcaacgga
gaggtgtcctttcaaagcgtccgcgtgaagatgatgatggagaaccgagtgaacgcaaacgcgagaga
gatgagcgcagcaaggacggcgtgggggaaataggagagcgcatattgtgatggtggatgcgtataa
accgaccaaataggagcttcgacaggcatcaaataaaacgaaaggctcagtcgaaagactgggccttt
cgttttatctgttgtttgtcggtgaacgctctcctgagtaggacaaatccgccc

### Amplification of the transgene nucleic acid in the organelles and mini-chromosome for gene expression in the organelles.

Although efficient systems for delivery of transgene nucleic acid (TNA) into organelles were established, a selectable marker and multiple rounds of selection are required to achieve an homoplasmic state of the transformants.

To address this issue we developed a DNA amplification system of TNA, allowing rapid achievement of an homoplasmic state of the transformants and/or by the introduction of autonomous mini-chromosomes without the need to insert TNA into the organelle genome.

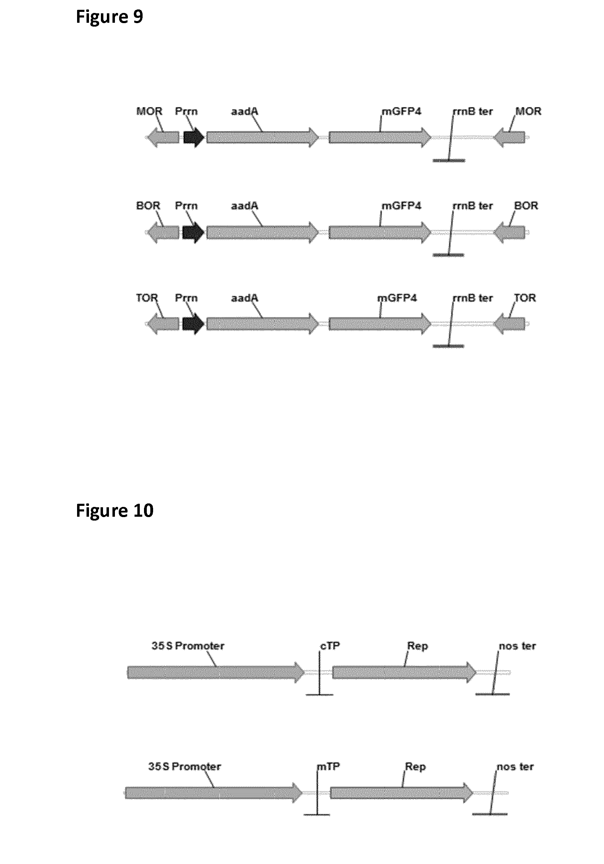

For this purpose we have employed the replication system of plant ssDNA geminiviruses. It has been shown that some geminiviruses can replicate in non-host organisms such as bacteria and yeast (Selth et al., 2002; Raghavan et al., 2004). Replication of the geminiviruses depends on host cell DNA polymerase, and requires a viral origin of replication and viral Replication Initiation Protein (RIP) encoded by the viral Rep gene. We have designed vectors for both fast achievement of homoplasmic state of the transformants and expression of the TNA in organelles from autonomous mini-chromosome (Figures 8 and 9).

In the first case two viral origins of replication (MOR, BOR or TOR) from Maize Streak Virus (MSV, subgroup I) (MOR), Beet Curly Top Virus (BCTV, subgroup II) (BOR) and Tomato Golden Mosaic Virus (TGMV, subgroup III) (TOR) were introduced on both sides of TNA (Figure 8). The expression of the viral Rep gene was performed from TNA or from a nuclear cassette where the Rep gene was fused to chloroplast or mitochondrial transit peptides (Figure 10). We have observed efficient amplification of TNA in the organelles, resulting in fast achievement of the homoplasmic state of the transformants.

In order to express TNA from the autonomous mini-chromosome, the TNA was modified by removing LFS and RFS, so that only the cassette with genes for expression in organelles was placed between two viral origins of replication (Figure 9). The expression of viral Rep gene was provided either from the TNA or from nuclear cassette where Rep gene was fused to the chloroplast or mitochondrial transit peptide.
**SEQ ID 43**
   **BCTV viral origin of replication (BOR)** 
gatcctgtactccgatgacgtggcttagcatattaacatatctattggagtattggagtattatatat
attagtacaactttcataagggccatccgttataatattaccggatggcccgaaaaaaatgggcaccc
aatcaaaacgtgacacgtggaaggggactgttgaatgatgtgacgtttttgagcgggaaacttcctga
ag
**SEQ ID 44**
   **MSV viral origin of replication (MOR)** 
Ccgacgacggaggttgaggctgagggatggcagactggcagctccaaactctatagtatacccgtgcg
ccttcgaaatccgccgctcccttgtcttatagtggttgcaaatgggccggaccgggccggcccagcag
gaaaagaaggcgcgcactaatattaccgcgccttcttttcctgcgagggcccggtagggcccgagcga
tttgatgtaaagtttggtcctgctttgtatgatttatctaaagcagcccat
**SEQ ID 45**
   **TGMV viral origin of replication (TOR)** 
Gtaattaagaggcttactaccaattgaggaggggctccaaaagttatatgaattggtagtaaggtagc
tcttatatattagaagttcctaagggcacgtggcggccatccgtttaatattaccggatggccgcgc
gatcgtcacccgacccgcttccgcaaattacgccgcattgtcgtctaagtggtcccgcatatgtgaag
ggccaatcatatttggccctgaaatctaagata
**SEQ ID 46**
   **BCTV Rep gene (B-rep)** 
Atgcctcctactaaaagatttcgtattcaagcaaaaacatatttcttacatatcctcagtgttctct
ttcaaaagaagaagctcttgagcaaattcaaagaatacaactttcatctaataaaaatatattaaaa
ttgccagagagctacacgaagatgggcaacctcatctccacgtcctgcttcaactcgaaggaaaagtt
cagatcacaaatatcagattattcgacctggtatccccaaccaggtcagcacatttccatccaaacat
tcagagagctaaatccagctccgacgtcaagtcctacgtagacaaggacggagacacaattgaatggg
gagaattccagatcgacggtagaagtgctagaggaggtcaacagacagctaacgactcatatgccaag
gcgttaaacgcaacttctcttgaccaagcacttcaaatattgaaggaagaacaaccaaaggattactt
ccttcaacatcacaatcttttgaacaatgctcaaaagatatttcagaggccacctgatccatggactc
cactatttcctctgtcctcattcacaaacgttcctgaggaaatgcaagaatgggctgatgcatatttc
ggggttgatgccgctgcgcggcctttaagatataatagtatcatagtagagggtgattcaagaacagg
gaagactatgtgggctagatctttagggcccacaattacatcacagggcacttagattttagcccta
gaacgtattatgatgaagtggaatacaacgtcattgatgacgtagatcccacttacttaaagatgaaa
cactggaaacaccttattggagcacaaaaggagtggcagacaaacttaaagtatggaaaaccacgtgt
cattaaaggtggtatcccctgcattatattatgcaatccaggacctgagagctcataccaacaatttc
ttgaaaaaccagaaaatgaagcccttaagtcctggacattacataattcaaccttctgcaaactccaa
ggtccgctctttaataaccaagcagcagcatcctcgcaaggtgactctaccctgtaa
**SEQ ID 47**
   **MSV Rep gene (M-rep)** 
atggcctcctcctcatccaaccgtcagttctcacaccggaacgctaacacgttcctaacctatccaaa
gtgtccagaaaatcctgaaatcgcctgtcagatgatctgggagctcgttgttcgttggattcccaaat
acattctatgtgcccgagaggcacataaagatggaagtttgcatttacatgcattgcttcagacagag
aagccggtaaggatatctgactcaaggttctttgatataaatgggtttcacccaaatattcagagtgc
caagtcagtaaacagggtgaggattacattctcaaggaacctctggctgtgtttgagagaggtactt
tcattcctaggaagtccccttcctaggaaaatctgattcagaggtaaaggaaaaaagccttctaaa
gatgaaataatgcgagacattatttcacacgctacttccaaagaagagtacctctccatgatccagaa
agagcttccctttgattggtccacaaaattgcagtattttgaatactctgcaaataagctttttcctg
agattcaggaagagttcaccaatcctcatccaccctcatcacctgatttactttgtaatgagtcaatc
aatgattggctccagcctaacatcttccagtcatcagatgaaagatcaagaaagcagagcctctacat
cgtcggcccaacaagaaccggaaaatctacttgggccagaagcctaggggttcataattactggcaaa
ataatgttgattggtcttcatacaacgaagacgcaatctataacatcgtagatgatattccgtttaaa
ttctgtccttgttggaaacagttagttggctgtcagaggatttcattgtaaaccccaagtatggtaa
aaagaaaaaggtgcagaagaagtctaagcctacaataatcctcgccaactcggatgaagattggatga
aggaaatgactccagggcagctggagtatttcgaggcaaactgcatcatttacattatgtcgccgggg
gagaaatggtattctccccctgagctgcctcctacggaggcagtacattcagatagatcttga
**SEQ ID 48**
   **TGMV Rep gene (T-rep)**

atgccatcgcatccaaaacggtttcaaataaatgccaaaaattattttcttacatatcctcagtgctc
cttgtccaaagaagaatcactttctcaattacaagccctaaacactccgattaacaaaaaattcataa
aaatctgcagagagcttcatgaagatgggcaacctcacctccacgtgcttattcagttcgaggaaaa
tactgctgccaaaatcaacgattcttcgacctggtatccccaacaaggtcagcacatttccatccaaa
cattcagagagctaaatcgtcttccgacgtcaagacgtacatcgacaaagacggagatactcttgtat
ggggagaattccaggtcgacggtcgaagtgctagaggaggttgccaaacatctaacgacgctgcagca
gaggcgttaaatgcttcttccaaagaagaagccctgcagataattagagagaaaatcccagaaaaata
tttatttcagttccacaatctaaatagcaatttagataggatatttgataagactcctgaaccatggc
ttcctccgttccacgtctcatcatttactaacgtgccagacgagatgagacaatgggctgaaaattat
tttggaaagagttccgctgcgcggccggagagacctattagtattatcatcgagggcgatagtcggac
gggaaagactatgtgggctcgttcactaggcccacataattatttgagcgggcatttggatctcaatt
ctagggtttactcaaacaaggttgagtataacgtcatcgatgatgtcacaccgcaatatctaaagttg
aaacattggaaagaactcattggggcccaaagagattggcagactaactgtaaatacggaaagccagt
tcaaattaaaggaggtatcccgtcaatcgtgctgtgcaatcctggagagggtgctagctataaagttt
tcctcgacaaagaggaaaacactccactaaagaactggactttccataatgcgaaattcgtcttcctc
aactccccccctctatcaaagctcaacacagagcagctaa

### References

Selth LA, Randles JW, Rezaian MA. Agrobacterium tumefaciens supports DNA replication of diverse geminivirus types. FEBS Lett. 2002, 10;516(1-3):179-82.
Vineetha Raghavan, Punjab S. Malik, Nirupam Roy Choudhury, and Sunil K. Mukherjee. The DNA-A Component of a Plant Geminivirus (Indian Mung Bean Yellow Mosaic Virus) Replicates in Budding Yeast Cells. J Virol. 2004, 78(5): 2405-2413.
Gianluca Veggiani, Bijan Zakeri, and Mark Howarth. Superglue from bacteria: unbreakable bridges for protein nanotechnology. Trends in Biotechnology. 2014, 32(10):506-12.
Long Li, Jacob O. Fierer, Tom A. Rapoport, and Mark Howarth. Structural Analysis and Optimization of the Covalent Association between SpyCatcher and a Peptide Tag. J Mol Biol. 2014, 23; 426(2): 309-317.
Wikoff, W.R. et al. Topologically linked protein rings in the bacteriophage HK97 capsid. Science. 2000, 289, 2129-2133
K. I. Ivanov, K. Eskelin, A. Lõhmus, K. Mäkinen. Molecular and cellular mechanisms underlying potyvirus infection. J. Gen. Virol. 2014, 95: 1415-1429.
Rantalainen KI, Uversky VN, Permi P, Kalkkinen N, Dunker AK, Mäkinen K. Potato virus A genome-linked protein VPg is an intrinsically disordered molten globule-like protein with a hydrophobic core. Virology. 2008, 1;377(2):280-8.
Grzela R, Szolajska E, Ebel C, Madern D, Favier A, Wojtal I, Zagorski W, Chroboczek J. Virulence factor of potato virus Y, genome-attached terminal protein VPg, is a highly disordered protein. J Biol Chem. 2008, 283(1):213-21.
Allan Olspert, Lauri Peil, Eugenie Hebrard, Denis Fargette and Erkki Truve. Protein-RNA linkage and post-translational modifications of two sobemovirus VPgs. Journal of General Virology. 2011, 92, 445-452.
Lampson BC, Inouye M, Inouye S. Retrons, msDNA, and the bacterial genome" . Cytogenet Genome Res. 2005, 110 (1-4): 491-9
Rozwadowski K and Lydiate D. 2003. https://patentscope.wipo.int/search/en/detail.jsf?docId=WO2003104470 &recNum=1&maxRec=&office=&prevFilter=&sortOption=&queryString=&tab=P CT+Biblio
Sahoo et al. An improved protocol for efficient transformation and regeneration of diverse indica rice cultivars. Plant MAtheods. 2011, 7:49
Tadashi Shimamoto, Hideki Kawanishi, Tomofusa Tsuchiya, Sumiko Inouye, and Masayori Inouye. In Vitro Synthesis of Multicopy Single-Stranded DNA, Using Separate Primer and Template RNAs, by Escherichia coli Reverse Transcriptase. J Bacteriol. 1998, 180(11): 2999-3002.

### Experimental Examples.

### Chloroplast transformation using groupII intron constructs.

Reference is made to constructs detailed in Table 1 throughout.

### Note to LS: TABLE 1 GOES IN HERE

We have utilised Agrobacterium-mediated transformation of tobacco (http://plantsci.missouri.edu/muptcf/protocols/tobacco.html) and rice Sahoo et al., 2011). In order to transform chloroplasts in tobacco, we have used the constructs OTV1-OTV4 (Table 1). The constructs contain TNA in domain IV of the corresponding groupII intron, while the reshuffled retron is flanking 3'-end of the groupII intron. The reverse transcriptase of the retron is fused with corresponding intron encoded protein (IEP), and fulfils three functions, namely translocate TNA-RNA to organelle, initiates reverse transcription from retron to generate priming for reverse transcription of the TNA by the IEP. We expect that reverse transcription is more efficient in this case as it is a natural configuration for reverse transcription by the IEP. The 3' and 5'-ends of the intron are also reverse transcribed in this case, but they are eliminated by homologous recombination machinery during TNA integration into the organelles genome.

The tobacco constructs OTV5 and OTV6 contain TNA at the 3'-end of the intron, and utilise direct priming of the TNA without reverse transcription of intron sequence. The reverse transcription in this case generated by combination of RT activities from both retron and the IEP.

Similar approach was utilised for rice transformation with constructs OTV7-OTV10 (Table 1).

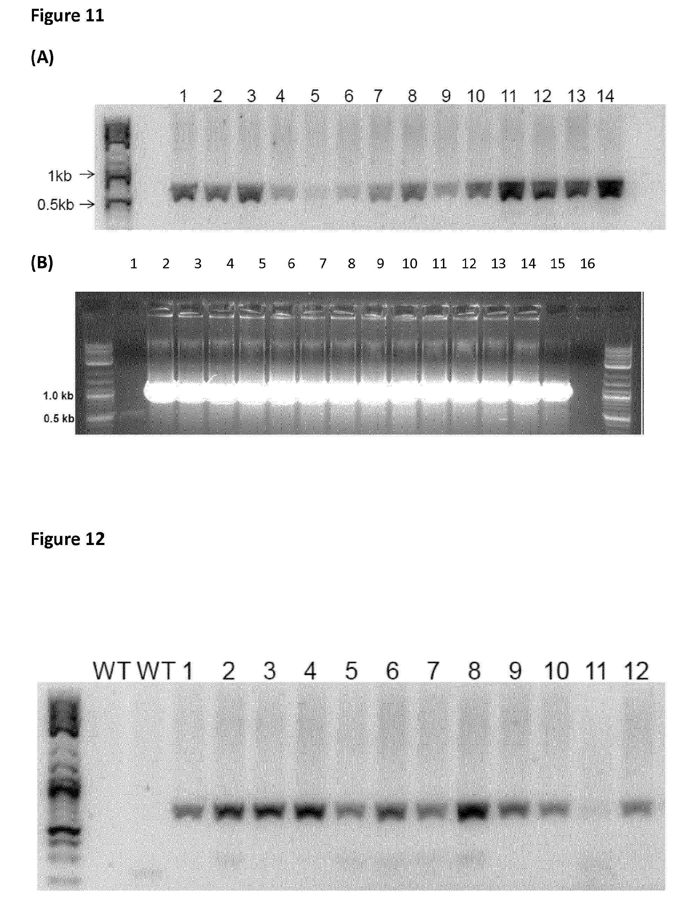

Successful transformation of tobacco and rice chloroplasts using groupII constructs was confirmed on spectinomycin resistant plants by PCR of flanking sequences and by sequencing of the corresponding PCR products (Figure 11A and B).

The following primers have been used for tobacco to generate a fragment of 720 bp for tobacco:
**SEQ ID 49**
   **TC1** ctgagtaggacaaatccgccc
**SEQ ID 50**
   **TC2** ggtggagatcatattcactctggtaccgtagt
   and a fragment of 1100bp for rice:
**SEQ ID 51**
   **RC1** accccgggacgagaagtagtagga
**SEQ ID 52**
   **RC2** atcgatcatgagattcatagttgcattact

### Chloroplast transformation using PVY-based vectors.

To transform chloroplast in tobacco using Potato Virus Y as a chloroplast translocation sequence, the OTV21, OTV22 and OTV23 constructs has been used. Co-transformation of the construct OTV27 containing SpyCatcher fused to chloroplast transit peptide was performed in combination with OTV22 (N-terminal SpyTag) or OTV23 (C-terminal SpyTag).

PCR analysis of flanking sequences using T1 and T2 primers on spectinomycin resistant transformants, and sequencing analysis of amplified fragments have confirmed insertion of transgene using this approach (Figure 12).

### Chloroplast transformation using modified Agrobacterium virD2 protein.

Agrobacterium-mediated transformation of the tobacco chloroplasts using modified strain GV3101 with knocked out virD2 and virE2 genes was performed. Complementary virD2 protein modified by fusion of chloroplast transit peptide (OTV29), or N-terminal SpyTag (OTV31) and C-terminal SpyTag (OTV32) was expressed from *Agrobacterium* virD operon promoter. The cassette carrying virD promoter, modified virD2 gene and bacterial rrnB terminator was integrated on binary vector outside of the T-DNA boarders. The OTV31 and OTV32 constructs carrying SpyTag were transformed in two steps, as SpyCatcher peptide (construct OTV27) should be already expresses in the cytoplast of plant cell before challenging plant cell with these constructs. The tobacco leaves were first infiltrated with *Agrobacterium* containing OTV27 construct, following second round of transformation of leaf explant from infiltrated plants with OTV31 or OTV32 two days later.

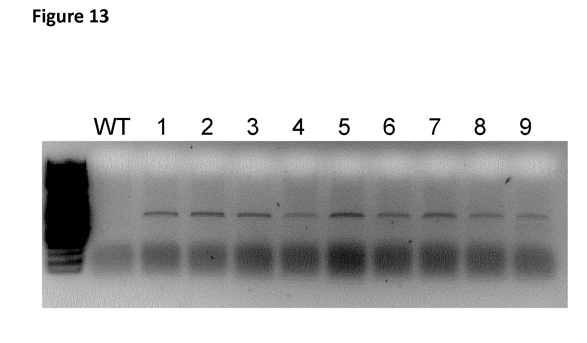

PCR analysis of flanking sequences using the T1 and T2 primers on the spectinomycin resistant transformants, and sequencing analysis of amplified fragments have confirmed insertion of transgene using this approach (Figure 13).

### TNA amplification in the chloroplast using Geminivirus replication system.

DNA approach for chloroplast transformation using modified virD2 gene has proved to be feasible but not efficient from point of view of copy number of transgene delivered to the chloroplasts. To address this issue, we have developed transgene amplification system in chloroplasts using Geminivirus replication system. It has been shown that Geminivirus could be replicated in *Agrobacterium* and yeast. Introduction of viral origin of replication and expression of viral Rep gene encoding replication initiation protein (RIR), was sufficient to replicate plasmid in these organisms.

To evaluate whether Geminivirus can be replicated in the chloroplasts, we have selected Maize Streak Virus-MSV (subclass I), Beet Top Curly Virus-BCTV (subclass II) and Tomato Golden Mosaic Virus-TGMV (subclass III). The constructs were prepared containing two viral origins of replication with chloroplast transformation cassette located between them. Resulted constructs OTV33, OTV34 and OTV35 containing correspondingly BCTV viral origins of replication (BOR), MSV viral origins (MOR), and TGMV viral origins (TOR), were delivered to the tobacco chloroplasts using modified virD2 *Agrobacterium* approach. The Rep gene for corresponding viral origin of replication was fused to chloroplast transit peptide and was co-expressed from nuclear promoter (OTV39, OTV40 and OTV41).

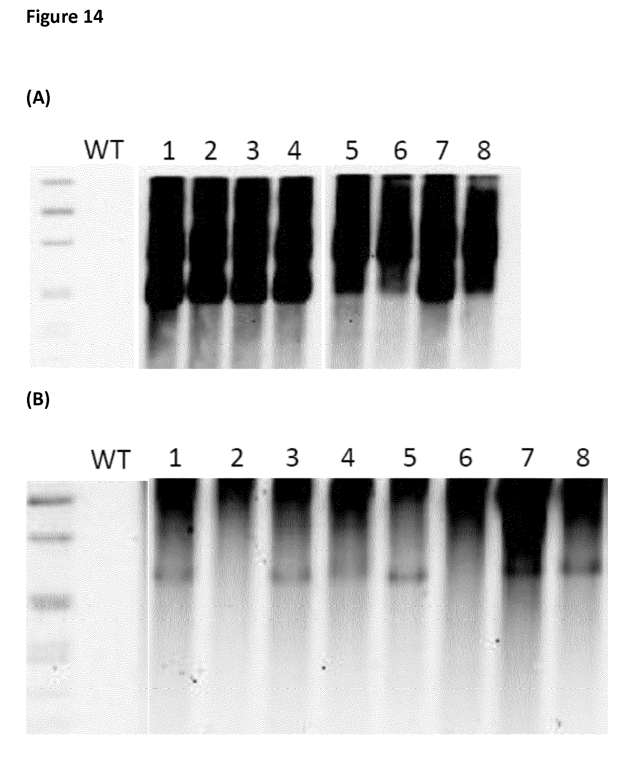

We have observed dramatic amplification of transgene nucleic acid with BCTV and TGMV origins (Figure 14A), while MSV origins were able to amplify transgene with modest efficiency (Figure 14B).

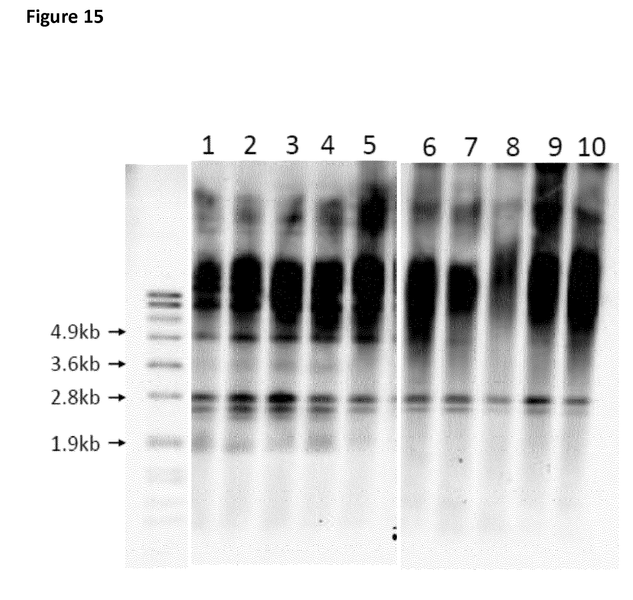

Next we wanted to see whether we could maintain transgene in the chloroplasts as mini-chromosome without integration in the chloroplast genome. For this purpose the constructs OTV45 and OTV46 which do not contain LFS and RFS were prepared and co-delivered with the construct OTV39 and OTV41 into the tobacco chloroplasts using combination of *Agrobactrium* with functional virD2 gene for constructs OTV39 and OTV41, and *Agrobacterium* with modified virD2 gene fused to chloroplast transit peptide. We have observed efficient delivery amplification of transgene cassette without insertion into the chloroplast genome (Figure 15).

### Mitochondria transformation using groupII intron constructs and PVY-based vectors.

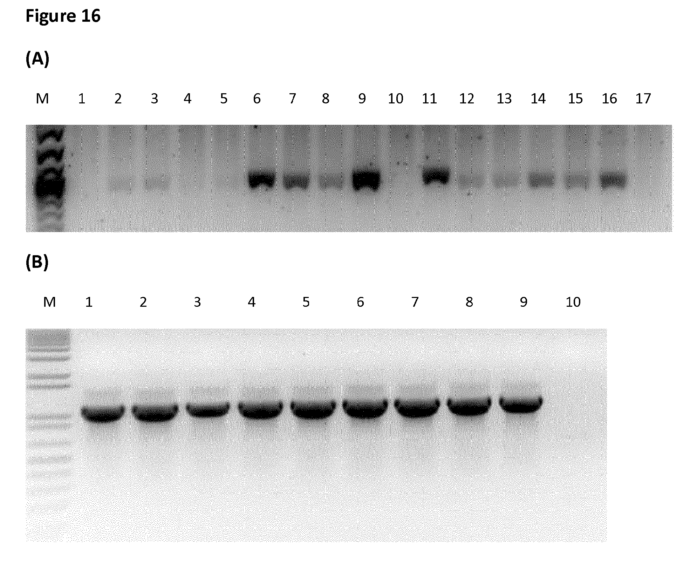

Transformation of mitochondria in tobacco and rice was performed in similar way as transformation of chloroplast using constructs OTV11-OTV16 for tobacco and OTV17-OTV20 for rice. Selection was performed for insertion of T-DNA into the nuclear genome, as there is no selectable marker for mitochondria transformation. The OTV24-OTV26 were utilised for PVY-based approach in combination with OTV28 vector. The plants recovered on kanamycin for nuclear insertion were than analysed for insertion of the transgene into the mitochondrial genome using PCR of flanking sequences and by sequencing of the PCR generated fragments. The following primers have been used for amplification of flanking sequences in tobacco to generate fragment of 1050 bp:
**SEQ ID 53**
   TM1 cgtcccataccttctgcctgtctca
**SEQ ID 54**
   TM2 gatggatacatacgatttcacttat
   and a fragment of 1170 bp for rice:
**SEQ ID 55**
   RM1 gggtaacttttatttatcattcaca
**SEQ ID 56**
   RM2 acttcggcgatcaccgcttctgccat
   We observed successful integration events with all approaches (Figure 16).

### Mitochondria transformation using modified Agrobacterium virD2 protein.

Agrobacterium-mediated transformation of the tobacco mitochondria using modified strain GV3101 with knocked out virD2 and virE2 genes was performed. Complementary virD2 protein modified by fusion of mitochondria transit peptide (OTV30), or N-terminal SpyTag (OTV31) and C-terminal SpyTag (OTV32) was expressed from *Agrobacterium* virD operon promoter. The cassette carrying virD promoter, modified virD2 gene and bacterial rrnB terminator was integrated on binary vector outside of the T-DNA boarders. The OTV31 and OTV32 constructs carrying SpyTag were transformed in two steps, as SpyCatcher peptide (construct OTV28) should be already expresses in the cytoplast of plant cell before challenging plant cell with these constructs. The tobacco leaves were first infiltrated with *Agrobacterium* containing OTV28 construct, following second round of transformation of leaf explant from infiltrated plants with OTV31 or OTV32 two days later. PCR analysis of flanking sequences has confirmed integration of transgene into the mitochondrial genome of tobacco (Figure 17).

### TNA amplification in the mitochondria using Geminivirus replication system.

Similar to chloroplast approach, to amplify transgene in the mitochondria using Geminivirus replication system we have prepared OTV47 (BOR) and OTV48 (TOR) constructs. These constructs were co-expressed with OTV42 and OTV44 to generate autonomous mini-chromosome of transgene in the mitochondria without its insertion into the mitochondrial genome. Southern analysis of transgenic plants has confirmed that at least BCTV and TGMV-based system could replicate in the mitochondria (Figure 18).

### Variant 1 of the invention

### STATEMENTS ON VARIANT 1

1. A method of transforming at least one species of plant cell organelle comprising:
   i) transforming the nucleus of a plant cell with a DNA cassette carrying at least one transgene nucleic acid (TNA) sequence of interest;
   ii) recruiting the transgene nucleic acid RNA generated by the transcription of the transgene nucleic acid sequence of step i) from the cytoplasm and directing it into the at least one species of plant organelle;
   iii) reverse transcribing the transgenic nucleic acid RNA of ii) into single stranded DNA (ssDNA) in the at least one organelle; and
   iv) inserting the single stranded DNA of iii) into the organelle genome via homologous recombination; and
   wherein the reverse transcribing event of step iii) within the organelle is performed by a **retron** specific reverse transcriptase sequence fused to at least one reverse transcriptase sequence different to the first.
2. A method of transforming a plant cell according to statement 1 comprising:
   1) introducing into the said plant cell a first nucleic acid sequence that comprises a nuclear promoter operably linked to a first nucleic acid sequence comprising a plant organellar transit peptide (TP) located adjacent to a **retron** specific reverse transcriptase sequence fused to at least one reverse transcriptase sequence different to the first, such as an IEP sequence, and a nuclear terminator;
   2) introducing into the said plant cell a second nucleic acid sequence that encodes for a group II intron operably linked to a plant nuclear promoter; and
   3) introducing into the said plant cell a third nucleic acid sequence that encodes for an organellar transgene cassette comprising a left flanking sequence, an organellar promoter, at least one transgene nucleic acid sequence of interest, an organellar terminator and a right flanking sequence ;
   4) introducing a fourth nucleic acid sequence that codes for a retron sequence for reverse transcription of the TNA.
3. A method according to statement 1 or statement 2, wherein the transgene nucleic acid sequence is a recombinant DNA sequence or an introduced native, isolated genomic DNA sequence.
4. A method according to any one of statements 1 to 3, wherein the third nucleic acid sequence of claim 2 step 3) is inserted into Domain IV of the group II intron of step 2).
5. A method according to any one of statements 1 to 3, wherein the third nucleic acid sequence of statement 1 step 3) is located at the 5' and/or 3' end of the group II intron of step 2).
6. A method according to any one of statements 1 to 3 and 5, wherein the third nucleic acid sequence of 3) is located at the 3' end of the group II intron of step 2).
7. A method according to any one of the preceding statements wherein the plant organelle is selected from a plant mitochondrion, and a plant plastid.
8. A method according to any one of the preceding statements, wherein the plant organelle is a mitochondrion.
9. A method according to any one of statements 1 to 7, wherein the plant organelle is selected from chloroplasts, proplastids, etioplasts, chromoplasts, amyloplasts, leucoplasts and elaioplasts, and is preferably a chloroplast.
10. A method according to any one of the preceding statements, wherein the transgene nucleic acid sequence is selected from a recombinant mammalian nucleic acid sequence, an isolated genomic mammalian nucleic acid sequence, a recombinant plant nucleic acid sequence and an isolated genomic plant nucleic acid sequence and two or more thereof.
11. A method according to any one of the preceding statements, wherein the DNA cassette comprises an organellar promoter selected from a mitochondrion specific promoter and a plastid specific promoter.
12. A method according to any one of the preceding statements, wherein the mitochondrion specific promoter is selected from mitochondrial promoter nucleotide sequences, such as ATP6, ATP9, Cob, rrn18, Rps13, Rps19, Cox3, Nad6, Nad9 5' untranslated sequences (promoter region) of tobacco mitochondria, and Arabidopsis mitochondria; and the plastid specific promoter sequence is selected from the group consisting of the RNA polymerase promoter, rpo B promoter element, atpB promoter element, the clpP promoter element, the 16S rDNA promoter element, PrbcL, Prps16, the Prrn16, Prrn-62, Pycf2-1577, PatpB-289, Prps2-152, Prps16-107, Pycf1-41, PatpI-207, PclpP-511, PclpP-173, PaccD-129, PaccD-129 promoter of the tobacco accD gene, the PclpP-53 promoter of the clpP gene, the Prrn-62 promoter of the rrn gene, the Prps16-107 promoter of the rps16 gene, the PatpB/E-290 promoter of the tobacco atpB/E gene, and the PrpoB-345 promoter of the rpoB gene.
13. A method according to claim any one of statements 1 to 12, wherein the transgene or isolated nucleic acid sequence is selected from insulin, preproinsulin, proinsulin, glucagon, interferons such as α-interferon, β-interferon, γ-interferon, blood-clotting factors selected from Factor VII, VIII, IX, X, XI, and XII, fertility hormones including luteinising hormone, follicle stimulating hormone growth factors including epidermal growth factor, platelet-derived growth factor, granulocyte colony stimulating factor and the like, prolactin, oxytocin, thyroid stimulating hormone, adrenocorticotropic hormone, calcitonin, parathyroid hormone, somatostatin, erythropoietin (EPO), enzymes such as β-glucocerebrosidase, haemoglobin, serum albumin, collagen, biotic and abiotic stress proteins, such as insecticidal and insect toxic proteins, for example from, or derived from Bacillus thuringiensis, nematicidal proteins, herbicide resistance proteins, (e.g. to glyphosate), salt-tolerance proteins, drought tolerant proteins, proteins capable of conferring cytoplasmic male sterility to plant breeding lines; nutritional enhancement proteins involved in the biosynthesis of phenolics, starches, sugars, alkaloids, vitamins, and edible vaccines, monoclonal antibodies and active fragments thereof, industrial enzymes and active fragments thereof.
14. A method according to any one of statements 1 to 13, wherein the transgene or isolated nucleic acid sequence is selected from proteins that confer cytoplasmic male sterility to a plant.
15. A method according to any one of the preceding statements, wherein the transgene or isolated nucleic acid sequence that is capable of conferring cytoplasmic male sterility to the plant is selected from the petunia mitochondrion *pcf* sequence, *orf107* sequence of sorghum and orf 79 of rice.
16. A method according to any one of the preceding statements wherein the retron is a DNA sequence comprising a *msr* element encoding an RNA sequence comprising a binding domain for retron-specific reverse transcriptase, and a *msd* element encoding a DNA component fused to the 3' end of a nucleic acid sequence or a fragment thereof and/or the 3' end of TNA, wherein the *msr* and *msd* elements comprise pairs of inverted repeat sequences forming doublestranded RNA regions driving reverse transcription of the *msd* element and/or reverse transcription of the *TNA:msd* element fusion product.
17. A method according to claim any one of the preceding statements, wherein the msr and msd elements comprise pairs of inverted repeat sequences selected from a1 and a2, and b1 and b2 sequences.
18. A method according to any one of the preceding statements, wherein the retron msDNA is a bacterial retron msDNA sequence, such as a sequence selected from Ec86, Mx162, Sal63, Ec67, Ec73, and Ec107.
19. A method according to any one of the preceding statements, wherein the at least one reverse transcriptase sequence different to the first is a groupII intron or an IEP fragment thereof that encodes reverse transcriptase functionality is selected from the LtrB intron, the RmIntORF, the a12 intron, the tobacco group II intron and the nad1 gene containing matK.
20. A method according to any one of the preceding statements wherein the plant organellar transit peptide is independently selected from the mitochondrial signal peptide from tobacco F1-ATPase-1 β subunit, and the Arabidopsis CPN60 protein; and the plastidial transit peptide independently from selected from the tobacco rbcS-cTP, and the Arabidopsis HSP70-cTP protein.
21. A plant cell obtained according to any one of statments 1 to 20.
22. A plant cell comprising transformed plant organelles as defined in any one of statements 1 to 20, wherein the transformed plant organelles comprise:
   i) an exogenous or heterologous left flanking sequence (LFS) and an exogenous or heterologous right flanking sequence (RFS);
   ii) at least one exogenous or heterologous organelle-specific promoter and at least one exogenous or heterologous organelle-specific terminator sequence; and
   iii) at least one exogenous or heterologous isolated transgene nucleic acid sequence of interest.
23. A plant cell according to statement 21, wherein the transformed organelles are selected from plant plastids and mitochondria transformed as defined in any one of statements 1 to 20.
24. A transformed plant organelle comprising:
   i) an exogenous or heterologous left flanking sequence (LFS) and an exogenous or heterologous right flanking sequence (RFS);
   ii) at least one exogenous or heterologous organelle-specific promoter and at least one exogenous or heterologous organelle-specific terminator sequence; and
   iii) at least one exogenous or heterologous isolated transgene nucleic acid sequence of interest.
25. A transformed plant organelle according to statement 24, wherein the transformed organelle is selected from a plant plastid and a mitochondrion transformed as defined in any one of statements 1 to 20.
26. A population of transformed plant organelles as defined in statement 23 or statement 25 comprised in a plant cell.
27. A population of transformed plant organelles according to statement 25, wherein the organelles are located in plant cells selected from tobacco *(Nicotiana tabacum)* and other *Nicotiana* species, arabidopsis, potato, corn(maize), canola (rape), rice, wheat, barley, brassica sp. such as cauliflower, broccoli (e.g. green and purple sprouting), cabbage (e.g. red, green and white cabbages), curly kale, Brussels sprouts, cotton, algae (e.g. blue green species), lemnospora, or moss (e.g. *physcomitrella patens),* tomato, capsicum, squashes, sunflower, soyabean, carrot, melons, grape vines, lettuce, strawberry, sugar beet, peas, and sorghum.
28. A population of transformed plant organelles according to statement 26 or statement 27 wherein the organelles are located in plant cells selected from cotton, rice, oilseed Brassica species such as canola, corn(maize) and soyabean.
29. A method of producing at least a heterologous or exogenous RNA species in a plant that comprises:
   1) introducing into a regenerable plant cell a first nucleic acid sequence that comprises a nuclear promoter operably linked to a first nucleic acid sequence comprising a plant organellar transit peptide (TP) located adjacent to a retron specific reverse transcriptase sequence fused to at least one reverse transcriptase sequence different to the first, such as a group II intron sequence or a fragment thereof possessing reverse transcriptase functionality, such as an IEP sequence, and a nuclear terminator;
   2) introducing into the said plant cell a second nucleic acid sequence that encodes for a group II intron operably linked to a plant nuclear promoter; and
   3) introducing into the said plant cell a third nucleic acid sequence that encodes for an organellar transgene cassette comprising a left flanking sequence, an organellar promoter, at least one transgene nucleic acid sequence of interest, an organellar terminator and a right flanking sequence ; and
   4) introducing a fourth nucleic acid sequence that codes for a retron sequence for reverse transcription of the TNA.
   5) growing said regenerable plant cell of steps 1) to 4);
   6) selecting a plant cell of (5), wherein the transgene comprised within the plant organellar transgene cassette is integrated into the organellar genome;
   7) regenerating a plant from the plant cell of (6); and
   8) growing the plant of (7).
30. A method according to statement 29, wherein the heterologous or exogenous RNA species encoded by the transgene that is integrated into the organellar genome is expressed as a heterologous or exogenous protein.
31. A method according to statement 29 or statement 30, wherein the plant organellar genome is independently selected from that of plant mitochondria and plant plastids.
32. An isolated polynucleotide sequence that comprises a plant nuclear promoter operably linked to a first nucleic acid sequence comprising a plant organellar transit peptide (TP) located adjacent to a retron specific reverse transcriptase first nucleic acid sequence that comprises a nuclear promoter operably linked to a first nucleic acid sequence comprising a plant organellar transit peptide (TP) located adjacent to a retron specific reverse transcriptase sequence fused to at least one reverse transcriptase sequence different to the first, such as a group II intron sequence or a fragment thereof possessing reverse transcriptase functionality, such as an IEP sequence and a nuclear terminator ; a second nucleic acid sequence that encodes for a group II intron operably linked to a plant nuclear promoter ; a third nucleic acid sequence that encodes for an organellar transgene cassette comprising a left flanking sequence, an organellar promoter, at least one transgene nucleic acid sequence of interest, an organellar terminator and a right flanking sequence ; and a fourth nucleic acid sequence that codes for a retron sequence for reverse transcription of the TNA for use in a method according to any one of statements 1 to 19 and statements 28 to 30.
33. An isolated polynucleotide sequence as defined in any one of statements 1 to 20 and statements 29 to 31, comprising genomic DNA.
34. An isolated polynucleotide sequence as defined in any one of statements 1 to 20 and statements 29 to 31, comprising a cDNA component.
35. A nucleic acid vector suitable for transformation of a plant cell or a bacterial cell, wherein the cell includes a polynucleotide sequence according to any one of statements 32 to 34.
36. A nucleic acid vector according to statement 35 for transformation of a bacterial cell.
37. A nucleic acid vector according to statement 36 for transforming an *Agrobacterium* cell.
38. A host cell containing a heterologous polynucleotide or nucleic acid vector according to any one of statements 32 to 37.
39. A host cell according to statement 38 which is a plant cell or a bacterial cell.
40. A host cell according to statement 38 or statement 39 comprised in a plant, a plant part or a plant propagule, or an extract or derivative of a plant or in a plant cell culture.
41. A method of producing a cell according to any one of statements 38 to 40, the method including incorporating said polynucleotide or nucleic acid vector into the cell by means of transformation.
42. A method according to statement 41 which includes regenerating a plant from a cell according to any one of statements 38 to 40 from one or more transformed cells.
43. A plant comprising a plant cell according to any one of statements 38 to 40.
44. A plant comprising a plant cell according to statement 43 that is selected from the group consisting of tobacco *(Nicotiana tabacum)* and other *Nicotiana* species, such as *Nicotiana benthamiana,* carrot, vegetable and oilseed *Brassica's,* melons, Capsicums, grape vines, lettuce, strawberry, sugar beet, wheat, barley, (corn)maize, rice, soybean, peas, sorghum, sunflower, tomato, cotton, and potato.
45. A plant comprising a plant cell according to statement 43 or statement 44 that is selected from the group consisting of cotton, rice, oilseed Brassica species such as canola, corn(maize) and soybean.
46. A method of producing a plant, the method including incorporating a polynucleotide sequence or nucleic acid vector according to any one of statements 31 to 36 into a plant cell and regenerating a plant from said cell.
47. Use of a polynucleotide sequence according to any one of statements 32 to 37 in the production of a transgenic plant.
48. Use of a polynucleotide sequence according to any one of statements 32 to 37 in the production of a polypeptide or protein in a plant.

All definitions for component parts of statements 1 to 48 of Variant 1 are found either in the accompanying description or in statements 1 to 48. The Experimental section provides technical descriptions of work performed relating to Variant 1.

### Variant 2 of the invention

### STATEMENTS ON VARIANT 2

1. A method for use in transforming a transgene nucleic acid of interest into a plant organelle in a plant cell comprising:
   1(a) deleting viral polymerase and coat protein sequences from the complete viral genome of a potyvirus and replacing them with transgenic nucleic acid **in cis,** wherein the said transgenic nucleic acid comprises a nuclear promoter operably linked to a viral 5' UTR sequence linked to the 5' end of a complete RNA translocation sequence of the potyvirus, wherein
      i) the 5' end of the potyviral RNA translocation sequence is covalently linked to the VPg protein therein and to an organellar transit peptide ; or
      ii) the potyviral RNA translocation sequence is modified by fusing a spytag short peptide sequence to the viral VPg protein at either the N- or C-terminus thereof; and introducing the product of i) or ii) into a plant cell ;
   1(b) introducing into the viral translocation sequence a second component nucleic acid sequence that encodes for an organellar transgene cassette comprising a left flanking sequence, an organellar promoter, at least one transgene nucleic acid sequence of interest, an organellar terminator and a right flanking sequence ; and
   1(c) introducing into the said plant cell a third component nucleic acid sequence that codes for a retron sequence for reverse transcription of the TNA; and
   1(d)(i) introducing into the said plant cell a fourth component nucleic acid sequence comprising a viral 3'UTR sequence ;
      1(d)(ii) introducing into the plant cell a nucleic acid sequence comprising a nuclear promoter operably linked to a nucleic acid sequence comprising a plant organellar transit peptide (TP) located adjacent to a retron-based reverse transcriptase fused to an intron encoding protein (IEP), and a nuclear terminator; and
   1(e)(i) introducing into the plant cell either a potyviral polymerase ***in trans*** under the control of a plant nuclear promoter sequence and a terminator ; or
      1(e)(ii) a spycatcher peptide fused to an organellar transit peptide, the said fused peptide being expressed under the control of a nuclear promoter.
2. A method according to statement 1, wherein the transgene nucleic acid sequence is a recombinant DNA sequence or an introduced native, isolated genomic DNA sequence.
3. A method according to statement 1 or statement 2, wherein the plant organelle is selected from a plant mitochondrion, and a plant plastid.
4. A method according to any one of the preceding statements, wherein the plant organelle is a mitochondrion.
5. A method according to any one of statementss 1 to 14, wherein the plant organelle is selected from chloroplasts, proplastids, etioplasts, chromoplasts, amyloplasts, leucoplasts and elaioplasts, and is preferably a chloroplast.
6. A method according to any one of the preceding statementss, wherein the transgene nucleic acid sequence is selected from a recombinant mammalian nucleic acid sequence, an isolated genomic mammalian nucleic acid sequence, a recombinant plant nucleic acid sequence and an isolated genomic plant nucleic acid sequence and two or more thereof.
7. A method according to any one of the preceding statements, wherein the DNA cassette comprises an organellar promoter selected from a mitochondrion specific promoter and a plastid specific promoter.
8. A method according to any one of the preceding statements, wherein the mitochondrion specific promoter is selected from mitochondrial promoter nucleotide sequences, such as ATP6, ATP9, Cob, rrn18, Rps13, Rps19, Cox3, Nad6, Nad9 5' untranslated sequences (promoter region) of tobacco mitochondria, and Arabidopsis mitochondria; and the plastid specific promoter sequence is selected from the group consisting of the RNA polymerase promoter, rpo B promoter element, atpB promoter element, the clpP promoter element, the 16S rDNA promoter element, PrbcL, Prps16, the Prrn16, Prrn-62, Pycf2-1577, PatpB-289, Prps2-152, Prps16-107, Pycf1-41, PatpI-207, PclpP-511, PclpP-173, PaccD-129, PaccD-129 promoter of the tobacco accD gene, the PclpP-53 promoter of the clpP gene, the Prrn-62 promoter of the rrn gene, the Prps16-107 promoter of the rps16 gene, the PatpB/E-290 promoter of the tobacco atpB/E gene, and the PrpoB-345 promoter of the rpoB gene.
9. A method according to any one of statements 1 to 8, wherein the transgene or isolated nucleic acid sequence is selected from insulin, preproinsulin, proinsulin, glucagon, interferons such as α-interferon, β-interferon, γ-interferon, blood-clotting factors selected from Factor VII, VIII, IX, X, XI, and XII, fertility hormones including luteinising hormone, follicle stimulating hormone growth factors including epidermal growth factor, platelet-derived growth factor, granulocyte colony stimulating factor and the like, prolactin, oxytocin, thyroid stimulating hormone, adrenocorticotropic hormone, calcitonin, parathyroid hormone, somatostatin, erythropoietin (EPO), enzymes such as β-glucocerebrosidase, haemoglobin, serum albumin, collagen, biotic and abiotic stress proteins, such as insecticidal and insect toxic proteins, for example from, or derived from Bacillus thuringiensis, nematicidal proteins, herbicide resistance proteins, (e.g. to glyphosate), salt-tolerance proteins, drought tolerant proteins, proteins capable of conferring cytoplasmic male sterility to plant breeding lines; nutritional enhancement proteins involved in the biosynthesis of phenolics, starches, sugars, alkaloids, vitamins, and edible vaccines, monoclonal antibodies and active fragments thereof, industrial enzymes and active fragments thereof.
10. A method according to any one of statementss 1 to 9, wherein the transgene or isolated nucleic acid sequence is selected from proteins that confer cytoplasmic male sterility to a plant.
11. A method according to any one of the preceding statements, wherein the transgene or isolated nucleic acid sequence that is capable of conferring cytoplasmic male sterility is the plant is selected from the petunia mitochondrion *pcf* sequence, *orf107* sequence of sorghum and orf 79 of rice.
12. A method according to any one of the preceding statements, wherein the plant organellar transit peptide is independently selected from the mitochondrial signal peptide from tobacco F1-ATPase-1 β subunit, and the Arabidopsis CPN60 protein; and the plastidial transit peptide independently from selected from the tobacco rbcS-cTP, and the Arabidopsis HSP70-cTP protein.
13. A plant cell obtained according to any one of statements 1 to 12.
14. A plant cell comprising transformed plant organelles as defined in statements 1 to 13, wherein the transformed plant organelles comprise:
   i) an exogenous or heterologous left flanking sequence (LFS) and an exogenous or heterologous right flanking sequence (RFS);
   ii) at least one exogenous or heterologous organelle-specific promoter and at least one exogenous or heterologous organelle-specific terminator sequence; and
   iii) at least one exogenous or heterologous isolated transgene nucleic acid sequence of interest.
15. A plant cell according to statement 14, wherein the transformed organelles are selected from plant plastids and mitochondria transformed as defined in any one of statements 1 to 13.
16. A transformed plant organelle comprising:
   i) an exogenous or heterologous left flanking sequence (LFS) and an exogenous or heterologous right flanking sequence (RFS);
   ii) at least one exogenous or heterologous organelle-specific promoter and at least one exogenous or heterologous organelle-specific terminator sequence; and
   iii) at least one exogenous or heterologous isolated transgene nucleic acid sequence of interest.
17. A transformed plant organelle according to statement 16, wherein the plant organelle is selected from a plant plastid and a mitochondrion transformed as defined in any one of statements 1 to 13.
18. A population of transformed plant organelles made up of transformed organelles according to statement 16 or statement 17 comprised in a plant cell.
19. A population of transformed plant organelles according to statement 18, wherein the organelles are located in plant cells selected from tobacco *(Nicotiana tabacum)* and other *Nicotiana* species, arabidopsis, potato, corn(maize), canola (rape), rice, wheat, barley, brassica sp. such as cauliflower, broccoli (e.g. green and purple sprouting), cabbage (e.g. red, green and white cabbages), curly kale, Brussels sprouts, cotton, algae (e.g. blue green species), lemnospora, or moss (e.g. *physcomitrella patens),* tomato, capsicum, squashes, sunflower, soyabean, carrot, melons, grape vines, lettuce, strawberry, sugar beet, peas, and sorghum.
20. A population of transformed plant organelles according to statement 18 or statement 19, wherein the organelles are located in plant cells selected from cotton, rice, oilseed Brassica species such as canola, corn(maize) and soyabean.
21. A method of producing at least a heterologous or exogenous RNA species in a plant that comprises:
   1(a) deleting viral polymerase and coat protein sequences from the complete viral genome of a potyvirus and replacing them with transgenic nucleic acid **in cis,** wherein the said transgenic nucleic acid comprises a nuclear promoter operably linked to a 5' UTR sequence linked to the 5' end of a complete RNA translocation sequence of the potyvirus forming a potyviral vector, wherein
      i) the potyviral RNA translocation sequence is modified by covalently linking the 5' end of the VPg protein therein to an organellar transit peptide ; or
      ii) the potyviral RNA translocation sequence is modified by fusing a spytag short peptide sequence to the viral VPg protein at either the N- or C-terminus thereof; and introducing the product of i) or ii) into a plant cell ;
   1(b) introducing into the viral translocation sequence a second component nucleic acid sequence that encodes for an organellar transgene cassette comprising a left flanking sequence, an organellar promoter, at least one transgene nucleic acid sequence of interest, an organellar terminator and a right flanking sequence ; and
   1(c) introducing into the said plant cell a third component nucleic acid sequence that codes for a retron sequence for reverse transcription of the TNA ; and
   1(d)(i) introducing into the said plant cell a fourth component nucleic acid acid sequence comprising a viral 3'UTR sequence ; and
   1(d)(ii) introducing into the plant cell a nucleic acid sequence comprising a nuclear promoter operably linked to a nucleic acid sequence comprising a plant organellar transit peptide (TP) located adjacent to a retron-based reverse transcriptase fused to a intron encoding protein (IEP), and a nuclear terminator; and
   1(e)(i) introducing into the plant cell a further vector that comprises either a potyviral polymerase ***in trans*** under the control of a plant nuclear promoter sequence and a terminator ; or
   1(e)(ii) introducing into the plant cell a further vector that does not include a potyviral polymerase-containing vector of 1(e)(i), the vector comprising a spycatcher peptide fused to an organellar transit peptide, the said fused peptide being expressed under the control of a nuclear promoter.
   2) growing said regenerable plant cell of steps 1a) to 1e) ;
   3) selecting a plant cell of (2), wherein the transgene comprised within the plant organellar transgene cassette is integrated into the organellar genome;
   4) regenerating a plant from the plant cell of (6); and
   5) growing the plant of (4).
22. A method according to statement 21, wherein the heterologous or exogenous RNA species encoded by the transgene that is integrated into the organellar genome is expressed as a heterologous or exogenous protein.
23. A method according to statement 21 or statement 22, wherein the plant organellar genome is independently selected from that of plant mitochondria and plant plastids.
24. An isolated polynucleotide sequence that comprises
   1(a) a first component nucleic acid sequence comprising a nuclear promoter operably linked to a 5' UTR sequence linked to the 5' end of a complete RNA translocation sequence of a potyvirus forming a potyviral vector, wherein
      i) the potyviral RNA translocation sequence is modified by covalently linking the 5' end of the VPg protein therein to an organellar transit peptide ; or
      ii) the potyviral RNA translocation sequence is modified by fusing a spytag short peptide sequence to the viral VPg protein at either the N- or C-terminus thereof; and introducing the product of i) or ii) into a plant cell;
   1(b) a second component nucleic acid sequence that encodes for an organellar transgene cassette comprising a left flanking sequence, an organellar promoter, at least one transgene nucleic acid sequence of interest, an organellar terminator and a right flanking sequence ; and
   1(c) a third component nucleic acid sequence that codes for a retron-based reverse transcriptase fused to a reverse transcriptase of a group II intron ;
   1(d) a fourth component nucleic acid acid sequence that is a 3' UTR sequence; and
   1(e)(i) a fifth component nucleic acid sequence that comprises either a potyviral polymerase *in trans* under the control of a plant nuclear promoter sequence and a bacterial terminator ; or
      1(e)(ii) a fifth component nucleic acid sequence that does not include a potyviral polymerase-containing vector of 1(e)(i), the vector comprising a spycatcher peptide fused to an organellar transit peptide, the said fused peptide being expressed under the control of a nuclear promoter,
   for use in a method according to any one of statementss 1 to 13 and statements 21 to 23.
25. An isolated polynucleotide sequence as defined in any one of statements 1 to 13 and statements 21 to 24, comprising genomic DNA.
26. An isolated polynucleotide sequence as defined in any one of statements 1 to 13 and statements 21 to 24, comprising a cDNA component.
27. A nucleic acid vector suitable for transformation of a plant cell or a bacterial cell, wherein the cell includes a polynucleotide sequence according to any one of statements 24 to 26.
28. A nucleic acid vector according to statement 27 for transformation of a bacterial cell.
29. A nucleic acid vector according to statement 28 for transforming an *Agrobacterium* cell.
30. A host cell containing a heterologous polynucleotide or nucleic acid vector according to any one of statements 24 to 29.
31. A host cell according to statement 30 which is a plant cell or a bacterial cell.
32. A host cell according to statment 30 or statement 31 comprised in a plant, a plant part or a plant propagule, or an extract or derivative of a plant or in a plant cell culture.
33. A method of producing a cell according to any one of statements 30 to 32, the method including incorporating said polynucleotide or nucleic acid vector into the cell by means of transformation.
34. A method according to statement 33 which includes regenerating a plant from a cell according to any one of statements 30 to 32 from one or more transformed cells.
35. A plant comprising a plant cell according to any one of statements 30 to 32.
36. A plant comprising a plant cell according to statement 35 that is selected from the group consisting of tobacco *(Nicotiana tabacum)* and other *Nicotiana* species, such as *Nicotiana benthamiana,* carrot, vegetable and oilseed *Brassica's,* melons, Capsicums, grape vines, lettuce, strawberry, sugar beet, wheat, barley, (corn)maize, rice, soybean, peas, sorghum, sunflower, tomato, cotton, and potato.
37. A plant comprising a plant cell according to statement 35 or statement 36 that is selected from the group consisting of cotton, rice, oilseed Brassica species such as canola, corn(maize) and soybean.
38. A method of producing a plant, the method including incorporating a polynucleotide sequence or nucleic acid vector according to any one of statements 24 to 29 into a plant cell and regenerating a plant from said cell.
39. Use of a polynucleotide sequence according to any one of statements 24 to 26 in the production of a transgenic plant.
40. Use of a polynucleotide sequence according to any one of statements 24 to 26 in the production of a polypeptide or protein in a plant.
   All definitions for component parts of statements 1 to 40 of Variant 2 are found either in the accompanying description or in statements 1 to 40. The Experimental section provides technical descriptions of work performed relating to Variant 2.

## Claims

1. An *Agrobacterium* strain comprising
a) dysfunctional native virD2 and/or virE2 DNA sequences, substantially knock out mutations of native virD2 and/or virE2 DNA sequences, or no native virD2 and/or virE2 DNA sequences; and/or
b) an Agrobacterium binary vector comprising a modified VirD2 DNA sequence lying outside of the T-DNA region comprising at least one of:
i) a DNA sequence encoding an organellar transit peptide fused to the 5' end of a VirD2 DNA sequence;
ii) a DNA sequence encoding a spytag peptide fused to the 5' end of a VirD2 DNA sequence; and
iii) a DNA sequence encoding a spytag peptide fused to the 3' end of a VirD2 DNA sequence.

2. A vector according to claim 1, wherein the modified VirD2 sequence is under the transcriptional control of a bacterial promoter, preferably a virD1 promoter.

3. A vector according to claim 1 or claim 2, wherein the organellar transit peptide is selected from a plastid transit peptide or a mitochondrion transit peptide.

4. A vector according to any one of claims 1 to 3, wherein the organellar transit peptide is selected from transit peptides of chloroplasts, proplastids, etioplasts, chromoplasts, amyloplasts, leucoplasts and elaioplasts, preferably a transit peptide of a chloroplast.

5. A vector according to any one of claims 1 to 3, wherein the organellar transit peptide is a mitochondrion transit peptide.

6. A vector according to any one of claims 1 to 5 further comprising at least one of
iv) an organellar transgene cassette comprising two origins of replication, one being located adjacent to and at the 5' end of a left flanking sequence and the second being located adjacent to and at the 3' end of a right flanking sequence, at least one DNA sequence of interest under operative control of an organellar promoter, and an organellar terminator; and
v) an organellar transgene cassette comprising two origins of replication located at the 5' and 3' ends of the cassette, respectively, at least one DNA sequence of interest under operative control of an organellar promoter, wherein the organellar promoter is positioned downstream of the origin of replication at the 5' end of the transgene cassette, and an organellar terminator and the organellar cassette does not contain left and right flanking sequences;
wherein the said origins of replication are all derived from a geminivirus and the DNA sequences making up iv) and v), respectively, are all located within left and right T-DNA borders on the vector.

7. A vector according to claim 6, wherein the origins of replication are selected from Maize Streak Virus (MSV, subgroup I), Beet Curly Top Virus (BCTV, subgroup II) and Tomato Golden Mosaic Virus (TGMV, subgroup III).

8. A vector according to any one of the preceding claims, wherein expression of a viral Rep gene is either from the transgene DNA sequence under operational control of an organelle promoter or from co-expression from a nuclear cassette comprising a Rep gene fused to an organellar transit peptide, wherein the fused peptide is under operational control of a nuclear promoter and a nuclear terminator.

9. A vector according to any one of claims 1 to 8, wherein the DNA sequence encodes a SpyCatcher peptide fused to an organellar transit peptide, wherein the fused peptide is under operational control of a constitutive or chemically induced nuclear promoter and a nuclear terminator.

10. A vector according to any one of claims 6 to 9, wherein the organellar promoters, organellar terminators, and organellar transit peptides of i) ii) and iii) are selected from mitochondrial transit peptides and plastid transit peptides selected from chloroplasts, proplastids, etioplasts, chromoplasts, amyloplasts, leucoplasts and elaioplasts, preferably a transit peptide of a chloroplast.

11. A vector according to any one of claims 6 to 10, wherein the DNA sequence of interest is selected from a recombinant mammalian nucleic acid sequence, an isolated genomic mammalian nucleic acid sequence, a recombinant plant nucleic acid sequence and an isolated genomic plant nucleic acid sequence and two or more thereof.

12. A vector according to any one of claims 6 to 11, wherein the DNA sequence of interest is selected from insulin, preproinsulin, proinsulin, glucagon, interferons such as α-interferon, β-interferon, γ-interferon, blood-clotting factors selected from Factor VII, VIII, IX, X, XI, and XII, fertility hormones including luteinising hormone, follicle stimulating hormone growth factors including epidermal growth factor, platelet-derived growth factor, granulocyte colony stimulating factor and the like, prolactin,
oxytocin, thyroid stimulating hormone, adrenocorticotropic hormone, calcitonin, parathyroid hormone, somatostatin, erythropoietin (EPO), enzymes such as β-glucocerebrosidase, haemoglobin, serum albumin, collagen, biotic and abiotic stress proteins, such as insecticidal and insect toxic proteins, for example from, or derived from Bacillus thuringiensis, nematicidal proteins, herbicide resistance proteins, (e.g. to glyphosate), salt-tolerance proteins, drought tolerant proteins, proteins capable of conferring cytoplasmic male sterility to plant breeding lines; nutritional enhancement proteins involved in the biosynthesis of phenolics, starches, sugars, alkaloids, vitamins, and edible vaccines, monoclonal antibodies and active fragments thereof, industrial enzymes and active fragments thereof.

13. An isolated polynucleotide sequence that comprising a modified VirD2 DNA sequence comprising at least one of:
i) a DNA sequence encoding an organellar transit peptide fused to the 5' end of a VirD2 DNA sequence;
ii) a DNA sequence encoding a spytag peptide fused to the 5' end of a VirD2 DNA sequence; and
iii) a DNA sequence encoding a spytag peptide fused to the 3' end of a VirD2 DNA sequence.

14. An isolated polynucleotide sequence according to claim 13 further comprising at least one of
iv) an organellar transgene cassette comprising two origins of replication, one being located adjacent to and at the 5' end of a left flanking sequence and the second being located adjacent to and at the 3' end of a right flanking sequence, at least one DNA sequence of interest under operative control of an organellar promoter, and an organellar terminator; and
v) an organellar transgene cassette comprising two origins of replication located at the 5' and 3' ends of the cassette, respectively, at least one DNA sequence of interest under operative control of an organellar promoter, wherein the organellar promoter is positioned downstream of the origin of replication at the 5' end of the transgene cassette, and an organellar terminator and the organellar cassette does not contain left and right flanking sequences; and
wherein the said origins of replication are all derived from a geminivirus.

15. An isolated polynucleotide sequence as defined in any one of claims 1 to 14 comprising genomic DNA and/or cDNA.

16. Use of a polynucleotide sequence according to claim 14 or claim 15 in the production of a transgenic plant.

17. Use of a polynucleotide sequence according to claim 14 or claim 15 in the production of a polypeptide or protein in a plant.

18. A method of transforming a plant cell with a DNA of interest via an *Agrobacterium* vector comprising the steps of:
a) introducing into a binary vector where a virD2 gene fused to a plant organelle transit peptide outside of the T-DNA region; and
b) introducing the DNA of interest into the T-DNA region on a modified strain of *Agrobacterium;* and
c) introducing the DNA of interest into the plant cell using at least one of:
iv) an organellar transgene cassette comprising two origins of replication, one being located adjacent to the 5' end of a left flanking sequence and the second being located adjacent to the 3' end of a right flanking sequence, at least one DNA sequence of interest encoding a transgene of interest under operative control of an organellar promoter, and an organellar terminator; and
v) an organellar transgene cassette comprising two origins of replication located at the 5' and 3' ends of the cassette, respectively, at least one DNA sequence of interest encoding a transgene of interest under operative control of an organellar promoter, the organellar promoter being positioned downstream of the origin of replication at the 5' end of the transgene cassette, an organellar terminator and wherein the organellar cassette does not contain left and right flanking sequences;
wherein the said origins of replication are all derived from a geminivirus and the DNA sequences making up iv) and v), respectively, are all located within a left border and a right border on the vector.

19. A method according to claim 18 further comprising introducing into the plant cell a second nucleic acid sequence comprising a Rep gene fused to a plant organelle transit peptide under operative control of a nuclear promoter, and a nuclear terminator.

20. A method of transforming a plant cell with a DNA of interest via an *Agrobacterium* vector comprising the steps of:
a) introducing into a binary vector a virD2 gene fused to a spytag peptide at the 5' or 3' end of the virD2 gene and outside of the T-DNA region;
a(i) introducing the DNA of interest into the T-DNA region on a modified strain of *Agrobacterium;* and
a (ii) introducing the DNA of interest into the plant cell using at least one of:
b(i) an organellar transgene cassette comprising two origins of replication, one being located adjacent to the 5' end of a left flanking sequence and the second being located adjacent to the 3' end of a right flanking sequence, at least one DNA sequence of interest encoding a transgene of interest under operative control of an organellar promoter and an organellar terminator; and
b(ii) an organellar transgene cassette comprising two origins of replication located at the 5' and 3' ends of the cassette, respectively, at least one DNA sequence of interest encoding a transgene of interest under operative control of an organellar promoter, the organellar promoter being positioned downstream of the origin of replication at the 5' end of the transgene cassette, and an organellar terminator,wherein the organellar cassette does not contain left and right flanking sequences, and further wherein the said origins of replication are all derived from a geminivirus and the DNA sequences making up b(i) and b(ii), respectively, are all located within a left border and a right border on the vector;
c) introducing into the plant cell a second nucleic acid sequence comprising a Rep gene fused to a plant organelle transit peptide under operative control of a nuclear promoter and a nuclear terminator;
d) introducing into the plant cell a third nucleic acid sequence comprising a SpyCatcher gene fused to a plant organelle transit peptide under operative control of a nuclear constitutive or chemically inducible promoter and a nuclear terminator.

21. A method according claim 20, wherein the organellar promoter and organellar terminator are selected from a plant mitochondrion promoter, a plant mitochondrion terminator, a plant plastid promoter, and a plant plastid terminator, respectively.

22. A method according to claim 20 or claim 21, wherein the plant organellar promoter and plant organellar terminator are selected from plastid promoters and plant plastid terminators selected from chloroplasts, proplastids, etioplasts, chromoplasts, amyloplasts, leucoplasts and elaioplasts, the promoter and terminator being preferably selected from chloroplasts.

23. A method according to any one of claims 20 to 22, wherein the DNA sequence of interest is selected from a recombinant mammalian nucleic acid sequence, an isolated genomic mammalian nucleic acid sequence, a recombinant plant nucleic acid sequence and an isolated genomic plant nucleic acid sequence and two or more thereof.

24. A method according to any one of claims 20 to 23, wherein the mitochondrion specific promoter is selected from mitochondrial promoter nucleotide sequences, such as ATP6, ATP9, Cob, rrn18, Rps13, Rps19, Cox3, Nad6, Nad9 5' untranslated sequences (promoter region) of tobacco mitochondria, and Arabidopsis mitochondria; and the plastid specific promoter sequence is selected from the group consisting of the RNA polymerase promoter, rpo B promoter element, atpB promoter element, the clpP promoter element, the 16S rDNA promoter element, PrbcL, Prps16, the Prrn16, Prrn-62, Pycf2-1577, PatpB-289, Prps2-152, Prps16-107, Pycf1-41, PatpI-207, PclpP-511, PclpP-173, PaccD-129, PaccD-129 promoter of the tobacco accD gene, the PclpP-53 promoter of the clpP gene, the Prrn-62 promoter of the rrn gene, the Prps16-107 promoter of the rps16 gene, the PatpB/E-290 promoter of the tobacco atpB/E gene, and the PrpoB-345 promoter of the rpoB gene.

25. A method according to claim any one of claims 20 to 24, wherein the DNA sequence of interest or isolated nucleic acid sequence of interest encodes a transgene of interest selected from insulin, preproinsulin, proinsulin, glucagon, interferons such as α-interferon, β-interferon, γ-interferon, blood-clotting factors selected from Factor VII, VIII, IX, X, XI, and XII, fertility hormones including luteinising hormone, follicle stimulating hormone growth factors including epidermal growth factor, platelet-derived growth factor, granulocyte colony stimulating factor and the like, prolactin, oxytocin, thyroid stimulating hormone, adrenocorticotropic hormone, calcitonin, parathyroid hormone, somatostatin, erythropoietin (EPO), enzymes such as β-glucocerebrosidase, haemoglobin, serum albumin, collagen, biotic and abiotic stress proteins, such as insecticidal and insect toxic proteins, for example from, or derived from Bacillus thuringiensis, nematicidal proteins, herbicide resistance proteins, (e.g. to glyphosate), salt-tolerance proteins, drought tolerant proteins, proteins capable of conferring cytoplasmic male sterility to plant breeding lines; nutritional enhancement proteins involved in the biosynthesis of phenolics, starches, sugars, alkaloids, vitamins, and edible vaccines, monoclonal antibodies and active fragments thereof, industrial enzymes and active fragments thereof.

26. A method according to any one of claims 20 to 25, wherein the transgene or isolated nucleic acid sequence that is capable of conferring cytoplasmic male sterility to the plant is selected from the petunia mitochondrion *pcf* sequence, *orf107* sequence of sorghum and orf 79 of rice.

27. A method of transforming a plant cell with a DNA of interest via an *Agrobacterium* vector comprising the steps of:
a) introducing into the plant cell at least a first nucleic acid sequence that comprises:
i) an organellar transgene cassette comprising two origins of replication located at the 5' and 3' ends of the cassette, respectively, at least one DNA sequence of interest encoding a transgene of interest under operative control of an organellar promoter, the organellar promoter being positioned downstream of the origin of replication at the 5' end of the transgene cassette, an organellar terminator and wherein the organellar cassette does not contain left and right flanking sequences; and
ii) and introducing into the plant cell at least a second nucleic acid sequence comprising a viral Rep gene co-presented on a nuclear cassette comprising a *Rep* gene fused to an organellar transit peptide, wherein the fused peptide is under operational control of a nuclear promoter and a nuclear terminator ; and
wherein the origin of replication is derived from a geminivirus and the DNA sequences making up the organellar transgene cassette of i) are all located within a left border and a right border on the vector.

28. A method according to claim 27, wherein the organellar transit peptide of ii) is independently selected from a mitochondrial transit peptide and a plastid transit peptide.

29. A method according to claim 27 or claim 28, wherein the mitochondrial transit peptide is selected from tobacco F1-ATPase-1 β subunit, and the Arabidopsis CPN60 protein; and the plastid transit peptide is independently selected from the tobacco rbcS-cTP and the Arabidopsis HSP70-cTP protein.

30. A plant cell obtained according to any one of claims 20 to 29.

31. A plant cell transformed with a vector, a transgene cassette, transgene or isolated DNA sequence as defined in any one of claims 1 to 29.

32. A plant cell according to claim 31, including transformed organelles selected from plant plastids and mitochondria transformed as defined in any one of claims 1 to 29.

33. A transformed plant organelle as defined in claim 32.

34. A population of transformed plant organelles according to claim 33 comprised in a plant cell.

35. A population of transformed plant organelles according to claim 34, wherein the organelles are located in plant cells selected from tobacco *(Nicotiana tabacum)* and other *Nicotiana* species, arabidopsis, potato, corn(maize), canola (rape), rice, wheat, barley, brassica sp. such as cauliflower, broccoli (e.g. green and purple sprouting), cabbage (e.g. red, green and white cabbages), curly kale, Brussels sprouts, cotton, algae (e.g. blue green species), lemnospora, or moss (e.g. *physcomitrella patens),* tomato, capsicum, squashes, sunflower, soyabean, carrot, melons, grape vines, lettuce, strawberry, sugar beet, peas, and sorghum.

36. A population of transformed plant organelles according to claim 34 or claim 35, wherein the organelles are located in plant cells selected from cotton, rice, oilseed Brassica species such as canola, corn(maize) and soyabean.

37. A method of producing at least a heterologous or exogenous protein in a plant that comprises:
1) introducing into a regenerable plant cell a vector, transgene cassette, transgene or isolated DNA sequence as defined in any one of claims 1 to 29;
2) growing said regenerable plant cell of step 1);
3) selecting a plant cell of 2), wherein the transgene or isolated DNA sequence is integrated into the organellar genome;
4) regenerating a plant from the plant cell of 3); and
5) growing the plant of (7).

38. A method according to claim 37, wherein the plant organellar genome is independently selected from that of plant mitochondria and plant plastids.

39. A host cell containing a heterologous polynucleotide or nucleic acid vector as defined in any one of claims 1 to 29.

40. A host cell according to claim 39 which is a plant cell or a bacterial cell.

41. A host cell according to claim 39 or claim 40 comprised in a plant, a plant part or a plant propagule, or an extract or derivative of a plant or in a plant cell culture.

42. A plant comprising a plant cell according to any one of claims 30 to 32.

43. A plant comprising a plant cell according to claim 42 that is selected from the group consisting of tobacco *(Nicotiana tabacum)* and other *Nicotiana* species, such as *Nicotiana benthamiana,* carrot, vegetable and oilseed *Brassica's,* melons, Capsicums, grape vines, lettuce, strawberry, sugar beet, wheat, barley, (corn)maize, rice, soybean, peas, sorghum, sunflower, tomato, cotton, and potato.

44. A plant comprising a plant cell according to claim 42 or claim 43 that is selected from the group consisting of cotton, rice, oilseed Brassica species such as canola, corn(maize) and soybean.
